# EUROPEAN PATENT APPLICATION

(11) **EP 4 696 681 A1**
(43) Date of publication of application: **18.02.2026**
(21) Application number: 24194677.1
(22) Date of filing: 14.08.2024
(51) Int. Cl.: C07D 261/04, A01N 43/74, A01P 13/02, C07D 413/12

(54) **HERBICIDAL THIOCARBAMATES CARRYING AN ISOXAZOLIN-SUBSTITUTED PHENYL RING**

(71) Applicant: BASF SE, 67056 Ludwigshafen am Rhein (DE)
(72) Inventor: SKOTNITZKI, Juri Gerhard, 67056 Ludwigshafen am Rhein (DE); WITSCHEL, Matthias, 67056 Ludwigshafen am Rhein (DE); BETZ, Michael, 67056 Ludwigshafen am Rhein (DE); SEITZ, Thomas, 67056 Ludwigshafen am Rhein (DE); NEWTON, Trevor William, 67435 Neustadt (DE); HARTMUELLER, Martin, 67117 Limburgerhof (DE); JOHNEN, Philipp Rudi, 67117 Limburgerhof (DE); PORRI, Aimone, 67117 Limburgerhof (DE); LOPEZ CARRILLO, Veronica, 67117 Limburgerhof (DE); ANDERS, Ulrike, 67117 Limburgerhof (DE); DOMBO, Peter, 67117 Limburgerhof (DE); LERCHL, Jens, 67117 Limburgerhof (DE)
(74) Representative: Meissner Bolte Partnerschaft mbB

(57) **Abstract**

The present invention relates to thiocarbamates carrying an isoxazolin-substituted phenyl ring of the formula (I) 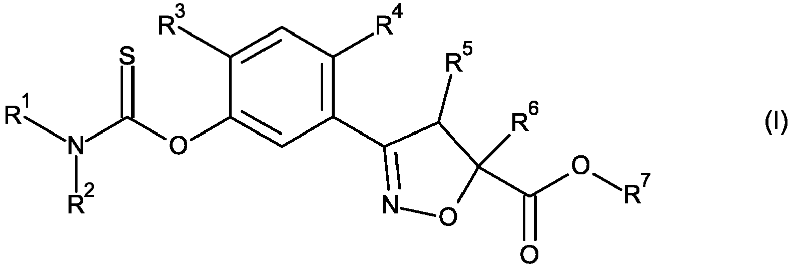 wherein the variables are as defined in the claims and the description, and to compositions comprising the same. The invention also relates to the use of said thiocarbamates or composiions containing the same for controlling/combatting undesired vegetation and to methods of controlling/combatting undesired vegetation comprising applying said thiocarbamates or compositions containing the same.

## Description

The present invention relates to thiocarbamates carrying an isoxazolin-substituted phenyl ring and compositions comprising the same. The invention also relates to the use of the thiocarbamates carrying an isoxazolin-substituted phenyl ring or the corresponding compositions for controlling unwanted vegetation. Furthermore, the invention relates to methods of controlling/combatting undesired vegetation comprising applying the thiocarbamates carrying an isoxazolin-substituted phenyl ring or the corresponding compositions.

### BACKGROUND OF THE INVENTION

For the purpose of controlling unwanted vegetation, especially in crops, there is an ongoing need for new herbicides that have high activity and selectivity together with a substantial lack of toxicity for humans and animals.

US 3,217,002 and US 3,224,862 relate to herbicidal phenylthionocarbamates which carry on the phenyl ring one or more halogen atoms and optionally a lower alkyl group, and where the nitrogen atom of the thiocarbamate group is part of a heterocyclic ring.

EP 0078099 A2 relates to herbicidal phenylcarbamates and phenylthionocarbamates which carry on the phenyl ring one or more halogen atoms and a group -OR, where R is alkyl which may be interrupted by one or more oxygen atoms, (halo)alkenyl or (halo)alkynyl, and where the nitrogen atom of the (thio)carbamate group is part of a heterocyclic ring.

EP 0065372 A1 relates to herbicidal phenylcarbamates and phenylthionocarbamates which carry on the phenyl ring one or more halogen atoms and a halocyclopropylmethoxy group, and where the nitrogen atom of the (thio)carbamate group is part of a heterocyclic ring.

H. Morinaka et al. describe in Weed Research, 1994, 39(1), 11-18 the herbicidal activity of phenylcarbamates and phenylthionocarbamates carrying on the phenyl ring, which is bound to the oxygen atom of the (thio)carbamate group, halogen, alkyl, alkoxy and/or nitro substituents and on the nitrogen atom of the (thio)carbamate group a 6-methoxy-2-pyridyl ring.

The compounds of the prior art often suffer from insufficient herbicidal activity, in particular at low application rates, and/or unsatisfactory selectivity resulting in a low compatibility with crop plants.

Accordingly, it is an object of the present invention to provide further phenylthiocarbamate compounds having a strong herbicidal activity, in particular even at low application rates, a sufficiently low toxicity for humans and animals and/or a high compatibility with crop plants, i.e. low phytotoxicity for crop plants. The phenylthiocarbamates should also show a broad activity spectrum against a large number of different unwanted plants.

These and further objectives are achieved by the compounds of formula (I) defined below including their agriculturally acceptable salts and stereoisomers.

### SUMMARY OF THE INVENTION

Accordingly, the present invention provides compounds of formula (I) 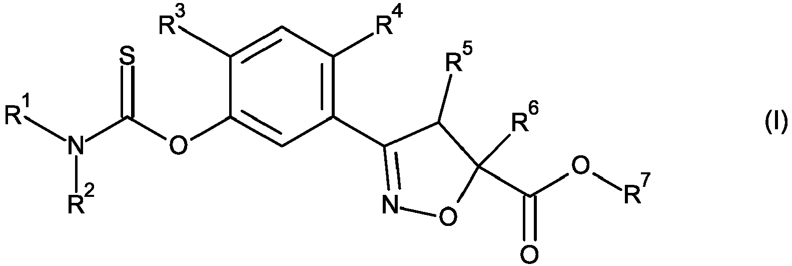 wherein:
- R¹ and R²,: independently of each other, are C₁-C₆-alkyl, C₁-C₆-haloalkyl, C₃-C₆-cycloalkyl or C₃-C₆-halocycloalkyl; or
- R¹ and R²,: together with the nitrogen atom they are bound to, form a 3- to 7-membered saturated heterocyclic ring which may contain a further heteroatom selected from
- N, O and S: as ring member, and where the heterocyclic ring may carry one or more substituents selected from the group consisting of halogen, cyano, C₁-C₄-alkyl and C₁-C₄-haloalkyl;
- R³ and R⁴,: independently of each other, are hydrogen, halogen, cyano or nitro;
- R⁵ is: hydrogen, C₁-C₆-alkyl, C₁-C₆-haloalkyl, C₂-C₆-alkenyl, C₂-C₆-haloalkenyl, C₁-C₆-alkoxy or C₁-C₆-haloalkoxy;
- R⁶ is: C₁-C₆-alkyl, C₁-C₆-haloalkyl, C₂-C₆-alkenyl, C₂-C₆-haloalkenyl, C₁-C₆-alkoxy or C₁-C₆-haloalkoxy; or
- R⁵ and R⁶: form together a C₃-C₅-alkylene group which may carry one or more substituents selected from the group consisting of halogen, cyano, C₁-C₄-alkyl and C₁-C₄-haloalkyl; and
- R⁷ is: hydrogen, C₁-C₆-alkyl, C₁-C₆-haloalkyl, C₃-C₆-cycloalkyl or C₃-C₆-halocycloalkyl;
or an agriculturally acceptable salt thereof, or a stereoisomer thereof.

The invention also relates to a composition comprising at least one compound of formula (I) and at least one auxiliary which is customary for formulating crop protection compounds.

The present invention also provides combinations comprising at least one compound of formula (I) (component A) and at least one further compound selected from the herbicidal compounds B (component B; different from A) and safeners C (component C).

The invention relates moreover to the use of a compound of formula (I) or of said compositions for controlling (combatting) unwanted vegetation, and to a method for controlling (combatting) unwanted vegetation which comprises allowing a herbicidally effective amount of at least one compound of formula (I) or of said compositions to act on plants, their seed and/or their habitat.

### DETAILED DESCRIPTION OF THE INVENTION

### Definitions:

Stereoisomers: The compounds of formula (I) may be present as mixtures of enantiomers or diastereomers but also in the form of the pure enantiomers or pure diastereomers. The invention provides both the pure enantiomers or pure diastereomers of the compounds of formula I, and their mixtures and the use according to the invention of the pure enantiomers or pure diastereomers of the compound of formula I or its mixtures. Suitable compounds of formula I also include all possible geometrical stereoisomers (cis/trans or Z/E isomers) as a specific form of diastereomers and mixtures thereof. Cis/trans isomers may be present with respect to an alkene, carbon-nitrogen double-bond, nitrogen-sulfur double bond, amide group or a cyclic, non-aromatic moiety. The term "stereoisomer(s)" encompasses both optical isomers, such as enantiomers or diastereomers existing due to more than one stereogenic center in the molecule, as well as geometrical isomers (cis/trans isomers). Just by way of example, stereogenic centers in the compounds (I) are the carbon atoms carrying R⁵ and R⁶. Further stereogenic centers may be present e.g. in alkyl substituents R¹, R², R⁶ and R⁷ if these contain at least 4 carbon atoms and have the corresponding structure, in haloalkyl or halocycloalkyl groups having the suitable substitution pattern, in suitably substituted carbon ring atoms in the ring formed by R¹, R² and the nitrogen atom they are bound to or in the ring formed by R⁵, R⁶ and the carbon atoms they are bound to, etc.

If the above-mentioned herbicidal compounds B and/or the safeners C have one or more stereogenic centres they may also be present as enantiomers or diastereomers, and it is possible to use both the pure enantiomers and diastereomers or their mixtures.

If the compounds of formula (I), the herbicidal compounds B and/or the safeners C as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diethylammonium, diisopropylammonium, trimethylammonium, triethylammonium, tris(isopropyl)ammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogen phosphate, nitrate, bicarbonate, carbonate, hexafluorosilicate, hexafluorophosphate, benzoate and also the anions of C₁-C₄-alkanoic acids, preferably formate, acetate, propionate and butyrate.

The term "undesired vegetation" ("weeds") is understood to include any vegetation growing in non-crop-areas or at a crop plant site or locus of seeded and otherwise desired crop, where the vegetation is any plant species, including their germinant seeds, emerging seedlings and established vegetation, other than the seeded or desired crop (if any). Weeds, in the broadest sense, are plants considered undesirable in a particular location.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "partially or completely halogenated" will be taken to mean that 1 or more, e.g. 1, 2, 3, 4 or 5 or all of the hydrogen atoms of a given radical have been replaced by a halogen atom, in particular by fluorine or chlorine. A partially or completely halogenated radical is termed below also "halo-radical". For example, partially or completely halogenated alkyl is also termed haloalkyl.

The term "alkyl" as used herein (and in the alkyl moieties of other groups comprising an alkyl group, e.g. alkoxy) denotes in each case a straight-chain or branched alkyl group having usually from 1 to 6 carbon atoms (= C₁-C₆-alkyl), in particular 1 to 4 carbon atoms (= C₁-C₄-alkyl) and especially from 1 to 3 carbon atoms (= C₁-C₃-alkyl) or 1 or 2 carbon atoms (= C₁-C₂-alkyl). C₁-C₂-Alkyl is methyl or ethyl. C₁-C₃-Alkyl is methyl, ethyl, n-propyl or iso-propyl. Examples of C₁-C₄-alkyl are methyl, ethyl, n-propyl, iso-propyl, n-butyl, 2-butyl (= sec-butyl), isobutyl and tert-butyl. Examples for C₁-C₆-alkyl are, in addition to those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and other positional isomers thereof.

The term "haloalkyl" as used herein (and in the haloalkyl moieties of other groups comprising a haloalkyl group, e.g. haloalkoxy), which is also expressed as "alkyl which is partially or fully halogenated", denotes in each case a straight-chain or branched alkyl group having usually from 1 to 6 carbon atoms (= C₁-C₆-haloalkyl), more frequently 1 to 4 carbon atoms (= C₁-C₄-haloalkyl) or 1 to 3 carbon atoms (= C₁-C₃-haloalkyl) or 1 to 2 carbon atoms (= C₁-C₂-haloalkyl), as defined above, wherein the hydrogen atoms of this group are partially or totally replaced with halogen atoms. Preferred haloalkyl moieties are selected from C₁-C₃-haloalkyl, specifically from C₁-C₂-haloalkyl, in particular from fluorinated C₁-C₂-alkyl. Examples for C₁-C₂-haloalkyl are fluoromethyl, difluoromethyl, trifluoromethyl, chloromethyl, dichloromethyl, trichloromethyl, chlorofluoromethyl, dichlorofluoromethyl, chlorodifluoromethyl, bromomethyl, 1-fluoroethyl, 2-fluoroethyl, 2,2-difluoroethyl, 2,2,2-trifluoroethyl, pentafluoroethyl, 1-chloroethyl, 2-chloroethyl, 2,2,-dichloroethyl, 2,2,2-trichloroethyl, 2-chloro-2-fluoroethyl, 2-chloro-2,2-difluoroethyl, 2,2-dichloro-2-fluoroethyl, 1-bromoethyl, and the like. Examples for C₁-C₃-haloalkyl are, in addition to those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, 1-chloropropyl, 2-chloropropyl, 3-chloropropyl, and the like. Examples for C₁-C₄-haloalkyl are, in addition to those mentioned for C₁-C₃-haloalkyl, 1-fluorobutyl, 2-fluorobutyl, 3-fluorobutyl, 4-fluorobutyl, 1-chlorobutyl, 2-chlorobutyl, 3-chlorobutyl, 4-chlorobutyl, and the like.

The term "alkenyl" as used herein denotes in each case a monounsaturated straight-chain or branched hydrocarbon radical having usually 2 to 6 carbon atoms (= C₂-C₆-alkenyl), in particular 2 to 4 carbon atoms (= C₂-C₄-alkenyl) or 2 or 3 carbon atoms (= C₂-C₃-alkenyl), and a double bond in any position, for example C₂-C₃-alkenyl, such as ethenyl, 1-propenyl, 2-propenyl or 1-methylethenyl; C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and other isomers thereof.

The term "haloalkenyl" as used herein, which may also be expressed as "alkenyl which is substituted by halogen", and the haloalkenyl moieties in haloalkenyloxy and the like refers to unsaturated straight-chain or branched hydrocarbon radicals having 2 to 6 (= C₂-C₆-haloalkenyl) or 2 to 4 (= C₂-C₄-haloalkenyl) or 2 to 3 (= C₂-C₃-haloalkenyl) carbon atoms and a double bond in any position, where some or all of the hydrogen atoms in these groups are replaced by halogen atoms as mentioned above, in particular fluorine, chlorine and bromine, for example chlorovinyl, chloroallyl and the like.

The term "cycloalkyl" as used denotes in each case a mono- or bicyclic, saturated cycloaliphatic radical having usually from 3 to 6 carbon atoms (= C₃-C₆-cycloalkyl), 3 to 5 carbon atoms (= C₃-C₅-cycloalkyl) or 3 to 4 carbon atoms (= C₃-C₄-cycloalkyl) as (only) ring members. Examples of monocyclic saturated cycloaliphatic radicals having 3 or 4 carbon atoms comprise cyclopropyl and cyclobutyl. Examples of monocyclic saturated cycloaliphatic radicals having 3 to 5 carbon atoms comprise cyclopropyl, cyclobutyl and cyclopentyl. Examples of monocyclic saturated cycloaliphatic radicals having 3 to 6 carbon atoms comprise cyclopropyl, cyclobutyl, cyclopentyl and cyclohexyl. Bicyclic cycloalkyl has 5 or 6 carbon atoms. Examples of bicyclic radicals having 5 or 6 carbon atoms comprise bicyclo[1.1.1]pentyl and bicyclo[2.1.1]hexyl. Preferably, cycloalkyl is monocyclic.

The term "halocycloalkyl" as used herein denotes in each case a mono- or bicyclic cycloaliphatic radical having usually from 3 to 6 carbon atoms ("C₃-C₄-halocycloalkyl"), 3 to 5 carbon atoms ("C₃-C₅-halocycloalkyl") or 3 to 4 carbon atoms ("C₃-C₅-halocycloalkyl"), wherein at least one, e.g. 1, 2, 3, 4 or 5 of the hydrogen atoms are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "alkoxy" as used herein denotes in each case a straight-chain or branched alkyl group usually having from 1 to 6 carbon atoms (= C₁-C₆-alkoxy), preferably 1 to 4 carbon atoms (= C₁-C₄-alkoxy), e.g. 1 to 3 carbon atoms (= C₁-C₃-alkoxy), in particular 1 or 2 carbon atoms (= C₁-C₂-alkoxy), which is bound to the remainder of the molecule via an oxygen atom. C₁-C₂-Alkoxy is methoxy or ethoxy. C₁-C₃-Alkoxy is additionally, for example, n-propoxy or 1-methylethoxy (isopropoxy). C₁-C₆-Alkoxy is additionally, for example, butoxy, 1-methylpropoxy (sec-butoxy), 2-methylpropoxy (isobutoxy) or 1,1-dimethylethoxy (tert-butoxy), pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

The term "haloalkoxy" as used herein denotes in each case a straight-chain or branched alkoxy group, as defined above, having from 1 to 6 carbon atoms (= C₁-C₆-haloalkoxy), preferably 1 to 4 carbon atoms (= C₁-C₄-alkoxy), e.g. 1 to 3 carbon atoms (= C₁-C₃-haloalkoxy), in particular 1 or 2 carbon atoms (= C₁-C₂-haloalkoxy), wherein the hydrogen atoms of this group are partially or totally replaced with halogen atoms, in particular fluorine atoms (in this case, the radical is also termed fluorinated alkoxy). C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₆-Haloalkoxy is additionally, for example, 4-fluorobutoxy, 4-chlorobutoxy, 4-bromobutoxy or nonafluorobutoxy , 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

Alkylene is a linear or branched divalent alkanediyl radical. C₃-C₅-Alkylene (= C₃-Cs-alkanediyl) is a linear or branched divalent alkyl radical having 3 to 5 carbon atoms. Examples are -CH₂CH₂CH₂-, -CH(CH₃)CH₂-, -CH₂CH(CH₃)-, -C(CH₃)₂-, -CH₂CH₂CH₂CH₂-, -CH(CH₃)CH₂CH₂-, -CH₂CH₂CH(CH₃)-, -C(CH₃)₂CH₂-, -CH₂C(CH₃)₂-, -(CH₂)₅-, and positional isomers thereof. Linear C₃-C₅-alkylene is -CH₂CH₂CH₂-, -CH₂CH₂CH₂CH₂- or -(CH₂)₅-.

R¹ and R², together with the nitrogen atom they are bound to, may form a 3- to 7-membered saturated heterocyclic ring which may contain a further heteroatom selected from N, O and S as ring member. Examples for such N-bound heterocyclic rings are aziridin-1-yl, azetidin-1-yl, pyrrolydin-1-yl, pyrazolidin-1-yl, oxazolidin-3-yl, isoxazolidin-2-yl, thiazolidin-3-yl, isothiazolidin-2-yl, imidazolidin-1-yl, piperidin-1-yl, hexahydro-pyridazin-1-yl, hexahydropyrimidin-1-yl, piperazin-1-yl, morpholin-4-yl, thiomorpholin-4-yl, azepan-1-yl, hexahydro-1,3-diazepin-1-yl, hexahydro-1,4-diazepin-1-yl, hexahydro-1,3-oxazepin-3-yl, hexa-hydro-1,4-oxazepin-4-yl, and the like.

If R¹ and R², together with the nitrogen atom they are bound to, form a 5-membered saturated heterocyclic ring, this is pyrrolidin-1-yl.

The remarks made below as to preferred embodiments of the variables (substituents) of the compounds of formula I are valid on their own as well as preferably in combination with each other, as well as in combination with the stereoisomers or salts thereof. The remarks made below concerning preferred embodiments of the variables further are valid on their own as well as preferably in combination with each other concerning the compounds of formula I, where applicable, as well as concerning the uses and methods according to the invention and the composition according to the invention.

Preferably, R¹ and R², together with the nitrogen atom they are bound to, form a 5-membered saturated heterocyclic ring (i.e. pyrrolidin-1-yl) which may carry one or more substituents selected from the group consisting of halogen, cyano, C₁-C₄-alkyl and C₁-C₄-haloalkyl. More preferably, R¹ and R², together with the nitrogen atom they are bound to, form a 5-membered saturated heterocyclic ring (i.e. pyrrolidin-1-yl) which may carry one or two substituents selected from the group consisting of halogen and C₁-C₄-alkyl, in particular from fluorine and methyl.

In an alternatively preferred embodiment, R¹ and R², independently of each other, are C₁-C₄-alkyl, in particular C₁-C₃-alkyl.

More preference is however given to R¹ and R² forming together with the nitrogen atom they are bound to a (N-bound) ring.

Preferably, R³ is hydrogen or halogen, specifically hydrogen or fluorine.

Preferably, R⁴ is halogen, specifically chlorine.

More preferably, R³ is hydrogen or halogen, and R⁴ is halogen. In a specific embodiment, R³ is hydrogen or fluorine, and R⁴ is chlorine.

Preferably, R⁵ is hydrogen or C₁-C₄-alkyl, more preferably hydrogen.

Preferably, R⁶ is C₁-C₄-alkyl, C₂-C₄-alkenyl or C₁-C₄-alkoxy. More preferably, R⁶ is C₁-C₃-alkyl, C₂-C₃-alkenyl or C₁-C₃-alkoxy. In particular, R⁶ is C₁-C₃-alkyl, C₂-C₃-alkenyl or C₁-C₂-alkoxy.

Preferably, R⁶ is C₁-C₄-alkyl, C₂-C₄-alkenyl or C₁-C₄-alkoxy and R⁵ is hydrogen. More preferably, R⁶ is C₁-C₃-alkyl, C₂-C₃-alkenyl or C₁-C₃-alkoxy and R⁵ is hydrogen. In particular, R⁶ is C₁-C₃-alkyl, C₂-C₃-alkenyl or C₁-C₂-alkoxy and R⁵ is hydrogen.

In an alternatively preferred embodiment, R⁵ and R⁶ form together a C₃-C₅-alkylene bridging group which may carry one or more substituents selected from the group consisting of halogen, cyano, C₁-C₄-alkyl and C₁-C₄-haloalkyl. More preferably, R⁵ and R⁶ form together a linear C₃-C₅-alkylene bridging group (i.e. -(CH₂)₃-, -(CH₂)₄- or -(CH₂)₅-) which may carry one or more substituents selected from the group consisting of halogen, cyano, C₁-C₄-alkyl and C₁-C₄-haloalkyl. Even more preferably, R⁵ and R⁶ form together a linear C₃-C₅-alkylene bridging group (which is unsubstituted), in particular a linear C₃-alkylene group (i.e. -(CH₂)₃-).

Preferably, R⁷ is hydrogen or C₁-C₄-alkyl, more preferably hydrogen or C₁-C₃-alkyl.

In a preferred embodiment
- R¹ and R²,: independently of each other, are C₁-C₄-alkyl; or
- R¹ and R²,: together with the nitrogen atom they are bound to, form a 5-membered saturated heterocyclic ring which may carry one or more substituents selected from the group consisting of halogen, cyano, C₁-C₄-alkyl and C₁-C₄-haloalkyl;
- R³ is: hydrogen or halogen;
- R⁴: is halogen;
- R⁵: is hydrogen;
- R⁶: is C₁-C₄-alkyl, C₂-C₄-alkenyl or C₁-C₄-alkoxy; or
- R⁵ and R⁶: form together a C₃-alkylene group; and
- R⁷: is hydrogen or C₁-C₄-alkyl.

In a more preferred embodiment,
- R¹ and R²,: independently of each other, are C₁-C₄-alkyl; or
- R¹ and R²,: together with the nitrogen atom they are bound to, form a 5-membered saturated heterocyclic ring which may carry one or more substituents selected from the group consisting of halogen and C₁-C₄-alkyl;
- R³ is: hydrogen or halogen;
- R⁴ is: halogen;
- R⁵ is: hydrogen;
- R⁶ is: C₁-C₄-alkyl, C₂-C₄-alkenyl or C₁-C₄-alkoxy; or
- R⁵ and R⁶: form together a C₃-alkylene group; and
- R⁷ is: hydrogen or C₁-C₄-alkyl.

In an even more preferred embodiment,
- R¹ and R²,: together with the nitrogen atom they are bound to, form a 5-membered saturated heterocyclic ring which may carry one or more substituents selected from the group consisting of halogen and C₁-C₄-alkyl;
- R³: is hydrogen or halogen;
- R⁴: is halogen;
- R⁵: is hydrogen;
- R⁶: is C₁-C₄-alkyl, C₂-C₄-alkenyl or C₁-C₄-alkoxy; or
- R⁵ and R⁶: form together a C₃-alkylene group; and
- R⁷: is hydrogen or C₁-C₄-alkyl.

In a particular embodiment,
- R¹ and R²: , independently of each other, are C₁-C₃-alkyl; or
- R¹ and R²,: together with the nitrogen atom they are bound to, form a 5-membered saturated heterocyclic ring which may carry one or two substituents selected from the group consisting of halogen and C₁-C₄-alkyl, preferably from fluorine and methyl; where in particular R¹ and R² form together a group -(CH₂)₄-, -(CH₂-CHF-CH₂-CH₂)-, -(CH₂-CF₂-CH₂-CH₂)- or -(CH₂-CH(CH₃)-CH₂-CH₂)-;
- R³: is hydrogen or fluorine;
- R⁴: is chlorine;
- R⁵: is hydrogen;
- R⁶: is C₁-C₃-alkyl, C₂-C₃-alkenyl or C₁-C₁-alkoxy; or
- R⁵ and R⁶: form together a C₃-alkylene group; and
- R⁷: is hydrogen or C₁-C₃-alkyl.

In a specific embodiment,
- R¹ and R²,: together with the nitrogen atom they are bound to, form a 5-membered saturated heterocyclic ring which may carry one or two substituents selected from the group consisting of halogen and C₁-C₄-alkyl, preferably from fluorine and methyl; where in particular R¹ and R² form together a group -(CH₂)₄-, -(CH₂-CHF-CH₂-CH₂)-, -(CH₂-CF₂-CH₂-CH₂)- or -(CH₂-CH(CH₃)-CH₂-CH₂)-;
- R³: is hydrogen or fluorine;
- R⁴: is chlorine;
- R⁵: is hydrogen;
- R⁶: is C₁-C₃-alkyl, C₂-C₃-alkenyl or C₁-C₁-alkoxy; or
- R⁵ and R⁶: form together a C₃-alkylene group; and
- R⁷: is hydrogen or C₁-C₃-alkyl.

Particularly preferred are compounds (I.1) in which for a single compound R³, R⁴, R⁵, R⁶ and R⁷ have one of the meanings as defined in a single line of table A

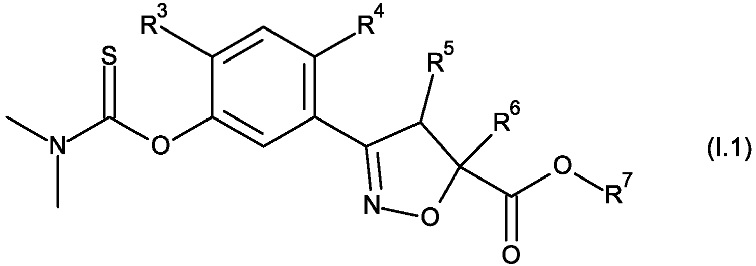

Particularly preferred are moreover compounds (I.2) in which for a single compound R³, R⁴, R⁵, R⁶ and R⁷ have one of the meanings as defined in a single line of table A

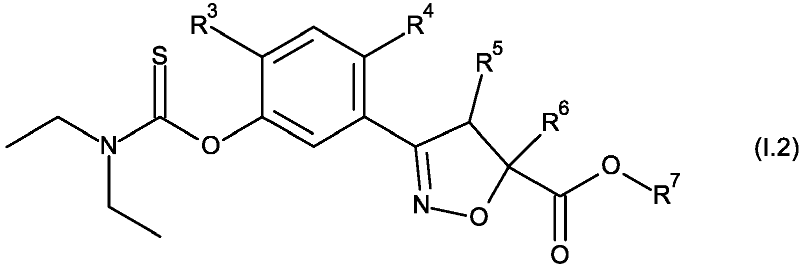

Particularly preferred are moreover compounds (I.3) in which for a single compound R³, R⁴, R⁵, R⁶ and R⁷ have one of the meanings as defined in a single line of table A

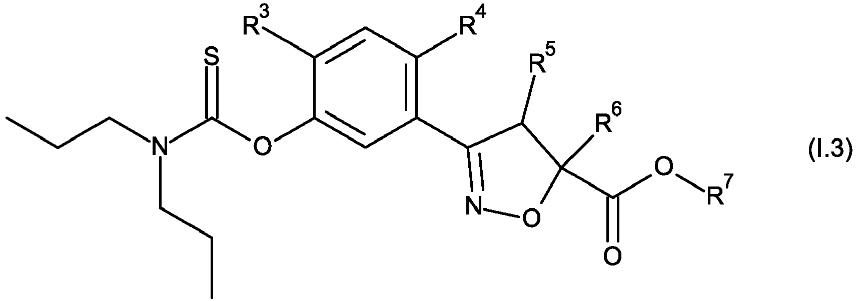

Particularly preferred are moreover compounds (I.4) in which for a single compound R³, R⁴, R⁵, R⁶ and R⁷ have one of the meanings as defined in a single line of table A

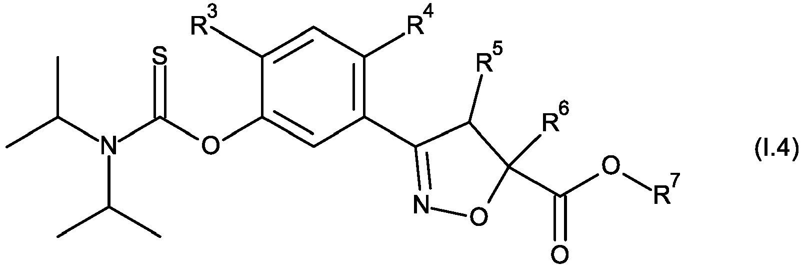

Particularly preferred are moreover compounds (I.5) in which for a single compound R³, R⁴, R⁵, R⁶ and R⁷ have one of the meanings as defined in a single line of table A

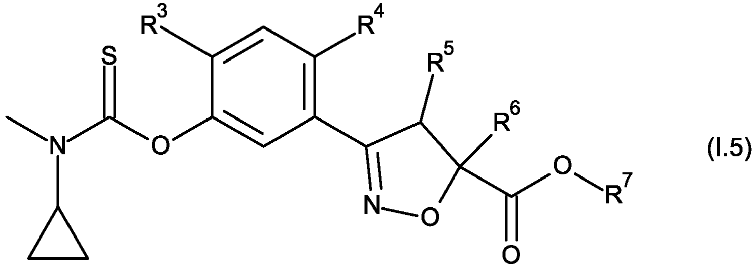

Particularly preferred are moreover compounds (I.6) in which for a single compound R³, R⁴, R⁵, R⁶ and R⁷ have one of the meanings as defined in a single line of table A

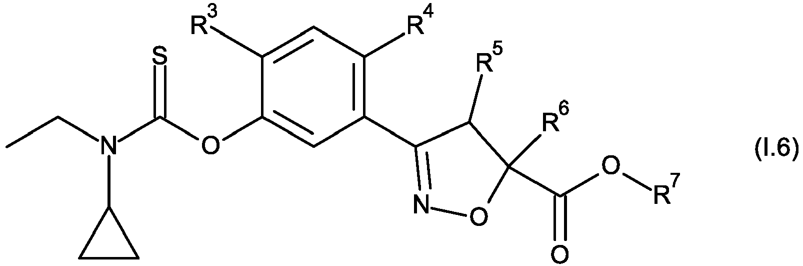

Particularly preferred are moreover compounds (I.7) in which for a single compound R³, R⁴, R⁵, R⁶ and R⁷ have one of the meanings as defined in a single line of table A

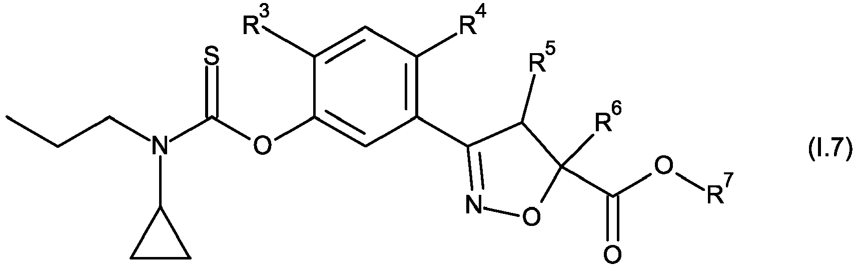

Particularly preferred are moreover compounds (I.8) in which for a single compound R³, R⁴, R⁵, R⁶ and R⁷ have one of the meanings as defined in a single line of table A

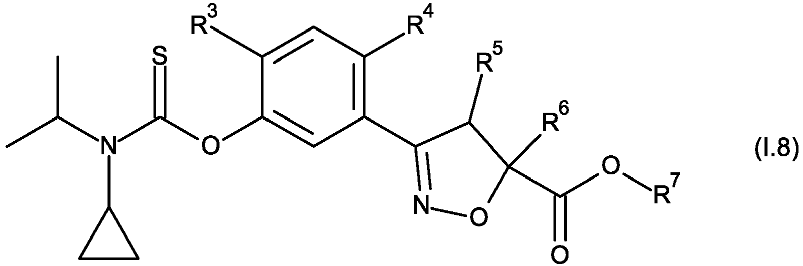

Particularly preferred are moreover compounds (I.9) in which for a single compound R³, R⁴, R⁵, R⁶ and R⁷ have one of the meanings as defined in a single line of table A

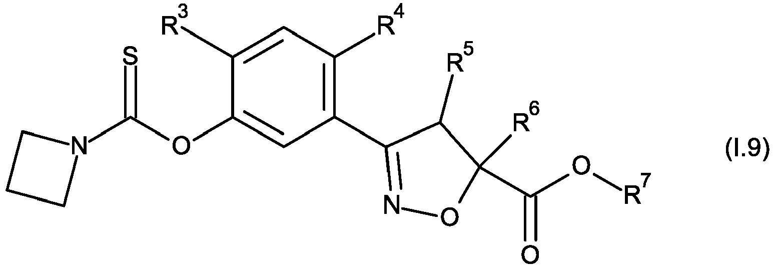

Particularly preferred are moreover compounds (!.10) in which for a single compound R³, R⁴, R⁵, R⁶ and R⁷ have one of the meanings as defined in a single line of table A

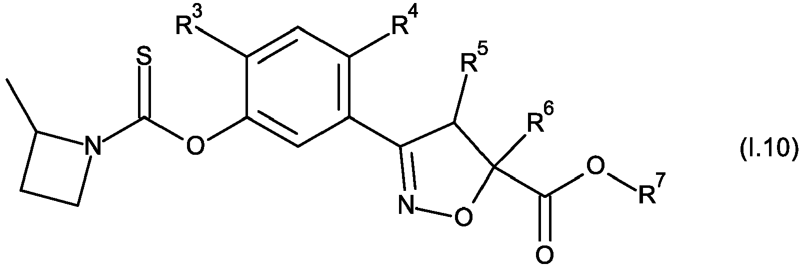

Particularly preferred are moreover compounds (!.11) in which for a single compound R³, R⁴, R⁵, R⁶ and R⁷ have one of the meanings as defined in a single line of table A

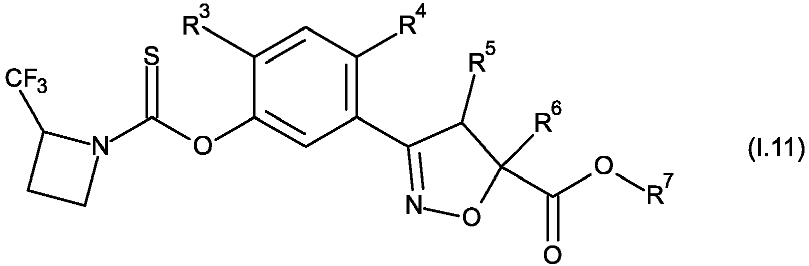

Particularly preferred are moreover compounds (1.12) in which for a single compound R³, R⁴, R⁵, R⁶ and R⁷ have one of the meanings as defined in a single line of table A

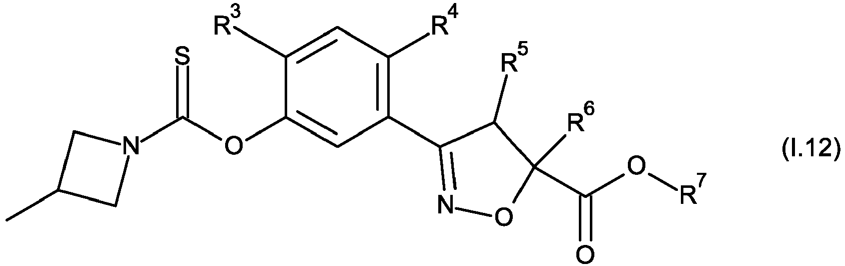

Particularly preferred are moreover compounds (1.13) in which for a single compound R³, R⁴, R⁵, R⁶ and R⁷ have one of the meanings as defined in a single line of table A

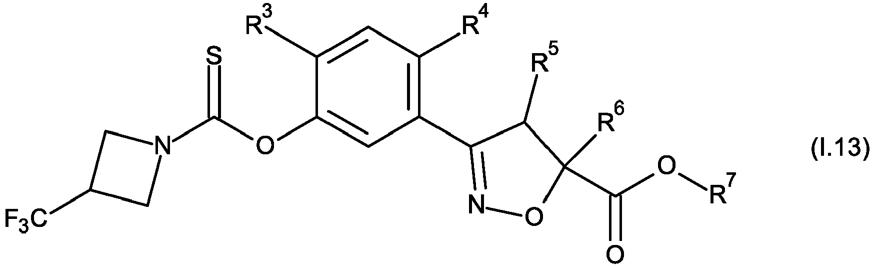

Particularly preferred are moreover compounds (1.14) in which for a single compound R³, R⁴, R⁵, R⁶ and R⁷ have one of the meanings as defined in a single line of table A

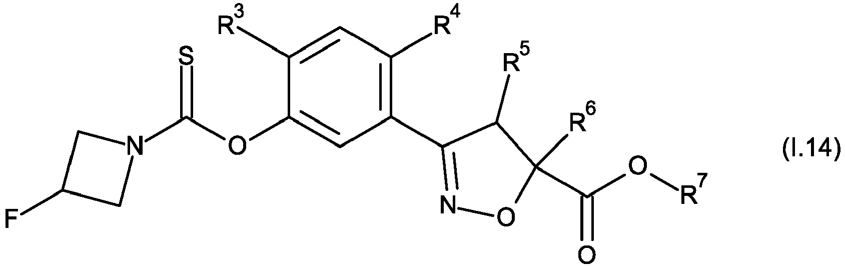

Particularly preferred are moreover compounds (1.15) in which for a single compound R³, R⁴, R⁵, R⁶ and R⁷ have one of the meanings as defined in a single line of table A

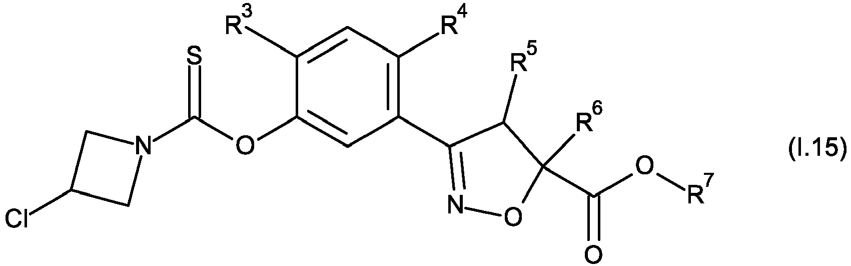

Particularly preferred are moreover compounds (1.16) in which for a single compound R³, R⁴, R⁵, R⁶ and R⁷ have one of the meanings as defined in a single line of table A

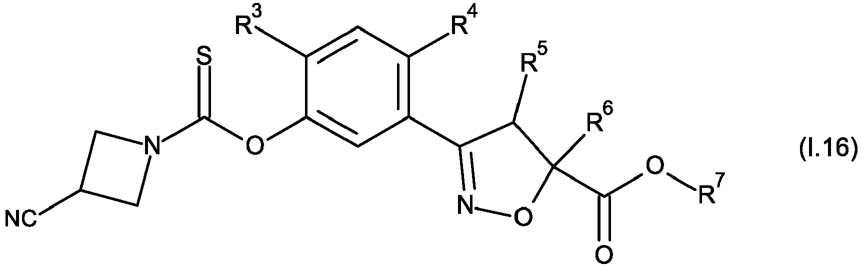

Particularly preferred are moreover compounds (1.17) in which for a single compound R³, R⁴, R⁵, R⁶ and R⁷ have one of the meanings as defined in a single line of table A

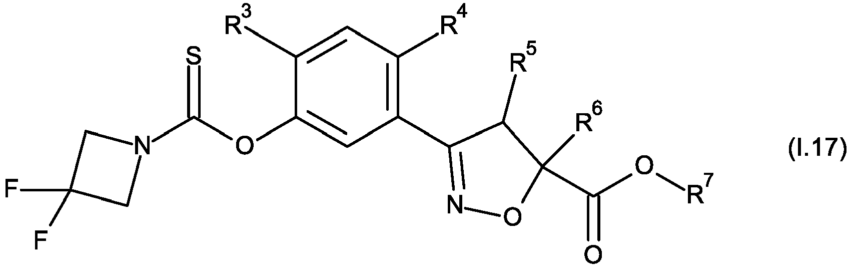

Particularly preferred are moreover compounds (1.18) in which for a single compound R³, R⁴, R⁵, R⁶ and R⁷ have one of the meanings as defined in a single line of table A

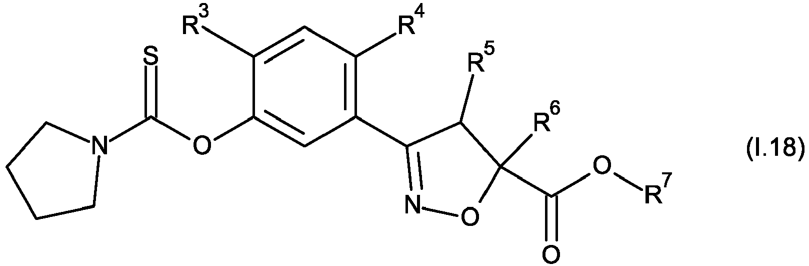

Particularly preferred are moreover compounds (1.19) in which for a single compound R³, R⁴, R⁵, R⁶ and R⁷ have one of the meanings as defined in a single line of table A

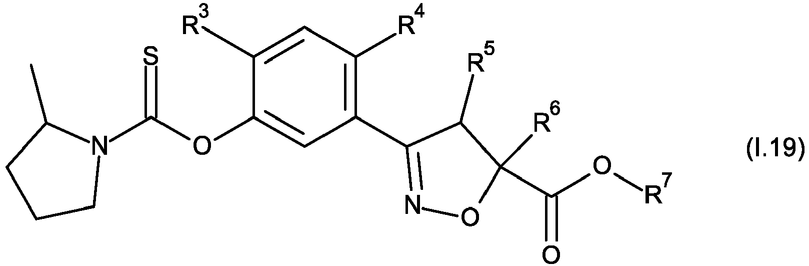

Particularly preferred are moreover compounds (I.20) in which for a single compound R³, R⁴, R⁵, R⁶ and R⁷ have one of the meanings as defined in a single line of table A

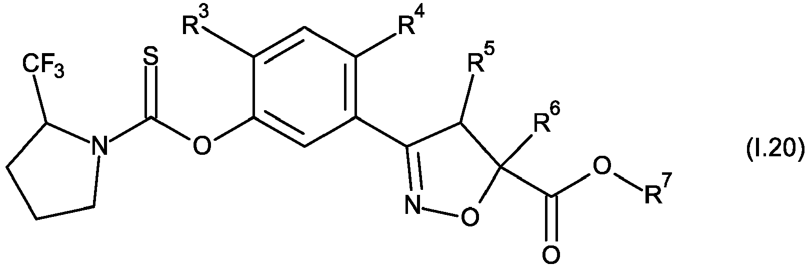

Particularly preferred are moreover compounds (I.21) in which for a single compound R³, R⁴, R⁵, R⁶ and R⁷ have one of the meanings as defined in a single line of table A

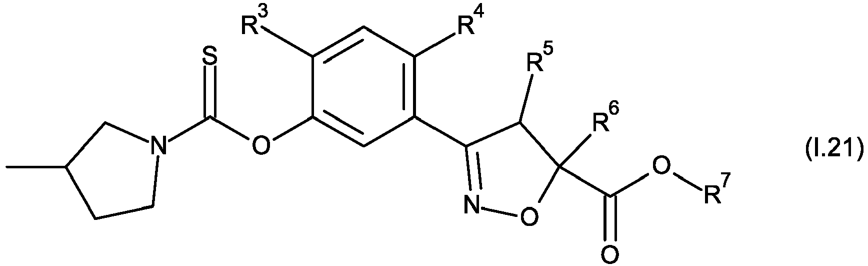

Particularly preferred are moreover compounds (I.22) in which for a single compound R³, R⁴, R⁵, R⁶ and R⁷ have one of the meanings as defined in a single line of table A

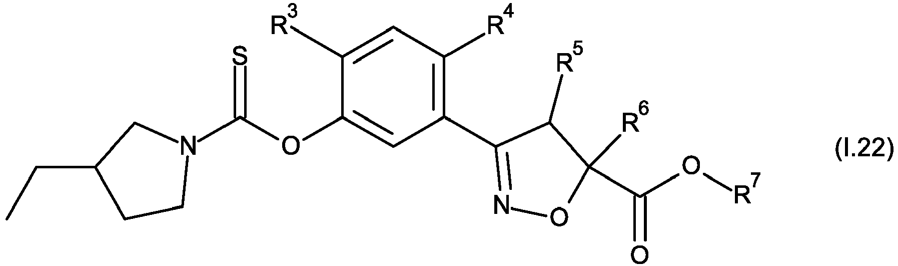

Particularly preferred are moreover compounds (I.23) in which for a single compound R³, R⁴, R⁵, R⁶ and R⁷ have one of the meanings as defined in a single line of table A

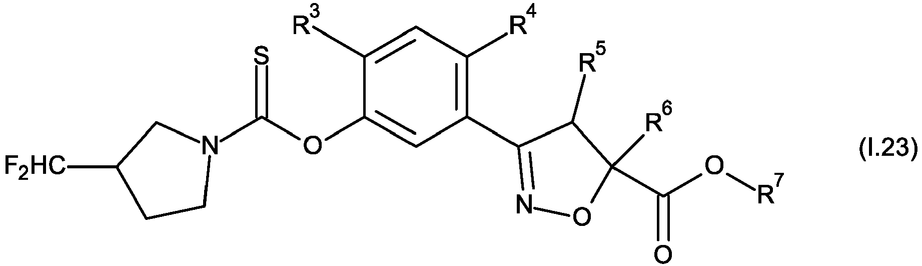

Particularly preferred are moreover compounds (I.24) in which for a single compound R³, R⁴, R⁵, R⁶ and R⁷ have one of the meanings as defined in a single line of table A

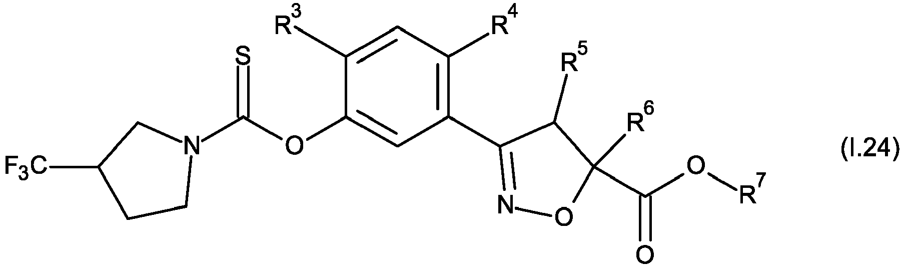

Particularly preferred are moreover compounds (I.25) in which for a single compound R³, R⁴, R⁵, R⁶ and R⁷ have one of the meanings as defined in a single line of table A

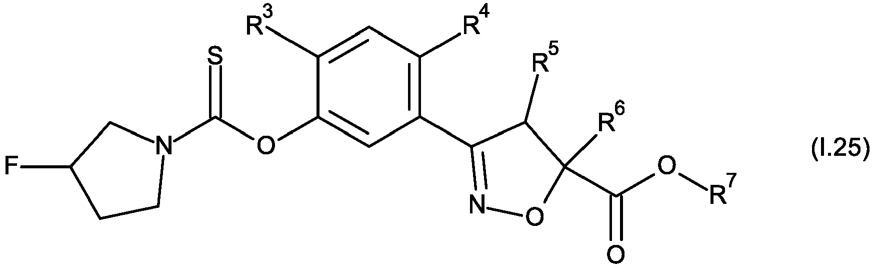

Particularly preferred are moreover compounds (I.26) in which for a single compound R³, R⁴, R⁵, R⁶ and R⁷ have one of the meanings as defined in a single line of table A

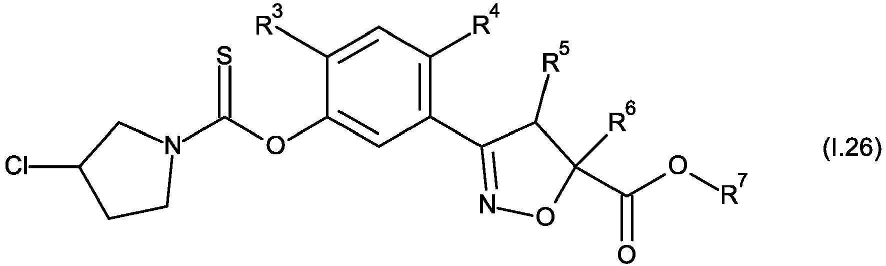

Particularly preferred are moreover compounds (I.27) in which for a single compound R³, R⁴, R⁵, R⁶ and R⁷ have one of the meanings as defined in a single line of table A

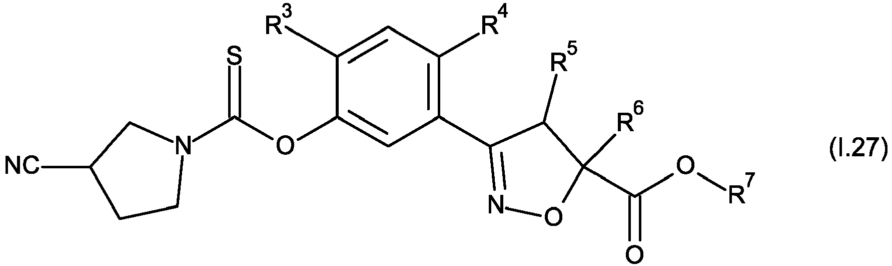

Particularly preferred are moreover compounds (I.28) in which for a single compound R³, R⁴, R⁵, R⁶ and R⁷ have one of the meanings as defined in a single line of table A

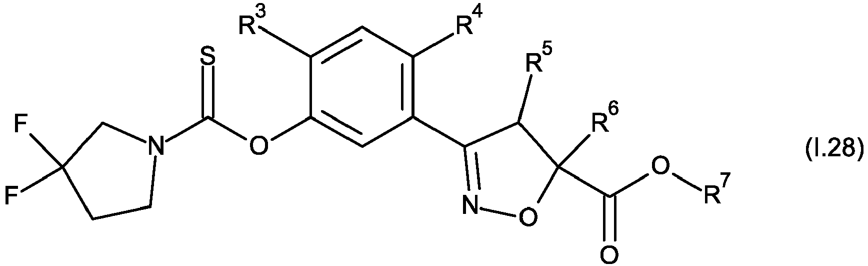

Particularly preferred are moreover compounds (I.29) in which for a single compound R³, R⁴, R⁵, R⁶ and R⁷ have one of the meanings as defined in a single line of table A

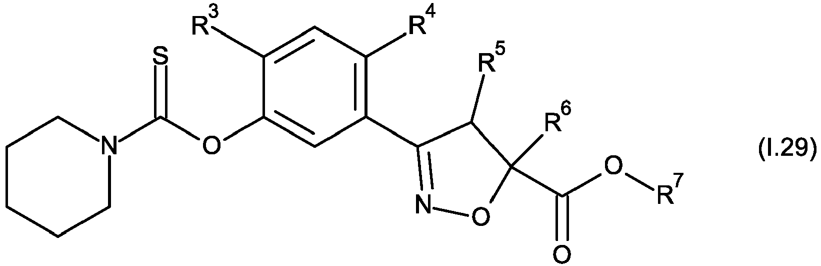

Particularly preferred are moreover compounds (I.30) in which for a single compound R³, R⁴, R⁵, R⁶ and R⁷ have one of the meanings as defined in a single line of table A

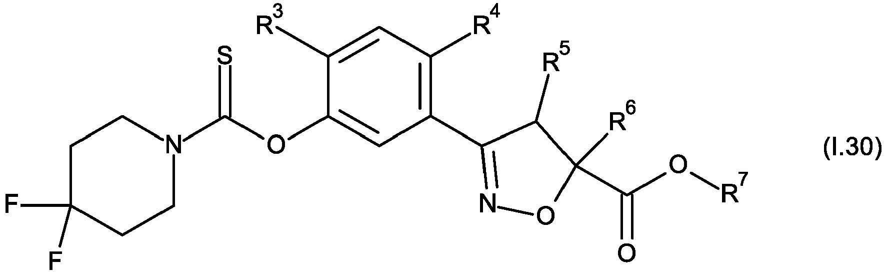

**Table A**

| No. | R³ | R⁴ | R⁵ | R⁶ | R⁷ |
|---|---|---|---|---|---|
| A.1. | H | H | H | Me | H |
| A.2. | H | F | H | Me | H |
| A.3. | H | Cl | H | Me | H |
| A.4. | H | Br | H | Me | H |
| A.5. | H | CN | H | Me | H |
| A.6. | H | NO₂ | H | Me | H |
| A.7. | F | H | H | Me | H |
| A.8. | Cl | H | H | Me | H |
| A.9. | Br | H | H | Me | H |
| A.10. | CN | H | H | Me | H |
| A.11. | NOz | H | H | Me | H |
| A.12. | F | F | H | Me | H |
| A.13. | F | Cl | H | Me | H |
| A.14. | F | Br | H | Me | H |
| A.15. | F | CN | H | Me | H |
| A.16. | F | NO₂ | H | Me | H |
| A.17. | Cl | F | H | Me | H |
| A.18. | Cl | Cl | H | Me | H |
| A.19. | Cl | Br | H | Me | H |
| A.20. | Cl | CN | H | Me | H |
| A.21. | Cl | NO₂ | H | Me | H |
| A.22. | H | H | H | Et | H |
| A.23. | H | F | H | Et | H |
| A.24. | H | Cl | H | Et | H |
| A.25. | H | Br | H | Et | H |
| A.26. | H | CN | H | Et | H |
| A.27. | H | NO₂ | H | Et | H |
| A.28. | F | H | H | Et | H |
| A.29. | Cl | H | H | Et | H |
| A.30. | Br | H | H | Et | H |
| A.31. | CN | H | H | Et | H |
| A.32. | NOz | H | H | Et | H |
| A.33. | F | F | H | Et | H |
| A.34. | F | Cl | H | Et | H |
| A.35. | F | Br | H | Et | H |
| A.36. | F | CN | H | Et | H |
| A.37. | F | NO₂ | H | Et | H |
| A.38. | Cl | F | H | Et | H |
| A.39. | Cl | Cl | H | Et | H |
| A.40. | Cl | Br | H | Et | H |
| A.41. | Cl | CN | H | Et | H |
| A.42. | Cl | NO₂ | H | Et | H |
| A.43. | H | H | H | nPr | H |
| A.44. | H | F | H | nPr | H |
| A.45. | H | Cl | H | nPr | H |
| A.46. | H | Br | H | nPr | H |
| A.47. | H | CN | H | nPr | H |
| A.48. | H | NO₂ | H | nPr | H |
| A.49. | F | H | H | nPr | H |
| A.50. | Cl | H | H | nPr | H |
| A.51. | Br | H | H | nPr | H |
| A.52. | CN | H | H | nPr | H |
| A.53. | NOz | H | H | nPr | H |
| A.54. | F | F | H | nPr | H |
| A.55. | F | Cl | H | nPr | H |
| A.56. | F | Br | H | nPr | H |
| A.57. | F | CN | H | nPr | H |
| A.58. | F | NO₂ | H | nPr | H |
| A.59. | Cl | F | H | nPr | H |
| A.60. | Cl | Cl | H | nPr | H |
| A.61. | Cl | Br | H | nPr | H |
| A.62. | Cl | CN | H | nPr | H |
| A.63. | Cl | NO₂ | H | nPr | H |
| A.64. | H | H | H | iPr | H |
| A.65. | H | F | H | iPr | H |
| A.66. | H | Cl | H | iPr | H |
| A.67. | H | Br | H | iPr | H |
| A.68. | H | CN | H | iPr | H |
| A.69. | H | NO₂ | H | iPr | H |
| A.70. | F | H | H | iPr | H |
| A.71. | Cl | H | H | iPr | H |
| A.72. | Br | H | H | iPr | H |
| A.73. | CN | H | H | iPr | H |
| A.74. | NOz | H | H | iPr | H |
| A.75. | F | F | H | iPr | H |
| A.76. | F | Cl | H | iPr | H |
| A.77. | F | Br | H | iPr | H |
| A.78. | F | CN | H | iPr | H |
| A.79. | F | NO₂ | H | iPr | H |
| A.80. | Cl | F | H | iPr | H |
| A.81. | Cl | Cl | H | iPr | H |
| A.82. | Cl | Br | H | iPr | H |
| A.83. | Cl | CN | H | iPr | H |
| A.84. | Cl | NO₂ | H | iPr | H |
| A.85. | H | H | H | vinyl | H |
| A.86. | H | F | H | vinyl | H |
| A.87. | H | Cl | H | vinyl | H |
| A.88. | H | Br | H | vinyl | H |
| A.89. | H | CN | H | vinyl | H |
| A.90. | H | NO₂ | H | vinyl | H |
| A.91. | F | H | H | vinyl | H |
| A.92. | Cl | H | H | vinyl | H |
| A.93. | Br | H | H | vinyl | H |
| A.94. | CN | H | H | vinyl | H |
| A.95. | NOz | H | H | vinyl | H |
| A.96. | F | F | H | vinyl | H |
| A.97. | F | Cl | H | vinyl | H |
| A.98. | F | Br | H | vinyl | H |
| A.99. | F | CN | H | vinyl | H |
| A.100. | F | NO₂ | H | vinyl | H |
| A.101. | Cl | F | H | vinyl | H |
| A.102. | Cl | Cl | H | vinyl | H |
| A.103. | Cl | Br | H | vinyl | H |
| A.104. | Cl | CN | H | vinyl | H |
| A.105. | Cl | NO₂ | H | vinyl | H |
| A.106. | H | H | H | allyl | H |
| A.107. | H | F | H | allyl | H |
| A.108. | H | Cl | H | allyl | H |
| A.109. | H | Br | H | allyl | H |
| A.110. | H | CN | H | allyl | H |
| A.111. | H | NO₂ | H | allyl | H |
| A.112. | F | H | H | allyl | H |
| A.113. | Cl | H | H | allyl | H |
| A.114. | Br | H | H | allyl | H |
| A.115. | CN | H | H | allyl | H |
| A.116. | NOz | H | H | allyl | H |
| A.117. | F | F | H | allyl | H |
| A.118. | F | Cl | H | allyl | H |
| A.119. | F | Br | H | allyl | H |
| A.120. | F | CN | H | allyl | H |
| A.121. | F | NO₂ | H | allyl | H |
| A.122. | Cl | F | H | allyl | H |
| A.123. | Cl | Cl | H | allyl | H |
| A.124. | Cl | Br | H | allyl | H |
| A.125. | Cl | CN | H | allyl | H |
| A.126. | Cl | NO₂ | H | allyl | H |
| A.127. | H | H | H | OMe | H |
| A.128. | H | F | H | OMe | H |
| A.129. | H | Cl | H | OMe | H |
| A.130. | H | Br | H | OMe | H |
| A.131. | H | CN | H | OMe | H |
| A.132. | H | NO₂ | H | OMe | H |
| A.133. | F | H | H | OMe | H |
| A.134. | Cl | H | H | OMe | H |
| A.135. | Br | H | H | OMe | H |
| A.136. | CN | H | H | OMe | H |
| A.137. | NOz | H | H | OMe | H |
| A.138. | F | F | H | OMe | H |
| A.139. | F | Cl | H | OMe | H |
| A.140. | F | Br | H | OMe | H |
| A.141. | F | CN | H | OMe | H |
| A.142. | F | NO₂ | H | OMe | H |
| A.143. | Cl | F | H | OMe | H |
| A.144. | Cl | Cl | H | OMe | H |
| A.145. | Cl | Br | H | OMe | H |
| A.146. | Cl | CN | H | OMe | H |
| A.147. | Cl | NO₂ | H | OMe | H |
| A.148. | H | H | H | OEt | H |
| A.149. | H | F | H | OEt | H |
| A.150. | H | Cl | H | OEt | H |
| A.151. | H | Br | H | OEt | H |
| A.152. | H | CN | H | OEt | H |
| A.153. | H | NO₂ | H | OEt | H |
| A.154. | F | H | H | OEt | H |
| A.155. | Cl | H | H | OEt | H |
| A.156. | Br | H | H | OEt | H |
| A.157. | CN | H | H | OEt | H |
| A.158. | NOz | H | H | OEt | H |
| A.159. | F | F | H | OEt | H |
| A.160. | F | Cl | H | OEt | H |
| A.161. | F | Br | H | OEt | H |
| A.162. | F | CN | H | OEt | H |
| A.163. | F | NO₂ | H | OEt | H |
| A.164. | Cl | F | H | OEt | H |
| A.165. | Cl | Cl | H | OEt | H |
| A.166. | Cl | Br | H | OEt | H |
| A.167. | Cl | CN | H | OEt | H |
| A.168. | Cl | NO₂ | H | OEt | H |
| A.169. | H | H | H | Me | Me |
| A.170. | H | F | H | Me | Me |
| A.171. | H | Cl | H | Me | Me |
| A.172. | H | Br | H | Me | Me |
| A.173. | H | CN | H | Me | Me |
| A.174. | H | NO₂ | H | Me | Me |
| A.175. | F | H | H | Me | Me |
| A.176. | Cl | H | H | Me | Me |
| A.177. | Br | H | H | Me | Me |
| A.178. | CN | H | H | Me | Me |
| A.179. | NO₂ | H | H | Me | Me |
| A.180. | F | F | H | Me | Me |
| A.181. | F | Cl | H | Me | Me |
| A.182. | F | Br | H | Me | Me |
| A.183. | F | CN | H | Me | Me |
| A.184. | F | NO₂ | H | Me | Me |
| A.185. | Cl | F | H | Me | Me |
| A.186. | Cl | Cl | H | Me | Me |
| A.187. | Cl | Br | H | Me | Me |
| A.188. | Cl | CN | H | Me | Me |
| A.189. | Cl | NO₂ | H | Me | Me |
| A.190. | H | H | H | Et | Me |
| A.191. | H | F | H | Et | Me |
| A.192. | H | Cl | H | Et | Me |
| A.193. | H | Br | H | Et | Me |
| A.194. | H | CN | H | Et | Me |
| A.195. | H | NO₂ | H | Et | Me |
| A.196. | F | H | H | Et | Me |
| A.197. | Cl | H | H | Et | Me |
| A.198. | Br | H | H | Et | Me |
| A.199. | CN | H | H | Et | Me |
| A.200. | NO₂ | H | H | Et | Me |
| A.201. | F | F | H | Et | Me |
| A.202. | F | Cl | H | Et | Me |
| A.203. | F | Br | H | Et | Me |
| A.204. | F | CN | H | Et | Me |
| A.205. | F | NO₂ | H | Et | Me |
| A.206. | Cl | F | H | Et | Me |
| A.207. | Cl | Cl | H | Et | Me |
| A.208. | Cl | Br | H | Et | Me |
| A.209. | Cl | CN | H | Et | Me |
| A.210. | Cl | NO₂ | H | Et | Me |
| A.211. | H | H | H | nPr | Me |
| A.212. | H | F | H | nPr | Me |
| A.213. | H | Cl | H | nPr | Me |
| A.214. | H | Br | H | nPr | Me |
| A.215. | H | CN | H | nPr | Me |
| A.216. | H | NO₂ | H | nPr | Me |
| A.217. | F | H | H | nPr | Me |
| A.218. | Cl | H | H | nPr | Me |
| A.219. | Br | H | H | nPr | Me |
| A.220. | CN | H | H | nPr | Me |
| A.221. | NO₂ | H | H | nPr | Me |
| A.222. | F | F | H | nPr | Me |
| A.223. | F | Cl | H | nPr | Me |
| A.224. | F | Br | H | nPr | Me |
| A.225. | F | CN | H | nPr | Me |
| A.226. | F | NO₂ | H | nPr | Me |
| A.227. | Cl | F | H | nPr | Me |
| A.228. | Cl | Cl | H | nPr | Me |
| A.229. | Cl | Br | H | nPr | Me |
| A.230. | Cl | CN | H | nPr | Me |
| A.231. | Cl | NO₂ | H | nPr | Me |
| A.232. | H | H | H | iPr | Me |
| A.233. | H | F | H | iPr | Me |
| A.234. | H | Cl | H | iPr | Me |
| A.235. | H | Br | H | iPr | Me |
| A.236. | H | CN | H | iPr | Me |
| A.237. | H | NO₂ | H | iPr | Me |
| A.238. | F | H | H | iPr | Me |
| A.239. | Cl | H | H | iPr | Me |
| A.240. | Br | H | H | iPr | Me |
| A.241. | CN | H | H | iPr | Me |
| A.242. | NO₂ | H | H | iPr | Me |
| A.243. y | F | F | H | iPr | Me |
| A.244. | F | Cl | H | iPr | Me |
| A.245. | F | Br | H | iPr | Me |
| A.246. | F | CN | H | iPr | Me |
| A.247. | F | NO₂ | H | iPr | Me |
| A.248. | Cl | F | H | iPr | Me |
| A.249. | Cl | Cl | H | iPr | Me |
| A.250. | Cl | Br | H | iPr | Me |
| A.251. | Cl | CN | H | iPr | Me |
| A.252. | Cl | NO₂ | H | iPr | Me |
| A.253. | H | H | H | vinyl | Me |
| A.254. | H | F | H | vinyl | Me |
| A.255. | H | Cl | H | vinyl | Me |
| A.256. | H | Br | H | vinyl | Me |
| A.257. | H | CN | H | vinyl | Me |
| A.258. | H | NO₂ | H | vinyl | Me |
| A.259. | F | H | H | vinyl | Me |
| A.260. | Cl | H | H | vinyl | Me |
| A.261. | Br | H | H | vinyl | Me |
| A.262. | CN | H | H | vinyl | Me |
| A.263. | NO₂ | H | H | vinyl | Me |
| A.264. | F | F | H | vinyl | Me |
| A.265. | F | Cl | H | vinyl | Me |
| A.266. | F | Br | H | vinyl | Me |
| A.267. | F | CN | H | vinyl | Me |
| A.268. | F | NO₂ | H | vinyl | Me |
| A.269. | Cl | F | H | vinyl | Me |
| A.270. | Cl | Cl | H | vinyl | Me |
| A.271. | Cl | Br | H | vinyl | Me |
| A.272. | Cl | CN | H | vinyl | Me |
| A.273. | Cl | NO₂ | H | vinyl | Me |
| A.274. | H | H | H | allyl | Me |
| A.275. | H | F | H | allyl | Me |
| A.276. | H | Cl | H | allyl | Me |
| A.277. | H | Br | H | allyl | Me |
| A.278. | H | CN | H | allyl | Me |
| A.279. | H | NO₂ | H | allyl | Me |
| A.280. | F | H | H | allyl | Me |
| A.281. | Cl | H | H | allyl | Me |
| A.282. | Br | H | H | allyl | Me |
| A.283. | CN | H | H | allyl | Me |
| A.284. | NO₂ | H | H | allyl | Me |
| A.285. | F | F | H | allyl | Me |
| A.286. | F | Cl | H | allyl | Me |
| A.287. | F | Br | H | allyl | Me |
| A.288. | F | CN | H | allyl | Me |
| A.289. | F | NO₂ | H | allyl | Me |
| A.290. | Cl | F | H | allyl | Me |
| A.291. | Cl | Cl | H | allyl | Me |
| A.292. | Cl | Br | H | allyl | Me |
| A.293. | Cl | CN | H | allyl | Me |
| A.294. | Cl | NO₂ | H | allyl | Me |
| A.295. | H | H | H | OMe | Me |
| A.296. | H | F | H | OMe | Me |
| A.297. | H | Cl | H | OMe | Me |
| A.298. | H | Br | H | OMe | Me |
| A.299. | H | CN | H | OMe | Me |
| A.300. | H | NO₂ | H | OMe | Me |
| A.301. | F | H | H | OMe | Me |
| A.302. | Cl | H | H | OMe | Me |
| A.303. | Br | H | H | OMe | Me |
| A.304. | CN | H | H | OMe | Me |
| A.305. | NO₂ | H | H | OMe | Me |
| A.306. | F | F | H | OMe | Me |
| A.307. | F | Cl | H | OMe | Me |
| A.308. | F | Br | H | OMe | Me |
| A.309. | F | CN | H | OMe | Me |
| A.310. | F | NO₂ | H | OMe | Me |
| A.311. | Cl | F | H | OMe | Me |
| A.312. | Cl | Cl | H | OMe | Me |
| A.313. | Cl | Br | H | OMe | Me |
| A.314. | Cl | CN | H | OMe | Me |
| A.315. | Cl | NO₂ | H | OMe | Me |
| A.316. | H | H | H | OEt | Me |
| A.317. | H | F | H | OEt | Me |
| A.318. | H | Cl | H | OEt | Me |
| A.319. | H | Br | H | OEt | Me |
| A.320. | H | CN | H | OEt | Me |
| A.321. | H | NO₂ | H | OEt | Me |
| A.322. | F | H | H | OEt | Me |
| A.323. | Cl | H | H | OEt | Me |
| A.324. | Br | H | H | OEt | Me |
| A.325. | CN | H | H | OEt | Me |
| A.326. | NO₂ | H | H | OEt | Me |
| A.327. | F | F | H | OEt | Me |
| A.328. | F | Cl | H | OEt | Me |
| A.329. | F | Br | H | OEt | Me |
| A.330. | F | CN | H | OEt | Me |
| A.331. | F | NO₂ | H | OEt | Me |
| A.332. | Cl | F | H | OEt | Me |
| A.333. | Cl | Cl | H | OEt | Me |
| A.334. | Cl | Br | H | OEt | Me |
| A.335. | Cl | CN | H | OEt | Me |
| A.336. | Cl | NO₂ | H | OEt | Me |
| A.337. | H | H | H | Me | Et |
| A.338. | H | F | H | Me | Et |
| A.339. | H | Cl | H | Me | Et |
| A.340. | H | Br | H | Me | Et |
| A.341. | H | CN | H | Me | Et |
| A.342. | H | NO₂ | H | Me | Et |
| A.343. | F | H | H | Me | Et |
| A.344. | Cl | H | H | Me | Et |
| A.345. | Br | H | H | Me | Et |
| A.346. | CN | H | H | Me | Et |
| A.347. | NO₂ | H | H | Me | Et |
| A.348. | F | F | H | Me | Et |
| A.349. | F | Cl | H | Me | Et |
| A.350. | F | Br | H | Me | Et |
| A.351. | F | CN | H | Me | Et |
| A.352. | F | NO₂ | H | Me | Et |
| A.353. | Cl | F | H | Me | Et |
| A.354. | Cl | Cl | H | Me | Et |
| A.355. | Cl | Br | H | Me | Et |
| A.356. | Cl | CN | H | Me | Et |
| A.357. | Cl | NO₂ | H | Me | Et |
| A.358. | H | H | H | Et | Et |
| A.359. | H | F | H | Et | Et |
| A.360. | H | Cl | H | Et | Et |
| A.361. | H | Br | H | Et | Et |
| A.362. | H | CN | H | Et | Et |
| A.363. | H | NO₂ | H | Et | Et |
| A.364. | F | H | H | Et | Et |
| A.365. | Cl | H | H | Et | Et |
| A.366. | Br | H | H | Et | Et |
| A.367. | CN | H | H | Et | Et |
| A.368. | NO₂ | H | H | Et | Et |
| A.369. | F | F | H | Et | Et |
| A.370. | F | Cl | H | Et | Et |
| A.371. | F | Br | H | Et | Et |
| A.372. | F | CN | H | Et | Et |
| A.373. | F | NO₂ | H | Et | Et |
| A.374. | Cl | F | H | Et | Et |
| A.375. | Cl | Cl | H | Et | Et |
| A.376. | Cl | Br | H | Et | Et |
| A.377. | Cl | CN | H | Et | Et |
| A.378. | Cl | NO₂ | H | Et | Et |
| A.379. | H | H | H | nPr | Et |
| A.380. | H | F | H | nPr | Et |
| A.381. | H | Cl | H | nPr | Et |
| A.382. | H | Br | H | nPr | Et |
| A.383. | H | CN | H | nPr | Et |
| A.384. | H | NO₂ | H | nPr | Et |
| A.385. | F | H | H | nPr | Et |
| A.386. | Cl | H | H | nPr | Et |
| A.387. | Br | H | H | nPr | Et |
| A.388. | CN | H | H | nPr | Et |
| A.389. | NO₂ | H | H | nPr | Et |
| A.390. | F | F | H | nPr | Et |
| A.391. | F | Cl | H | nPr | Et |
| A.392. | F | Br | H | nPr | Et |
| A.393. | F | CN | H | nPr | Et |
| A.394. | F | NO₂ | H | nPr | Et |
| A.395. | Cl | F | H | nPr | Et |
| A.396. | Cl | Cl | H | nPr | Et |
| A.397. | Cl | Br | H | nPr | Et |
| A.398. | Cl | CN | H | nPr | Et |
| A.399. | Cl | NO₂ | H | nPr | Et |
| A.400. | H | H | H | iPr | Et |
| A.401. | H | F | H | iPr | Et |
| A.402. | H | Cl | H | iPr | Et |
| A.403. | H | Br | H | iPr | Et |
| A.404. | H | CN | H | iPr | Et |
| A.405. | H | NO₂ | H | iPr | Et |
| A.406. | F | H | H | iPr | Et |
| A.407. | Cl | H | H | iPr | Et |
| A.408. | Br | H | H | iPr | Et |
| A.409. | CN | H | H | iPr | Et |
| A.410. | NO₂ | H | H | iPr | Et |
| A.411. | F | F | H | iPr | Et |
| A.412. | F | Cl | H | iPr | Et |
| A.413. | F | Br | H | iPr | Et |
| A.414. | F | CN | H | iPr | Et |
| A.415. | F | NO₂ | H | iPr | Et |
| A.416. | Cl | F | H | iPr | Et |
| A.417. | Cl | Cl | H | iPr | Et |
| A.418. | Cl | Br | H | iPr | Et |
| A.419. | Cl | CN | H | iPr | Et |
| A.420. | Cl | NO₂ | H | iPr | Et |
| A.421. | H | H | H | vinyl | Et |
| A.422. | H | F | H | vinyl | Et |
| A.423. | H | Cl | H | vinyl | Et |
| A.424. | H | Br | H | vinyl | Et |
| A.425. | H | CN | H | vinyl | Et |
| A.426. | H | NO₂ | H | vinyl | Et |
| A.427. | F | H | H | vinyl | Et |
| A.428. | Cl | H | H | vinyl | Et |
| A.429. | Br | H | H | vinyl | Et |
| A.430. | CN | H | H | vinyl | Et |
| A.431. | NO₂ | H | H | vinyl | Et |
| A.432. | F | F | H | vinyl | Et |
| A.433. | F | Cl | H | vinyl | Et |
| A.434. | F | Br | H | vinyl | Et |
| A.435. | F | CN | H | vinyl | Et |
| A.436. | F | NO₂ | H | vinyl | Et |
| A.437. | Cl | F | H | vinyl | Et |
| A.438. | Cl | Cl | H | vinyl | Et |
| A.439. | Cl | Br | H | vinyl | Et |
| A.440. | Cl | CN | H | vinyl | Et |
| A.441. | Cl | NO₂ | H | vinyl | Et |
| A.442. | H | H | H | allyl | Et |
| A.443. | H | F | H | allyl | Et |
| A.444. | H | Cl | H | allyl | Et |
| A.445. | H | Br | H | allyl | Et |
| A.446. | H | CN | H | allyl | Et |
| A.447. | H | NO₂ | H | allyl | Et |
| A.448. | F | H | H | allyl | Et |
| A.449. | Cl | H | H | allyl | Et |
| A.450. | Br | H | H | allyl | Et |
| A.451. | CN | H | H | allyl | Et |
| A.452. | NO₂ | H | H | allyl | Et |
| A.453. | F | F | H | allyl | Et |
| A.454. | F | Cl | H | allyl | Et |
| A.455. | F | Br | H | allyl | Et |
| A.456. | F | CN | H | allyl | Et |
| A.457. | F | NO₂ | H | allyl | Et |
| A.458. | Cl | F | H | allyl | Et |
| A.459. | Cl | Cl | H | allyl | Et |
| A.460. | Cl | Br | H | allyl | Et |
| A.461. | Cl | CN | H | allyl | Et |
| A.462. | Cl | NO₂ | H | allyl | Et |
| A.463. | H | H | H | OMe | Et |
| A.464. | H | F | H | OMe | Et |
| A.465. | H | Cl | H | OMe | Et |
| A.466. | H | Br | H | OMe | Et |
| A.467. | H | CN | H | OMe | Et |
| A.468. | H | NO₂ | H | OMe | Et |
| A.469. | F | H | H | OMe | Et |
| A.470. | Cl | H | H | OMe | Et |
| A.471. | Br | H | H | OMe | Et |
| A.472. | CN | H | H | OMe | Et |
| A.473. | NO₂ | H | H | OMe | Et |
| A.474. | F | F | H | OMe | Et |
| A.475. | F | Cl | H | OMe | Et |
| A.476. | F | Br | H | OMe | Et |
| A.477. | F | CN | H | OMe | Et |
| A.478. | F | NO₂ | H | OMe | Et |
| A.479. | Cl | F | H | OMe | Et |
| A.480. | Cl | Cl | H | OMe | Et |
| A.481. | Cl | Br | H | OMe | Et |
| A.482. | Cl | CN | H | OMe | Et |
| A.483. | Cl | NO₂ | H | OMe | Et |
| A.484. | H | H | H | OEt | Et |
| A.485. | H | F | H | OEt | Et |
| A.486. | H | Cl | H | OEt | Et |
| A.487. | H | Br | H | OEt | Et |
| A.488. | H | CN | H | OEt | Et |
| A.489. | H | NO₂ | H | OEt | Et |
| A.490. | F | H | H | OEt | Et |
| A.491. | Cl | H | H | OEt | Et |
| A.492. | Br | H | H | OEt | Et |
| A.493. | CN | H | H | OEt | Et |
| A.494. | NO₂ | H | H | OEt | Et |
| A.495. | F | F | H | OEt | Et |
| A.496. | F | Cl | H | OEt | Et |
| A.497. | F | Br | H | OEt | Et |
| A.498. | F | CN | H | OEt | Et |
| A.499. | F | NO₂ | H | OEt | Et |
| A.500. | Cl | F | H | OEt | Et |
| A.501. | Cl | Cl | H | OEt | Et |
| A.502. | Cl | Br | H | OEt | Et |
| A.503. | Cl | CN | H | OEt | Et |
| A.504. | Cl | NO₂ | H | OEt | Et |
| A.505. | H | H | H | Me | nPr |
| A.506. | H | F | H | Me | nPr |
| A.507. | H | Cl | H | Me | nPr |
| A.508. | H | Br | H | Me | nPr |
| A.509. | H | CN | H | Me | nPr |
| A.510. | H | NO₂ | H | Me | nPr |
| A.511. | F | H | H | Me | nPr |
| A.512. | Cl | H | H | Me | nPr |
| A.513. | Br | H | H | Me | nPr |
| A.514. | CN | H | H | Me | nPr |
| A.515. | NO₂ | H | H | Me | nPr |
| A.516. | F | F | H | Me | nPr |
| A.517. | F | Cl | H | Me | nPr |
| A.518. | F | Br | H | Me | nPr |
| A.519. | F | CN | H | Me | nPr |
| A.520. | F | NO₂ | H | Me | nPr |
| A.521. | Cl | F | H | Me | nPr |
| A.522. | Cl | Cl | H | Me | nPr |
| A.523. | Cl | Br | H | Me | nPr |
| A.524. | Cl | CN | H | Me | nPr |
| A.525. | Cl | NO₂ | H | Me | nPr |
| A.526. | H | H | H | Et | nPr |
| A.527. | H | F | H | Et | nPr |
| A.528. | H | Cl | H | Et | nPr |
| A.529. | H | Br | H | Et | nPr |
| A.530. | H | CN | H | Et | nPr |
| A.531. | H | NO₂ | H | Et | nPr |
| A.532. | F | H | H | Et | nPr |
| A.533. | Cl | H | H | Et | nPr |
| A.534. | Br | H | H | Et | nPr |
| A.535. | CN | H | H | Et | nPr |
| A.536. | NO₂ | H | H | Et | nPr |
| A.537. | F | F | H | Et | nPr |
| A.538. | F | Cl | H | Et | nPr |
| A.539. | F | Br | H | Et | nPr |
| A.540. | F | CN | H | Et | nPr |
| A.541. | F | NO₂ | H | Et | nPr |
| A.542. | Cl | F | H | Et | nPr |
| A.543. | Cl | Cl | H | Et | nPr |
| A.544. | Cl | Br | H | Et | nPr |
| A.545. | Cl | CN | H | Et | nPr |
| A.546. | Cl | NO₂ | H | Et | nPr |
| A.547. | H | H | H | nPr | nPr |
| A.548. | H | F | H | nPr | nPr |
| A.549. | H | Cl | H | nPr | nPr |
| A.550. | H | Br | H | nPr | nPr |
| A.551. | H | CN | H | nPr | nPr |
| A.552. | H | NO₂ | H | nPr | nPr |
| A.553. | F | H | H | nPr | nPr |
| A.554. | Cl | H | H | nPr | nPr |
| A.555. | Br | H | H | nPr | nPr |
| A.556. | CN | H | H | nPr | nPr |
| A.557. | NO₂ | H | H | nPr | nPr |
| A.558. | F | F | H | nPr | nPr |
| A.559. | F | Cl | H | nPr | nPr |
| A.560. | F | Br | H | nPr | nPr |
| A.561. | F | CN | H | nPr | nPr |
| A.562. | F | NO₂ | H | nPr | nPr |
| A.563. | Cl | F | H | nPr | nPr |
| A.564. | Cl | Cl | H | nPr | nPr |
| A.565. | Cl | Br | H | nPr | nPr |
| A.566. | Cl | CN | H | nPr | nPr |
| A.567. | Cl | NO₂ | H | nPr | nPr |
| A.568. | H | H | H | iPr | nPr |
| A.569. | H | F | H | iPr | nPr |
| A.570. | H | Cl | H | iPr | nPr |
| A.571. | H | Br | H | iPr | nPr |
| A.572. | H | CN | H | iPr | nPr |
| A.573. | H | NO₂ | H | iPr | nPr |
| A.574. | F | H | H | iPr | nPr |
| A.575. | Cl | H | H | iPr | nPr |
| A.576. | Br | H | H | iPr | nPr |
| A.577. | CN | H | H | iPr | nPr |
| A.578. | NO₂ | H | H | iPr | nPr |
| A.579. | F | F | H | iPr | nPr |
| A.580. | F | Cl | H | iPr | nPr |
| A.581. | F | Br | H | iPr | nPr |
| A.582. | F | CN | H | iPr | nPr |
| A.583. | F | NO₂ | H | iPr | nPr |
| A.584. | Cl | F | H | iPr | nPr |
| A.585. | Cl | Cl | H | iPr | nPr |
| A.586. | Cl | Br | H | iPr | nPr |
| A.587. | Cl | CN | H | iPr | nPr |
| A.588. | Cl | NO₂ | H | iPr | nPr |
| A.589. | H | H | H | vinyl | nPr |
| A.590. | H | F | H | vinyl | nPr |
| A.591. | H | Cl | H | vinyl | nPr |
| A.592. | H | Br | H | vinyl | nPr |
| A.593. | H | CN | H | vinyl | nPr |
| A.594. | H | NO₂ | H | vinyl | nPr |
| A.595. | F | H | H | vinyl | nPr |
| A.596. | Cl | H | H | vinyl | nPr |
| A.597. | Br | H | H | vinyl | nPr |
| A.598. | CN | H | H | vinyl | nPr |
| A.599. | NO₂ | H | H | vinyl | nPr |
| A.600. | F | F | H | vinyl | nPr |
| A.601. | F | Cl | H | vinyl | nPr |
| A.602. | F | Br | H | vinyl | nPr |
| A.603. | F | CN | H | vinyl | nPr |
| A.604. | F | NO₂ | H | vinyl | nPr |
| A.605. | Cl | F | H | vinyl | nPr |
| A.606. | Cl | Cl | H | vinyl | nPr |
| A.607. | Cl | Br | H | vinyl | nPr |
| A.608. | Cl | CN | H | vinyl | nPr |
| A.609. | Cl | NO₂ | H | vinyl | nPr |
| A.610. | H | H | H | allyl | nPr |
| A.611. | H | F | H | allyl | nPr |
| A.612. | H | Cl | H | allyl | nPr |
| A.613. | H | Br | H | allyl | nPr |
| A.614. | H | CN | H | allyl | nPr |
| A.615. | H | NO₂ | H | allyl | nPr |
| A.616. | F | H | H | allyl | nPr |
| A.617. | Cl | H | H | allyl | nPr |
| A.618. | Br | H | H | allyl | nPr |
| A.619. | CN | H | H | allyl | nPr |
| A.620. | NO₂ | H | H | allyl | nPr |
| A.621. | F | F | H | allyl | nPr |
| A.622. | F | Cl | H | allyl | nPr |
| A.623. | F | Br | H | allyl | nPr |
| A.624. | F | CN | H | allyl | nPr |
| A.625. | F | NO₂ | H | allyl | nPr |
| A.626. | Cl | F | H | allyl | nPr |
| A.627. | Cl | Cl | H | allyl | nPr |
| A.628. | Cl | Br | H | allyl | nPr |
| A.629. | Cl | CN | H | allyl | nPr |
| A.630. | Cl | NO₂ | H | allyl | nPr |
| A.631. | H | H | H | OMe | nPr |
| A.632. | H | F | H | OMe | nPr |
| A.633. | H | Cl | H | OMe | nPr |
| A.634. | H | Br | H | OMe | nPr |
| A.635. | H | CN | H | OMe | nPr |
| A.636. | H | NO₂ | H | OMe | nPr |
| A.637. | F | H | H | OMe | nPr |
| A.638. | Cl | H | H | OMe | nPr |
| A.639. | Br | H | H | OMe | nPr |
| A.640. | CN | H | H | OMe | nPr |
| A.641. | NO₂ | H | H | OMe | nPr |
| A.642. | F | F | H | OMe | nPr |
| A.643. | F | Cl | H | OMe | nPr |
| A.644. | F | Br | H | OMe | nPr |
| A.645. | F | CN | H | OMe | nPr |
| A.646. | F | NO₂ | H | OMe | nPr |
| A.647. | Cl | F | H | OMe | nPr |
| A.648. | Cl | Cl | H | OMe | nPr |
| A.649. | Cl | Br | H | OMe | nPr |
| A.650. | Cl | CN | H | OMe | nPr |
| A.651. | Cl | NO₂ | H | OMe | nPr |
| A.652. | H | H | H | OEt | nPr |
| A.653. | H | F | H | OEt | nPr |
| A.654. | H | Cl | H | OEt | nPr |
| A.655. | H | Br | H | OEt | nPr |
| A.656. | H | CN | H | OEt | nPr |
| A.657. | H | NO₂ | H | OEt | nPr |
| A.658. | F | H | H | OEt | nPr |
| A.659. | Cl | H | H | OEt | nPr |
| A.660. | Br | H | H | OEt | nPr |
| A.661. | CN | H | H | OEt | nPr |
| A.662. | NO₂ | H | H | OEt | nPr |
| A.663. | F | F | H | OEt | nPr |
| A.664. | F | Cl | H | OEt | nPr |
| A.665. | F | Br | H | OEt | nPr |
| A.666. | F | CN | H | OEt | nPr |
| A.667. | F | NO₂ | H | OEt | nPr |
| A.668. | Cl | F | H | OEt | nPr |
| A.669. | Cl | Cl | H | OEt | nPr |
| A.670. | Cl | Br | H | OEt | nPr |
| A.671. | Cl | CN | H | OEt | nPr |
| A.672. | Cl | NO₂ | H | OEt | nPr |
| A.673. | H | H | H | Me | iPr |
| A.674. | H | F | H | Me | iPr |
| A.675. | H | Cl | H | Me | iPr |
| A.676. | H | Br | H | Me | iPr |
| A.677. | H | CN | H | Me | iPr |
| A.678. | H | NO₂ | H | Me | iPr |
| A.679. | F | H | H | Me | iPr |
| A.680. | Cl | H | H | Me | iPr |
| A.681. | Br | H | H | Me | iPr |
| A.682. | CN | H | H | Me | iPr |
| A.683. | NO₂ | H | H | Me | iPr |
| A.684. | F | F | H | Me | iPr |
| A.685. | F | Cl | H | Me | iPr |
| A.686. | F | Br | H | Me | iPr |
| A.687. | F | CN | H | Me | iPr |
| A.688. | F | NO₂ | H | Me | iPr |
| A.689. | Cl | F | H | Me | iPr |
| A.690. | Cl | Cl | H | Me | iPr |
| A.691. | Cl | Br | H | Me | iPr |
| A.692. | Cl | CN | H | Me | iPr |
| A.693. | Cl | NO₂ | H | Me | iPr |
| A.694. | H | H | H | Et | iPr |
| A.695. | H | F | H | Et | iPr |
| A.696. | H | Cl | H | Et | iPr |
| A.697. | H | Br | H | Et | iPr |
| A.698. | H | CN | H | Et | iPr |
| A.699. | H | NO₂ | H | Et | iPr |
| A.700. | F | H | H | Et | iPr |
| A.701. | Cl | H | H | Et | iPr |
| A.702. | Br | H | H | Et | iPr |
| A.703. | CN | H | H | Et | iPr |
| A.704. | NO₂ | H | H | Et | iPr |
| A.705. | F | F | H | Et | iPr |
| A.706. | F | Cl | H | Et | iPr |
| A.707. | F | Br | H | Et | iPr |
| A.708. | F | CN | H | Et | iPr |
| A.709. | F | NO₂ | H | Et | iPr |
| A.710. | Cl | F | H | Et | iPr |
| A. 711. | Cl | Cl | H | Et | iPr |
| A.712. | Cl | Br | H | Et | iPr |
| A.713. | Cl | CN | H | Et | iPr |
| A.714. | Cl | NO₂ | H | Et | iPr |
| A.715. | H | H | H | nPr | iPr |
| A.716. | H | F | H | nPr | iPr |
| A.717. | H | Cl | H | nPr | iPr |
| A.718. | H | Br | H | nPr | iPr |
| A.719. | H | CN | H | nPr | iPr |
| A.720. | H | NO₂ | H | nPr | iPr |
| A.721. | F | H | H | nPr | iPr |
| A.722. | Cl | H | H | nPr | iPr |
| A.723. | Br | H | H | nPr | iPr |
| A.724. | CN | H | H | nPr | iPr |
| A.725. | NO₂ | H | H | nPr | iPr |
| A.726. | F | F | H | nPr | iPr |
| A.727. | F | Cl | H | nPr | iPr |
| A.728. | F | Br | H | nPr | iPr |
| A.729. | F | CN | H | nPr | iPr |
| A.730. | F | NO₂ | H | nPr | iPr |
| A.731. | Cl | F | H | nPr | iPr |
| A.732. | Cl | Cl | H | nPr | iPr |
| A.733. | Cl | Br | H | nPr | iPr |
| A.734. | Cl | CN | H | nPr | iPr |
| A.735. | Cl | NO₂ | H | nPr | iPr |
| A.736. | H | H | H | iPr | iPr |
| A.737. | H | F | H | iPr | iPr |
| A.738. | H | Cl | H | iPr | iPr |
| A.739. | H | Br | H | iPr | iPr |
| A.740. | H | CN | H | iPr | iPr |
| A.741. | H | NO₂ | H | iPr | iPr |
| A.742. | F | H | H | iPr | iPr |
| A.743. | Cl | H | H | iPr | iPr |
| A.744. | Br | H | H | iPr | iPr |
| A.745. | CN | H | H | iPr | iPr |
| A.746. | NO₂ | H | H | iPr | iPr |
| A.747. | F | F | H | iPr | iPr |
| A.748. | F | Cl | H | iPr | iPr |
| A.749. | F | Br | H | iPr | iPr |
| A.750. | F | CN | H | iPr | iPr |
| A.751. | F | NO₂ | H | iPr | iPr |
| A.752. | Cl | F | H | iPr | iPr |
| A.753. | Cl | Cl | H | iPr | iPr |
| A.754. | Cl | Br | H | iPr | iPr |
| A.755. | Cl | CN | H | iPr | iPr |
| A.756. | Cl | NO₂ | H | iPr | iPr |
| A.757. | H | H | H | vinyl | iPr |
| A.758. | H | F | H | vinyl | iPr |
| A.759. | H | Cl | H | vinyl | iPr |
| A.760. | H | Br | H | vinyl | iPr |
| A.761. | H | CN | H | vinyl | iPr |
| A.762. | H | NO₂ | H | vinyl | iPr |
| A.763. | F | H | H | vinyl | iPr |
| A.764. | Cl | H | H | vinyl | iPr |
| A.765. | Br | H | H | vinyl | iPr |
| A.766. | CN | H | H | vinyl | iPr |
| A.767. | NO₂ | H | H | vinyl | iPr |
| A.768. | F | F | H | vinyl | iPr |
| A.769. | F | Cl | H | vinyl | iPr |
| A.770. | F | Br | H | vinyl | iPr |
| A.771. | F | CN | H | vinyl | iPr |
| A.772. | F | NO₂ | H | vinyl | iPr |
| A.773. | Cl | F | H | vinyl | iPr |
| A.774. | Cl | Cl | H | vinyl | iPr |
| A.775. | Cl | Br | H | vinyl | iPr |
| A.776. | Cl | CN | H | vinyl | iPr |
| A.777. | Cl | NO₂ | H | vinyl | iPr |
| A.778. | H | H | H | allyl | iPr |
| A.779. | H | F | H | allyl | iPr |
| A.780. | H | Cl | H | allyl | iPr |
| A.781. | H | Br | H | allyl | iPr |
| A.782. | H | CN | H | allyl | iPr |
| A.783. | H | NO₂ | H | allyl | iPr |
| A.784. | F | H | H | allyl | iPr |
| A.785. | Cl | H | H | allyl | iPr |
| A.786. | Br | H | H | allyl | iPr |
| A.787. | CN | H | H | allyl | iPr |
| A.788. | NO₂ | H | H | allyl | iPr |
| A.789. | F | F | H | allyl | iPr |
| A.790. | F | Cl | H | allyl | iPr |
| A.791. | F | Br | H | allyl | iPr |
| A.792. | F | CN | H | allyl | iPr |
| A.793. | F | NO₂ | H | allyl | iPr |
| A.794. | Cl | F | H | allyl | iPr |
| A.795. | Cl | Cl | H | allyl | iPr |
| A.796. | Cl | Br | H | allyl | iPr |
| A.797. | Cl | CN | H | allyl | iPr |
| A.798. | Cl | NO₂ | H | allyl | iPr |
| A.799. | H | H | H | OMe | iPr |
| A.800. | H | F | H | OMe | iPr |
| A.801. | H | Cl | H | OMe | iPr |
| A.802. | H | Br | H | OMe | iPr |
| A.803. | H | CN | H | OMe | iPr |
| A.804. | H | NO₂ | H | OMe | iPr |
| A.805. | F | H | H | OMe | iPr |
| A.806. | Cl | H | H | OMe | iPr |
| A.807. | Br | H | H | OMe | iPr |
| A.808. | CN | H | H | OMe | iPr |
| A.809. | NO₂ | H | H | OMe | iPr |
| A.810. | F | F | H | OMe | iPr |
| A.811. | F | Cl | H | OMe | iPr |
| A.812. | F | Br | H | OMe | iPr |
| A.813. | F | CN | H | OMe | iPr |
| A.814. | F | NO₂ | H | OMe | iPr |
| A.815. | Cl | F | H | OMe | iPr |
| A.816. | Cl | Cl | H | OMe | iPr |
| A.817. | Cl | Br | H | OMe | iPr |
| A.818. | Cl | CN | H | OMe | iPr |
| A.819. | Cl | NO₂ | H | OMe | iPr |
| A.820. | H | H | H | OEt | iPr |
| A.821. | H | F | H | OEt | iPr |
| A.822. | H | Cl | H | OEt | iPr |
| A.823. | H | Br | H | OEt | iPr |
| A.824. | H | CN | H | OEt | iPr |
| A.825. | H | NO₂ | H | OEt | iPr |
| A.826. | F | H | H | OEt | iPr |
| A.827. | Cl | H | H | OEt | iPr |
| A.828. | Br | H | H | OEt | iPr |
| A.829. | CN | H | H | OEt | iPr |
| A.830. | NO₂ | H | H | OEt | iPr |
| A.831. | F | F | H | OEt | iPr |
| A.832. | F | Cl | H | OEt | iPr |
| A.833. | F | Br | H | OEt | iPr |
| A.834. | F | CN | H | OEt | iPr |
| A.835. | F | NO₂ | H | OEt | iPr |
| A.836. | Cl | F | H | OEt | iPr |
| A.837. | Cl | Cl | H | OEt | iPr |
| A.838. | Cl | Br | H | OEt | iPr |
| A.839. | Cl | CN | H | OEt | iPr |
| A.840. | Cl | NO₂ | H | OEt | iPr |
| A.841. | H | H | H | Me | nBu |
| A.842. | H | F | H | Me | nBu |
| A.843. | H | Cl | H | Me | nBu |
| A.844. | H | Br | H | Me | nBu |
| A.845. | H | CN | H | Me | nBu |
| A.846. | H | NO₂ | H | Me | nBu |
| A.847. | F | H | H | Me | nBu |
| A.848. | Cl | H | H | Me | nBu |
| A.849. | Br | H | H | Me | nBu |
| A.850. | CN | H | H | Me | nBu |
| A.851. | NO₂ | H | H | Me | nBu |
| A.852. | F | F | H | Me | nBu |
| A.853. | F | Cl | H | Me | nBu |
| A.854. | F | Br | H | Me | nBu |
| A.855. | F | CN | H | Me | nBu |
| A.856. | F | NO₂ | H | Me | nBu |
| A.857. | Cl | F | H | Me | nBu |
| A.858. | Cl | Cl | H | Me | nBu |
| A.859. | Cl | Br | H | Me | nBu |
| A.860. | Cl | CN | H | Me | nBu |
| A.861. | Cl | NO₂ | H | Me | nBu |
| A.862. | H | H | H | Et | nBu |
| A.863. | H | F | H | Et | nBu |
| A.864. | H | Cl | H | Et | nBu |
| A.865. | H | Br | H | Et | nBu |
| A.866. | H | CN | H | Et | nBu |
| A.867. | H | NO₂ | H | Et | nBu |
| A.868. | F | H | H | Et | nBu |
| A.869. | Cl | H | H | Et | nBu |
| A.870. | Br | H | H | Et | nBu |
| A.871. | CN | H | H | Et | nBu |
| A.872. | NO₂ | H | H | Et | nBu |
| A.873. | F | F | H | Et | nBu |
| A.874. | F | Cl | H | Et | nBu |
| A.875. | F | Br | H | Et | nBu |
| A.876. | F | CN | H | Et | nBu |
| A.877. | F | NO₂ | H | Et | nBu |
| A.878. | Cl | F | H | Et | nBu |
| A.879. | Cl | Cl | H | Et | nBu |
| A.880. | Cl | Br | H | Et | nBu |
| A.881. | Cl | CN | H | Et | nBu |
| A.882. | Cl | NO₂ | H | Et | nBu |
| A.883. | H | H | H | nPr | nBu |
| A.884. | H | F | H | nPr | nBu |
| A.885. | H | Cl | H | nPr | nBu |
| A.886. | H | Br | H | nPr | nBu |
| A.887. | H | CN | H | nPr | nBu |
| A.888. | H | NO₂ | H | nPr | nBu |
| A.889. | F | H | H | nPr | nBu |
| A.890. | Cl | H | H | nPr | nBu |
| A.891. | Br | H | H | nPr | nBu |
| A.892. | CN | H | H | nPr | nBu |
| A.893. | NO₂ | H | H | nPr | nBu |
| A.894. | F | F | H | nPr | nBu |
| A.895. | F | Cl | H | nPr | nBu |
| A.896. | F | Br | H | nPr | nBu |
| A.897. | F | CN | H | nPr | nBu |
| A.898. | F | NO₂ | H | nPr | nBu |
| A.899. | Cl | F | H | nPr | nBu |
| A.900. | Cl | Cl | H | nPr | nBu |
| A.901. | Cl | Br | H | nPr | nBu |
| A.902. | Cl | CN | H | nPr | nBu |
| A.903. | Cl | NO₂ | H | nPr | nBu |
| A.904. | H | H | H | iPr | nBu |
| A.905. | H | F | H | iPr | nBu |
| A.906. | H | Cl | H | iPr | nBu |
| A.907. | H | Br | H | iPr | nBu |
| A.908. | H | CN | H | iPr | nBu |
| A.909. | H | NO₂ | H | iPr | nBu |
| A.910. | F | H | H | iPr | nBu |
| A.911. | Cl | H | H | iPr | nBu |
| A.912. | Br | H | H | iPr | nBu |
| A.913. | CN | H | H | iPr | nBu |
| A.914. | NO₂ | H | H | iPr | nBu |
| A.915. | F | F | H | iPr | nBu |
| A.916. | F | Cl | H | iPr | nBu |
| A.917. | F | Br | H | iPr | nBu |
| A.918. | F | CN | H | iPr | nBu |
| A.919. | F | NO₂ | H | iPr | nBu |
| A.920. | Cl | F | H | iPr | nBu |
| A.921. | Cl | Cl | H | iPr | nBu |
| A.922. | Cl | Br | H | iPr | nBu |
| A.923. | Cl | CN | H | iPr | nBu |
| A.924. | Cl | NO₂ | H | iPr | nBu |
| A.925. | H | H | H | vinyl | nBu |
| A.926. | H | F | H | vinyl | nBu |
| A.927. | H | Cl | H | vinyl | nBu |
| A.928. | H | Br | H | vinyl | nBu |
| A.929. | H | CN | H | vinyl | nBu |
| A.930. | H | NO₂ | H | vinyl | nBu |
| A.931. | F | H | H | vinyl | nBu |
| A.932. | Cl | H | H | vinyl | nBu |
| A.933. | Br | H | H | vinyl | nBu |
| A.934. | CN | H | H | vinyl | nBu |
| A.935. | NO₂ | H | H | vinyl | nBu |
| A.936. | F | F | H | vinyl | nBu |
| A.937. | F | Cl | H | vinyl | nBu |
| A.938. | F | Br | H | vinyl | nBu |
| A.939. | F | CN | H | vinyl | nBu |
| A.940. | F | NO₂ | H | vinyl | nBu |
| A.941. | Cl | F | H | vinyl | nBu |
| A.942. | Cl | Cl | H | vinyl | nBu |
| A.943. | Cl | Br | H | vinyl | nBu |
| A.944. | Cl | CN | H | vinyl | nBu |
| A.945. | Cl | NO₂ | H | vinyl | nBu |
| A.946. | H | H | H | allyl | nBu |
| A.947. | H | F | H | allyl | nBu |
| A.948. | H | Cl | H | allyl | nBu |
| A.949. | H | Br | H | allyl | nBu |
| A.950. | H | CN | H | allyl | nBu |
| A.951. | H | NO₂ | H | allyl | nBu |
| A.952. | F | H | H | allyl | nBu |
| A.953. | Cl | H | H | allyl | nBu |
| A.954. | Br | H | H | allyl | nBu |
| A.955. | CN | H | H | allyl | nBu |
| A.956. | NO₂ | H | H | allyl | nBu |
| A.957. | F | F | H | allyl | nBu |
| A.958. | F | Cl | H | allyl | nBu |
| A.959. | F | Br | H | allyl | nBu |
| A.960. | F | CN | H | allyl | nBu |
| A.961. | F | NO₂ | H | allyl | nBu |
| A.962. | Cl | F | H | allyl | nBu |
| A.963. | Cl | Cl | H | allyl | nBu |
| A.964. | Cl | Br | H | allyl | nBu |
| A.965. | Cl | CN | H | allyl | nBu |
| A.966. | Cl | NO₂ | H | allyl | nBu |
| A.967. | H | H | H | OMe | nBu |
| A.968. | H | F | H | OMe | nBu |
| A.969. | H | Cl | H | OMe | nBu |
| A.970. | H | Br | H | OMe | nBu |
| A.971. | H | CN | H | OMe | nBu |
| A.972. | H | NO₂ | H | OMe | nBu |
| A.973. | F | H | H | OMe | nBu |
| A.974. | Cl | H | H | OMe | nBu |
| A.975. | Br | H | H | OMe | nBu |
| A.976. | CN | H | H | OMe | nBu |
| A.977. | NO₂ | H | H | OMe | nBu |
| A.978. | F | F | H | OMe | nBu |
| A.979. | F | Cl | H | OMe | nBu |
| A.980. | F | Br | H | OMe | nBu |
| A.981. | F | CN | H | OMe | nBu |
| A.982. | F | NO₂ | H | OMe | nBu |
| A.983. | Cl | F | H | OMe | nBu |
| A.984. | Cl | Cl | H | OMe | nBu |
| A.985. | Cl | Br | H | OMe | nBu |
| A.986. | Cl | CN | H | OMe | nBu |
| A.987. | Cl | NO₂ | H | OMe | nBu |
| A.988. | H | H | H | OEt | nBu |
| A.989. | H | F | H | OEt | nBu |
| A.990. | H | Cl | H | OEt | nBu |
| A.991. | H | Br | H | OEt | nBu |
| A.992. | H | CN | H | OEt | nBu |
| A.993. | H | NO₂ | H | OEt | nBu |
| A.994. | F | H | H | OEt | nBu |
| A.995. | Cl | H | H | OEt | nBu |
| A.996. | Br | H | H | OEt | nBu |
| A.997. | CN | H | H | OEt | nBu |
| A.998. | NO₂ | H | H | OEt | nBu |
| A.999. | F | F | H | OEt | nBu |
| A.1000. | F | Cl | H | OEt | nBu |
| A.1001. | F | Br | H | OEt | nBu |
| A.1002. | F | CN | H | OEt | nBu |
| A.1003. | F | NO₂ | H | OEt | nBu |
| A.1004. | Cl | F | H | OEt | nBu |
| A.1005. | Cl | Cl | H | OEt | nBu |
| A.1006. | Cl | Br | H | OEt | nBu |
| A.1007. | Cl | CN | H | OEt | nBu |
| A.1008. | Cl | NO₂ | H | OEt | nBu |
| A.1009. | H | H | H | Me | 2Bu |
| A.1010. | H | F | H | Me | 2Bu |
| A.1011. | H | Cl | H | Me | 2Bu |
| A.1012. | H | Br | H | Me | 2Bu |
| A.1013. | H | CN | H | Me | 2Bu |
| A.1014. | H | NO₂ | H | Me | 2Bu |
| A.1015. | F | H | H | Me | 2Bu |
| A.1016. | Cl | H | H | Me | 2Bu |
| A.1017. | Br | H | H | Me | 2Bu |
| A.1018. | CN | H | H | Me | 2Bu |
| A.1019. | NO₂ | H | H | Me | 2Bu |
| A.1020. | F | F | H | Me | 2Bu |
| A.1021. | F | Cl | H | Me | 2Bu |
| A.1022. | F | Br | H | Me | 2Bu |
| A.1023. | F | CN | H | Me | 2Bu |
| A.1024. | F | NO₂ | H | Me | 2Bu |
| A.1025. | Cl | F | H | Me | 2Bu |
| A.1026. | Cl | Cl | H | Me | 2Bu |
| A.1027. | Cl | Br | H | Me | 2Bu |
| A.1028. | Cl | CN | H | Me | 2Bu |
| A.1029. | Cl | NO₂ | H | Me | 2Bu |
| A.1030. | H | H | H | Et | 2Bu |
| A.1031. | H | F | H | Et | 2Bu |
| A.1032. | H | Cl | H | Et | 2Bu |
| A.1033. | H | Br | H | Et | 2Bu |
| A.1034. | H | CN | H | Et | 2Bu |
| A.1035. | H | NO₂ | H | Et | 2Bu |
| A.1036. | F | H | H | Et | 2Bu |
| A.1037. | Cl | H | H | Et | 2Bu |
| A.1038. | Br | H | H | Et | 2Bu |
| A.1039. | CN | H | H | Et | 2Bu |
| A.1040. | NO₂ | H | H | Et | 2Bu |
| A.1041. | F | F | H | Et | 2Bu |
| A. 1042. | F | Cl | H | Et | 2Bu |
| A.1043. | F | Br | H | Et | 2Bu |
| A.1044. | F | CN | H | Et | 2Bu |
| A.1045. | F | NO₂ | H | Et | 2Bu |
| A. 1046. | Cl | F | H | Et | 2Bu |
| A.1047. | Cl | Cl | H | Et | 2Bu |
| A.1048. | Cl | Br | H | Et | 2Bu |
| A.1049. | Cl | CN | H | Et | 2Bu |
| A.1050. | Cl | NO₂ | H | Et | 2Bu |
| A.1051. | H | H | H | nPr | 2Bu |
| A.1052. | H | F | H | nPr | 2Bu |
| A.1053. | H | Cl | H | nPr | 2Bu |
| A.1054. | H | Br | H | nPr | 2Bu |
| A.1055. | H | CN | H | nPr | 2Bu |
| A.1056. | H | NO₂ | H | nPr | 2Bu |
| A.1057. | F | H | H | nPr | 2Bu |
| A.1058. | Cl | H | H | nPr | 2Bu |
| A.1059. | Br | H | H | nPr | 2Bu |
| A.1060. | CN | H | H | nPr | 2Bu |
| A.1061. | NO₂ | H | H | nPr | 2Bu |
| A.1062. | F | F | H | nPr | 2Bu |
| A.1063. | F | Cl | H | nPr | 2Bu |
| A.1064. | F | Br | H | nPr | 2Bu |
| A.1065. | F | CN | H | nPr | 2Bu |
| A.1066. | F | NO₂ | H | nPr | 2Bu |
| A.1067. | Cl | F | H | nPr | 2Bu |
| A.1068. | Cl | Cl | H | nPr | 2Bu |
| A.1069. | Cl | Br | H | nPr | 2Bu |
| A.1070. | Cl | CN | H | nPr | 2Bu |
| A.1071. | Cl | NO₂ | H | nPr | 2Bu |
| A.1072. | H | H | H | iPr | 2Bu |
| A.1073. | H | F | H | iPr | 2Bu |
| A.1074. | H | Cl | H | iPr | 2Bu |
| A.1075. | H | Br | H | iPr | 2Bu |
| A.1076. | H | CN | H | iPr | 2Bu |
| A.1077. | H | NO₂ | H | iPr | 2Bu |
| A.1078. | F | H | H | iPr | 2Bu |
| A.1079. | Cl | H | H | iPr | 2Bu |
| A.1080. | Br | H | H | iPr | 2Bu |
| A.1081. | CN | H | H | iPr | 2Bu |
| A.1082. | NO₂ | H | H | iPr | 2Bu |
| A.1083. | F | F | H | iPr | 2Bu |
| A.1084. | F | Cl | H | iPr | 2Bu |
| A.1085. | F | Br | H | iPr | 2Bu |
| A.1086. | F | CN | H | iPr | 2Bu |
| A.1087. | F | NO₂ | H | iPr | 2Bu |
| A.1088. | Cl | F | H | iPr | 2Bu |
| A.1089. | Cl | Cl | H | iPr | 2Bu |
| A.1090. | Cl | Br | H | iPr | 2Bu |
| A.1091. | Cl | CN | H | iPr | 2Bu |
| A.1092. | Cl | NO₂ | H | iPr | 2Bu |
| A.1093. | H | H | H | vinyl | 2Bu |
| A.1094. | H | F | H | vinyl | 2Bu |
| A.1095. | H | Cl | H | vinyl | 2Bu |
| A.1096. | H | Br | H | vinyl | 2Bu |
| A.1097. | H | CN | H | vinyl | 2Bu |
| A.1098. | H | NO₂ | H | vinyl | 2Bu |
| A.1099. | F | H | H | vinyl | 2Bu |
| A.1100. | Cl | H | H | vinyl | 2Bu |
| A.1101. | Br | H | H | vinyl | 2Bu |
| A. 1102. | CN | H | H | vinyl | 2Bu |
| A. 1103. | NO₂ | H | H | vinyl | 2Bu |
| A. 1104. | F | F | H | vinyl | 2Bu |
| A. 1105. | F | Cl | H | vinyl | 2Bu |
| A.1106. | F | Br | H | vinyl | 2Bu |
| A. 1107. | F | CN | H | vinyl | 2Bu |
| A. 1108. | F | NO₂ | H | vinyl | 2Bu |
| A. 1109. | Cl | F | H | vinyl | 2Bu |
| A.1110. | Cl | Cl | H | vinyl | 2Bu |
| A.1111. | Cl | Br | H | vinyl | 2Bu |
| A.1112. | Cl | CN | H | vinyl | 2Bu |
| A.1113. | Cl | NO₂ | H | vinyl | 2Bu |
| A.1114. | H | H | H | allyl | 2Bu |
| A.1115. | H | F | H | allyl | 2Bu |
| A.1116. | H | Cl | H | allyl | 2Bu |
| A.1117. | H | Br | H | allyl | 2Bu |
| A.1118. | H | CN | H | allyl | 2Bu |
| A.1119. | H | NO₂ | H | allyl | 2Bu |
| A.1120. | F | H | H | allyl | 2Bu |
| A.1121. | Cl | H | H | allyl | 2Bu |
| A.1122. | Br | H | H | allyl | 2Bu |
| A.1123. | CN | H | H | allyl | 2Bu |
| A.1124. | NO₂ | H | H | allyl | 2Bu |
| A.1125. | F | F | H | allyl | 2Bu |
| A.1126. | F | Cl | H | allyl | 2Bu |
| A.1127. | F | Br | H | allyl | 2Bu |
| A.1128. | F | CN | H | allyl | 2Bu |
| A.1129. | F | NO₂ | H | allyl | 2Bu |
| A. 1130. | Cl | F | H | allyl | 2Bu |
| A.1131. | Cl | Cl | H | allyl | 2Bu |
| A. 1132. | Cl | Br | H | allyl | 2Bu |
| A.1133. | Cl | CN | H | allyl | 2Bu |
| A. 1134. | Cl | NO₂ | H | allyl | 2Bu |
| A.1135. | H | H | H | OMe | 2Bu |
| A.1136. | H | F | H | OMe | 2Bu |
| A.1137. | H | Cl | H | OMe | 2Bu |
| A.1138. | H | Br | H | OMe | 2Bu |
| A.1139. | H | CN | H | OMe | 2Bu |
| A.1140. | H | NO₂ | H | OMe | 2Bu |
| A.1141. | F | H | H | OMe | 2Bu |
| A.1142. | Cl | H | H | OMe | 2Bu |
| A.1143. | Br | H | H | OMe | 2Bu |
| A.1144. | CN | H | H | OMe | 2Bu |
| A.1145. | NO₂ | H | H | OMe | 2Bu |
| A.1146. | F | F | H | OMe | 2Bu |
| A.1147. | F | Cl | H | OMe | 2Bu |
| A.1148. | F | Br | H | OMe | 2Bu |
| A.1149. | F | CN | H | OMe | 2Bu |
| A.1150. | F | NO₂ | H | OMe | 2Bu |
| A.1151. | Cl | F | H | OMe | 2Bu |
| A.1152. | Cl | Cl | H | OMe | 2Bu |
| A.1153. | Cl | Br | H | OMe | 2Bu |
| A.1154. | Cl | CN | H | OMe | 2Bu |
| A.1155. | Cl | NO₂ | H | OMe | 2Bu |
| A.1156. | H | H | H | OEt | 2Bu |
| A.1157. | H | F | H | OEt | 2Bu |
| A.1158. | H | Cl | H | OEt | 2Bu |
| A. 1159. | H | Br | H | OEt | 2Bu |
| A.1160. | H | CN | H | OEt | 2Bu |
| A.1161. | H | NO₂ | H | OEt | 2Bu |
| A.1162. | F | H | H | OEt | 2Bu |
| A.1163. | Cl | H | H | OEt | 2Bu |
| A.1164. | Br | H | H | OEt | 2Bu |
| A.1165. | CN | H | H | OEt | 2Bu |
| A.1166. | NO₂ | H | H | OEt | 2Bu |
| A.1167. | F | F | H | OEt | 2Bu |
| A.1168. | F | Cl | H | OEt | 2Bu |
| A.1169. | F | Br | H | OEt | 2Bu |
| A.1170. | F | CN | H | OEt | 2Bu |
| A.1171. | F | NO₂ | H | OEt | 2Bu |
| A. 1172. | Cl | F | H | OEt | 2Bu |
| A. 1173. | Cl | Cl | H | OEt | 2Bu |
| A. 1174. | Cl | Br | H | OEt | 2Bu |
| A.1175. | Cl | CN | H | OEt | 2Bu |
| A. 1176. | Cl | NO₂ | H | OEt | 2Bu |
| A. 1177. | H | H | H | Me | iBu |
| A.1178. | H | F | H | Me | iBu |
| A.1179. | H | Cl | H | Me | iBu |
| A. 1180. | H | Br | H | Me | iBu |
| A.1181. | H | CN | H | Me | iBu |
| A. 1182. | H | NO₂ | H | Me | iBu |
| A.1183. | F | H | H | Me | iBu |
| A.1184. | Cl | H | H | Me | iBu |
| A.1185. | Br | H | H | Me | iBu |
| A.1186. | CN | H | H | Me | iBu |
| A. 1187. | NO₂ | H | H | Me | iBu |
| A.1188. | F | F | H | Me | iBu |
| A.1189. | F | Cl | H | Me | iBu |
| A. 1190. | F | Br | H | Me | iBu |
| A.1191. | F | CN | H | Me | iBu |
| A. 1192. | F | NO₂ | H | Me | iBu |
| A.1193. | Cl | F | H | Me | iBu |
| A.1194. | Cl | Cl | H | Me | iBu |
| A.1195. | Cl | Br | H | Me | iBu |
| A.1196. | Cl | CN | H | Me | iBu |
| A. 1197. | Cl | NO₂ | H | Me | iBu |
| A.1198. | H | H | H | Et | iBu |
| A. 1199. | H | F | H | Et | iBu |
| A.1200. | H | Cl | H | Et | iBu |
| A.1201. | H | Br | H | Et | iBu |
| A.1202. | H | CN | H | Et | iBu |
| A.1203. | H | NO₂ | H | Et | iBu |
| A.1204. | F | H | H | Et | iBu |
| A.1205. | Cl | H | H | Et | iBu |
| A.1206. | Br | H | H | Et | iBu |
| A.1207. | CN | H | H | Et | iBu |
| A.1208. | NO₂ | H | H | Et | iBu |
| A.1209. | F | F | H | Et | iBu |
| A.1210. | F | Cl | H | Et | iBu |
| A.1211. | F | Br | H | Et | iBu |
| A.1212. | F | CN | H | Et | iBu |
| A.1213. | F | NO₂ | H | Et | iBu |
| A.1214. | Cl | F | H | Et | iBu |
| A.1215. | Cl | Cl | H | Et | iBu |
| A.1216. | Cl | Br | H | Et | iBu |
| A.1217. | Cl | CN | H | Et | iBu |
| A.1218. | Cl | NO₂ | H | Et | iBu |
| A.1219. | H | H | H | nPr | iBu |
| A.1220. | H | F | H | nPr | iBu |
| A.1221. | H | Cl | H | nPr | iBu |
| A.1222. | H | Br | H | nPr | iBu |
| A.1223. | H | CN | H | nPr | iBu |
| A.1224. | H | NO₂ | H | nPr | iBu |
| A.1225. | F | H | H | nPr | iBu |
| A.1226. | Cl | H | H | nPr | iBu |
| A.1227. | Br | H | H | nPr | iBu |
| A.1228. | CN | H | H | nPr | iBu |
| A.1229. | NO₂ | H | H | nPr | iBu |
| A.1230. | F | F | H | nPr | iBu |
| A.1231. | F | Cl | H | nPr | iBu |
| A.1232. | F | Br | H | nPr | iBu |
| A.1233. | F | CN | H | nPr | iBu |
| A.1234. | F | NO₂ | H | nPr | iBu |
| A.1235. | Cl | F | H | nPr | iBu |
| A.1236. | Cl | Cl | H | nPr | iBu |
| A.1237. | Cl | Br | H | nPr | iBu |
| A.1238. | Cl | CN | H | nPr | iBu |
| A.1239. | Cl | NO₂ | H | nPr | iBu |
| A.1240. | H | H | H | iPr | iBu |
| A.1241. | H | F | H | iPr | iBu |
| A.1242. | H | Cl | H | iPr | iBu |
| A.1243. | H | Br | H | iPr | iBu |
| A.1244. | H | CN | H | iPr | iBu |
| A.1245. | H | NO₂ | H | iPr | iBu |
| A.1246. | F | H | H | iPr | iBu |
| A.1247. | Cl | H | H | iPr | iBu |
| A.1248. | Br | H | H | iPr | iBu |
| A.1249. | CN | H | H | iPr | iBu |
| A.1250. | NO₂ | H | H | iPr | iBu |
| A.1251. | F | F | H | iPr | iBu |
| A.1252. | F | Cl | H | iPr | iBu |
| A.1253. | F | Br | H | iPr | iBu |
| A.1254. | F | CN | H | iPr | iBu |
| A.1255. | F | NO₂ | H | iPr | iBu |
| A.1256. | Cl | F | H | iPr | iBu |
| A.1257. | Cl | Cl | H | iPr | iBu |
| A.1258. | Cl | Br | H | iPr | iBu |
| A.1259. | Cl | CN | H | iPr | iBu |
| A.1260. | Cl | NO₂ | H | iPr | iBu |
| A.1261. | H | H | H | vinyl | iBu |
| A.1262. | H | F | H | vinyl | iBu |
| A.1263. | H | Cl | H | vinyl | iBu |
| A.1264. | H | Br | H | vinyl | iBu |
| A.1265. | H | CN | H | vinyl | iBu |
| A.1266. | H | NO₂ | H | vinyl | iBu |
| A.1267. | F | H | H | vinyl | iBu |
| A.1268. | Cl | H | H | vinyl | iBu |
| A.1269. | Br | H | H | vinyl | iBu |
| A.1270. | CN | H | H | vinyl | iBu |
| A.1271. | NO₂ | H | H | vinyl | iBu |
| A.1272. | F | F | H | vinyl | iBu |
| A.1273. | F | Cl | H | vinyl | iBu |
| A.1274. | F | Br | H | vinyl | iBu |
| A.1275. | F | CN | H | vinyl | iBu |
| A.1276. | F | NO₂ | H | vinyl | iBu |
| A.1277. | Cl | F | H | vinyl | iBu |
| A.1278. | Cl | Cl | H | vinyl | iBu |
| A.1279. | Cl | Br | H | vinyl | iBu |
| A.1280. | Cl | CN | H | vinyl | iBu |
| A.1281. | Cl | NO₂ | H | vinyl | iBu |
| A.1282. | H | H | H | allyl | iBu |
| A.1283. | H | F | H | allyl | iBu |
| A.1284. | H | Cl | H | allyl | iBu |
| A.1285. | H | Br | H | allyl | iBu |
| A.1286. | H | CN | H | allyl | iBu |
| A.1287. | H | NO₂ | H | allyl | iBu |
| A.1288. | F | H | H | allyl | iBu |
| A.1289. | Cl | H | H | allyl | iBu |
| A.1290. | Br | H | H | allyl | iBu |
| A.1291. | CN | H | H | allyl | iBu |
| A.1292. | NO₂ | H | H | allyl | iBu |
| A.1293. | F | F | H | allyl | iBu |
| A.1294. | F | Cl | H | allyl | iBu |
| A.1295. | F | Br | H | allyl | iBu |
| A.1296. | F | CN | H | allyl | iBu |
| A.1297. | F | NO₂ | H | allyl | iBu |
| A.1298. | Cl | F | H | allyl | iBu |
| A.1299. | Cl | Cl | H | allyl | iBu |
| A.1300. | Cl | Br | H | allyl | iBu |
| A.1301. | Cl | CN | H | allyl | iBu |
| A.1302. | Cl | NO₂ | H | allyl | iBu |
| A.1303. | H | H | H | OMe | iBu |
| A.1304. | H | F | H | OMe | iBu |
| A.1305. | H | Cl | H | OMe | iBu |
| A.1306. | H | Br | H | OMe | iBu |
| A.1307. | H | CN | H | OMe | iBu |
| A.1308. | H | NO₂ | H | OMe | iBu |
| A.1309. | F | H | H | OMe | iBu |
| A.1310. | Cl | H | H | OMe | iBu |
| A.1311. | Br | H | H | OMe | iBu |
| A.1312. | CN | H | H | OMe | iBu |
| A.1313. | NO₂ | H | H | OMe | iBu |
| A.1314. | F | F | H | OMe | iBu |
| A.1315. | F | Cl | H | OMe | iBu |
| A.1316. | F | Br | H | OMe | iBu |
| A.1317. | F | CN | H | OMe | iBu |
| A.1318. | F | NO₂ | H | OMe | iBu |
| A.1319. | Cl | F | H | OMe | iBu |
| A.1320. | Cl | Cl | H | OMe | iBu |
| A.1321. | Cl | Br | H | OMe | iBu |
| A.1322. | Cl | CN | H | OMe | iBu |
| A.1323. | Cl | NO₂ | H | OMe | iBu |
| A.1324. | H | H | H | OEt | iBu |
| A.1325. | H | F | H | OEt | iBu |
| A.1326. | H | Cl | H | OEt | iBu |
| A.1327. | H | Br | H | OEt | iBu |
| A.1328. | H | CN | H | OEt | iBu |
| A.1329. | H | NO₂ | H | OEt | iBu |
| A.1330. | F | H | H | OEt | iBu |
| A.1331. | Cl | H | H | OEt | iBu |
| A.1332. | Br | H | H | OEt | iBu |
| A.1333. | CN | H | H | OEt | iBu |
| A.1334. | NO₂ | H | H | OEt | iBu |
| A.1335. | F | F | H | OEt | iBu |
| A.1336. | F | Cl | H | OEt | iBu |
| A.1337. | F | Br | H | OEt | iBu |
| A.1338. | F | CN | H | OEt | iBu |
| A.1339. | F | NO₂ | H | OEt | iBu |
| A.1340. | Cl | F | H | OEt | iBu |
| A.1341. | Cl | Cl | H | OEt | iBu |
| A.1342. | Cl | Br | H | OEt | iBu |
| A.1343. | Cl | CN | H | OEt | iBu |
| A.1344. | Cl | NO₂ | H | OEt | iBu |
| A.1345. | H | H | H | Me | tBu |
| A. 1346. | H | F | H | Me | tBu |
| A.1347. | H | Cl | H | Me | tBu |
| A.1348. | H | Br | H | Me | tBu |
| A.1349. | H | CN | H | Me | tBu |
| A.1350. | H | NO₂ | H | Me | tBu |
| A.1351. | F | H | H | Me | tBu |
| A.1352. | Cl | H | H | Me | tBu |
| A.1353. | Br | H | H | Me | tBu |
| A. 1354. | CN | H | H | Me | tBu |
| A.1355. | NO₂ | H | H | Me | tBu |
| A.1356. | F | F | H | Me | tBu |
| A.1357. | F | Cl | H | Me | tBu |
| A.1358. | F | Br | H | Me | tBu |
| A.1359. | F | CN | H | Me | tBu |
| A.1360. | F | NO₂ | H | Me | tBu |
| A.1361. | Cl | F | H | Me | tBu |
| A.1362. | Cl | Cl | H | Me | tBu |
| A.1363. | Cl | Br | H | Me | tBu |
| A.1364. | Cl | CN | H | Me | tBu |
| A.1365. | Cl | NO₂ | H | Me | tBu |
| A. 1366. | H | H | H | Et | tBu |
| A. 1367. | H | F | H | Et | tBu |
| A. 1368. | H | Cl | H | Et | tBu |
| A. 1369. | H | Br | H | Et | tBu |
| A.1370. | H | CN | H | Et | tBu |
| A.1371. | H | NO₂ | H | Et | tBu |
| A.1372. | F | H | H | Et | tBu |
| A.1373. | Cl | H | H | Et | tBu |
| A. 1374. | Br | H | H | Et | tBu |
| A.1375. | CN | H | H | Et | tBu |
| A. 1376. | NO₂ | H | H | Et | tBu |
| A.1377. | F | F | H | Et | tBu |
| A.1378. | F | Cl | H | Et | tBu |
| A.1379. | F | Br | H | Et | tBu |
| A.1380. | F | CN | H | Et | tBu |
| A.1381. | F | NO₂ | H | Et | tBu |
| A.1382. | Cl | F | H | Et | tBu |
| A.1383. | Cl | Cl | H | Et | tBu |
| A. 1384. | Cl | Br | H | Et | tBu |
| A.1385. | Cl | CN | H | Et | tBu |
| A. 1386. | Cl | NO₂ | H | Et | tBu |
| A.1387. | H | H | H | nPr | tBu |
| A.1388. | H | F | H | nPr | tBu |
| A.1389. | H | Cl | H | nPr | tBu |
| A.1390. | H | Br | H | nPr | tBu |
| A.1391. | H | CN | H | nPr | tBu |
| A.1392. | H | NO₂ | H | nPr | tBu |
| A.1393. | F | H | H | nPr | tBu |
| A.1394. | Cl | H | H | nPr | tBu |
| A.1395. | Br | H | H | nPr | tBu |
| A.1396. | CN | H | H | nPr | tBu |
| A.1397. | NO₂ | H | H | nPr | tBu |
| A.1398. | F | F | H | nPr | tBu |
| A.1399. | F | Cl | H | nPr | tBu |
| A.1400. | F | Br | H | nPr | tBu |
| A.1401. | F | CN | H | nPr | tBu |
| A.1402. | F | NO₂ | H | nPr | tBu |
| A.1403. | Cl | F | H | nPr | tBu |
| A.1404. | Cl | Cl | H | nPr | tBu |
| A.1405. | Cl | Br | H | nPr | tBu |
| A.1406. | Cl | CN | H | nPr | tBu |
| A.1407. | Cl | NO₂ | H | nPr | tBu |
| A.1408. | H | H | H | iPr | tBu |
| A.1409. | H | F | H | iPr | tBu |
| A.1410. | H | Cl | H | iPr | tBu |
| A.1411. | H | Br | H | iPr | tBu |
| A.1412. | H | CN | H | iPr | tBu |
| A.1413. | H | NO₂ | H | iPr | tBu |
| A.1414. | F | H | H | iPr | tBu |
| A.1415. | Cl | H | H | iPr | tBu |
| A.1416. | Br | H | H | iPr | tBu |
| A.1417. | CN | H | H | iPr | tBu |
| A.1418. | NO₂ | H | H | iPr | tBu |
| A.1419. | F | F | H | iPr | tBu |
| A.1420. | F | Cl | H | iPr | tBu |
| A.1421. | F | Br | H | iPr | tBu |
| A.1422. | F | CN | H | iPr | tBu |
| A.1423. | F | NO₂ | H | iPr | tBu |
| A. 1424. | Cl | F | H | iPr | tBu |
| A.1425. | Cl | Cl | H | iPr | tBu |
| A. 1426. | Cl | Br | H | iPr | tBu |
| A.1427. | Cl | CN | H | iPr | tBu |
| A.1428. | Cl | NO₂ | H | iPr | tBu |
| A.1429. | H | H | H | vinyl | tBu |
| A.1430. | H | F | H | vinyl | tBu |
| A.1431. | H | Cl | H | vinyl | tBu |
| A.1432. | H | Br | H | vinyl | tBu |
| A.1433. | H | CN | H | vinyl | tBu |
| A.1434. | H | NO₂ | H | vinyl | tBu |
| A.1435. | F | H | H | vinyl | tBu |
| A.1436. | Cl | H | H | vinyl | tBu |
| A.1437. | Br | H | H | vinyl | tBu |
| A.1438. | CN | H | H | vinyl | tBu |
| A.1439. | NO₂ | H | H | vinyl | tBu |
| A. 1440. | F | F | H | vinyl | tBu |
| A.1441. | F | Cl | H | vinyl | tBu |
| A.1442. | F | Br | H | vinyl | tBu |
| A. 1443. | F | CN | H | vinyl | tBu |
| A. 1444. | F | NO₂ | H | vinyl | tBu |
| A. 1445. | Cl | F | H | vinyl | tBu |
| A. 1446. | Cl | Cl | H | vinyl | tBu |
| A. 1447. | Cl | Br | H | vinyl | tBu |
| A. 1448. | Cl | CN | H | vinyl | tBu |
| A. 1449. | Cl | NO₂ | H | vinyl | tBu |
| A.1450. | H | H | H | allyl | tBu |
| A.1451. | H | F | H | allyl | tBu |
| A.1452. | H | Cl | H | allyl | tBu |
| A.1453. | H | Br | H | allyl | tBu |
| A.1454. | H | CN | H | allyl | tBu |
| A.1455. | H | NO₂ | H | allyl | tBu |
| A.1456. | F | H | H | allyl | tBu |
| A.1457. | Cl | H | H | allyl | tBu |
| A.1458. | Br | H | H | allyl | tBu |
| A.1459. | CN | H | H | allyl | tBu |
| A. 1460. | NO₂ | H | H | allyl | tBu |
| A.1461. | F | F | H | allyl | tBu |
| A.1462. | F | Cl | H | allyl | tBu |
| A. 1463. | F | Br | H | allyl | tBu |
| A. 1464. | F | CN | H | allyl | tBu |
| A. 1465. | F | NO₂ | H | allyl | tBu |
| A. 1466. | Cl | F | H | allyl | tBu |
| A. 1467. | Cl | Cl | H | allyl | tBu |
| A. 1468. | Cl | Br | H | allyl | tBu |
| A. 1469. | Cl | CN | H | allyl | tBu |
| A.1470. | Cl | NO₂ | H | allyl | tBu |
| A.1471. | H | H | H | OMe | tBu |
| A.1472. | H | F | H | OMe | tBu |
| A.1473. | H | Cl | H | OMe | tBu |
| A.1474. | H | Br | H | OMe | tBu |
| A.1475. | H | CN | H | OMe | tBu |
| A.1476. | H | NO₂ | H | OMe | tBu |
| A.1477. | F | H | H | OMe | tBu |
| A.1478. | Cl | H | H | OMe | tBu |
| A.1479. | Br | H | H | OMe | tBu |
| A.1480. | CN | H | H | OMe | tBu |
| A.1481. | NO₂ | H | H | OMe | tBu |
| A.1482. | F | F | H | OMe | tBu |
| A.1483. | F | Cl | H | OMe | tBu |
| A.1484. | F | Br | H | OMe | tBu |
| A.1485. | F | CN | H | OMe | tBu |
| A.1486. | F | NO₂ | H | OMe | tBu |
| A.1487. | Cl | F | H | OMe | tBu |
| A.1488. | Cl | Cl | H | OMe | tBu |
| A.1489. | Cl | Br | H | OMe | tBu |
| A.1490. | Cl | CN | H | OMe | tBu |
| A.1491. | Cl | NO₂ | H | OMe | tBu |
| A.1492. | H | H | H | OEt | tBu |
| A.1493. | H | F | H | OEt | tBu |
| A.1494. | H | Cl | H | OEt | tBu |
| A.1495. | H | Br | H | OEt | tBu |
| A.1496. | H | CN | H | OEt | tBu |
| A.1497. | H | NO₂ | H | OEt | tBu |
| A.1498. | F | H | H | OEt | tBu |
| A.1499. | Cl | H | H | OEt | tBu |
| A.1500. | Br | H | H | OEt | tBu |
| A.1501. | CN | H | H | OEt | tBu |
| A.1502. | NO₂ | H | H | OEt | tBu |
| A.1503. | F | F | H | OEt | tBu |
| A. 1504. | F | Cl | H | OEt | tBu |
| A.1505. | F | Br | H | OEt | tBu |
| A.1506. | F | CN | H | OEt | tBu |
| A.1507. | F | NO₂ | H | OEt | tBu |
| A.1508. | Cl | F | H | OEt | tBu |
| A.1509. | Cl | Cl | H | OEt | tBu |
| A.1510. | Cl | Br | H | OEt | tBu |
| A.1511. | Cl | CN | H | OEt | tBu |
| A.1512. | Cl | NO₂ | H | OEt | tBu |
| A.1513. | H | H | H | Me | cPr |
| A.1514. | H | F | H | Me | cPr |
| A.1515. | H | Cl | H | Me | cPr |
| A.1516. | H | Br | H | Me | cPr |
| A.1517. | H | CN | H | Me | cPr |
| A.1518. | H | NO₂ | H | Me | cPr |
| A.1519. | F | H | H | Me | cPr |
| A.1520. | Cl | H | H | Me | cPr |
| A.1521. | Br | H | H | Me | cPr |
| A.1522. | CN | H | H | Me | cPr |
| A.1523. | NO₂ | H | H | Me | cPr |
| A.1524. | F | F | H | Me | cPr |
| A.1525. | F | Cl | H | Me | cPr |
| A.1526. | F | Br | H | Me | cPr |
| A.1527. | F | CN | H | Me | cPr |
| A.1528. | F | NO₂ | H | Me | cPr |
| A.1529. | Cl | F | H | Me | cPr |
| A.1530. | Cl | Cl | H | Me | cPr |
| A.1531. | Cl | Br | H | Me | cPr |
| A.1532. | Cl | CN | H | Me | cPr |
| A.1533. | Cl | NO₂ | H | Me | cPr |
| A. 1534. | H | H | H | Et | cPr |
| A.1535. | H | F | H | Et | cPr |
| A. 1536. | H | Cl | H | Et | cPr |
| A.1537. | H | Br | H | Et | cPr |
| A.1538. | H | CN | H | Et | cPr |
| A.1539. | H | NO₂ | H | Et | cPr |
| A. 1540. | F | H | H | Et | cPr |
| A.1541. | Cl | H | H | Et | cPr |
| A. 1542. | Br | H | H | Et | cPr |
| A. 1543. | CN | H | H | Et | cPr |
| A. 1544. | NO₂ | H | H | Et | cPr |
| A. 1545. | F | F | H | Et | cPr |
| A. 1546. | F | Cl | H | Et | cPr |
| A. 1547. | F | Br | H | Et | cPr |
| A. 1548. | F | CN | H | Et | cPr |
| A. 1549. | F | NO₂ | H | Et | cPr |
| A.1550. | Cl | F | H | Et | cPr |
| A.1551. | Cl | Cl | H | Et | cPr |
| A.1552. | Cl | Br | H | Et | cPr |
| A.1553. | Cl | CN | H | Et | cPr |
| A. 1554. | Cl | NO₂ | H | Et | cPr |
| A.1555. | H | H | H | nPr | cPr |
| A.1556. | H | F | H | nPr | cPr |
| A.1557. | H | Cl | H | nPr | cPr |
| A.1558. | H | Br | H | nPr | cPr |
| A.1559. | H | CN | H | nPr | cPr |
| A.1560. | H | NO₂ | H | nPr | cPr |
| A.1561. | F | H | H | nPr | cPr |
| A.1562. | Cl | H | H | nPr | cPr |
| A.1563. | Br | H | H | nPr | cPr |
| A.1564. | CN | H | H | nPr | cPr |
| A.1565. | NO₂ | H | H | nPr | cPr |
| A. 1566. | F | F | H | nPr | cPr |
| A.1567. | F | Cl | H | nPr | cPr |
| A.1568. | F | Br | H | nPr | cPr |
| A.1569. | F | CN | H | nPr | cPr |
| A.1570. | F | NO₂ | H | nPr | cPr |
| A.1571. | Cl | F | H | nPr | cPr |
| A.1572. | Cl | Cl | H | nPr | cPr |
| A.1573. | Cl | Br | H | nPr | cPr |
| A.1574. | Cl | CN | H | nPr | cPr |
| A.1575. | Cl | NO₂ | H | nPr | cPr |
| A.1576. | H | H | H | iPr | cPr |
| A.1577. | H | F | H | iPr | cPr |
| A.1578. | H | Cl | H | iPr | cPr |
| A.1579. | H | Br | H | iPr | cPr |
| A.1580. | H | CN | H | iPr | cPr |
| A.1581. | H | NO₂ | H | iPr | cPr |
| A.1582. | F | H | H | iPr | cPr |
| A.1583. | Cl | H | H | iPr | cPr |
| A.1584. | Br | H | H | iPr | cPr |
| A.1585. | CN | H | H | iPr | cPr |
| A.1586. | NO₂ | H | H | iPr | cPr |
| A.1587. | F | F | H | iPr | cPr |
| A.1588. | F | Cl | H | iPr | cPr |
| A.1589. | F | Br | H | iPr | cPr |
| A.1590. | F | CN | H | iPr | cPr |
| A.1591. | F | NO₂ | H | iPr | cPr |
| A.1592. | Cl | F | H | iPr | cPr |
| A.1593. | Cl | Cl | H | iPr | cPr |
| A.1594. | Cl | Br | H | iPr | cPr |
| A.1595. | Cl | CN | H | iPr | cPr |
| A.1596. | Cl | NO₂ | H | iPr | cPr |
| A.1597. | H | H | H | vinyl | cPr |
| A.1598. | H | F | H | vinyl | cPr |
| A.1599. | H | Cl | H | vinyl | cPr |
| A.1600. | H | Br | H | vinyl | cPr |
| A.1601. | H | CN | H | vinyl | cPr |
| A.1602. | H | NO₂ | H | vinyl | cPr |
| A.1603. | F | H | H | vinyl | cPr |
| A.1604. | Cl | H | H | vinyl | cPr |
| A.1605. | Br | H | H | vinyl | cPr |
| A.1606. | CN | H | H | vinyl | cPr |
| A.1607. | NO₂ | H | H | vinyl | cPr |
| A.1608. | F | F | H | vinyl | cPr |
| A.1609. | F | Cl | H | vinyl | cPr |
| A.1610. | F | Br | H | vinyl | cPr |
| A.1611. | F | CN | H | vinyl | cPr |
| A.1612. | F | NO₂ | H | vinyl | cPr |
| A.1613. | Cl | F | H | vinyl | cPr |
| A.1614. | Cl | Cl | H | vinyl | cPr |
| A.1615. | Cl | Br | H | vinyl | cPr |
| A.1616. | Cl | CN | H | vinyl | cPr |
| A.1617. | Cl | NO₂ | H | vinyl | cPr |
| A.1618. | H | H | H | allyl | cPr |
| A.1619. | H | F | H | allyl | cPr |
| A.1620. | H | Cl | H | allyl | cPr |
| A.1621. | H | Br | H | allyl | cPr |
| A.1622. | H | CN | H | allyl | cPr |
| A.1623. | H | NO₂ | H | allyl | cPr |
| A. 1624. | F | H | H | allyl | cPr |
| A.1625. | Cl | H | H | allyl | cPr |
| A. 1626. | Br | H | H | allyl | cPr |
| A.1627. | CN | H | H | allyl | cPr |
| A.1628. | NO₂ | H | H | allyl | cPr |
| A.1629. | F | F | H | allyl | cPr |
| A.1630. | F | Cl | H | allyl | cPr |
| A.1631. | F | Br | H | allyl | cPr |
| A.1632. | F | CN | H | allyl | cPr |
| A.1633. | F | NO₂ | H | allyl | cPr |
| A.1634. | Cl | F | H | allyl | cPr |
| A.1635. | Cl | Cl | H | allyl | cPr |
| A.1636. | Cl | Br | H | allyl | cPr |
| A.1637. | Cl | CN | H | allyl | cPr |
| A.1638. | Cl | NO₂ | H | allyl | cPr |
| A.1639. | H | H | H | OMe | cPr |
| A. 1640. | H | F | H | OMe | cPr |
| A.1641. | H | Cl | H | OMe | cPr |
| A.1642. | H | Br | H | OMe | cPr |
| A. 1643. | H | CN | H | OMe | cPr |
| A. 1644. | H | NO₂ | H | OMe | cPr |
| A. 1645. | F | H | H | OMe | cPr |
| A. 1646. | Cl | H | H | OMe | cPr |
| A. 1647. | Br | H | H | OMe | cPr |
| A. 1648. | CN | H | H | OMe | cPr |
| A. 1649. | NO₂ | H | H | OMe | cPr |
| A.1650. | F | F | H | OMe | cPr |
| A.1651. | F | Cl | H | OMe | cPr |
| A.1652. | F | Br | H | OMe | cPr |
| A.1653. | F | CN | H | OMe | cPr |
| A.1654. | F | NO₂ | H | OMe | cPr |
| A.1655. | Cl | F | H | OMe | cPr |
| A.1656. | Cl | Cl | H | OMe | cPr |
| A.1657. | Cl | Br | H | OMe | cPr |
| A.1658. | Cl | CN | H | OMe | cPr |
| A.1659. | Cl | NO₂ | H | OMe | cPr |
| A. 1660. | H | H | H | OEt | cPr |
| A.1661. | H | F | H | OEt | cPr |
| A.1662. | H | Cl | H | OEt | cPr |
| A. 1663. | H | Br | H | OEt | cPr |
| A. 1664. | H | CN | H | OEt | cPr |
| A. 1665. | H | NO₂ | H | OEt | cPr |
| A. 1666. | F | H | H | OEt | cPr |
| A. 1667. | Cl | H | H | OEt | cPr |
| A. 1668. | Br | H | H | OEt | cPr |
| A. 1669. | CN | H | H | OEt | cPr |
| A.1670. | NO₂ | H | H | OEt | cPr |
| A.1671. | F | F | H | OEt | cPr |
| A.1672. | F | Cl | H | OEt | cPr |
| A.1673. | F | Br | H | OEt | cPr |
| A.1674. | F | CN | H | OEt | cPr |
| A.1675. | F | NO₂ | H | OEt | cPr |
| A.1676. | Cl | F | H | OEt | cPr |
| A.1677. | Cl | Cl | H | OEt | cPr |
| A.1678. | Cl | Br | H | OEt | cPr |
| A.1679. | Cl | CN | H | OEt | cPr |
| A.1680. | Cl | NO₂ | H | OEt | cPr |
| A.1681. | H | H | H | Me | cBu |
| A.1682. | H | F | H | Me | cBu |
| A.1683. | H | Cl | H | Me | cBu |
| A.1684. | H | Br | H | Me | cBu |
| A.1685. | H | CN | H | Me | cBu |
| A.1686. | H | NO₂ | H | Me | cBu |
| A.1687. | F | H | H | Me | cBu |
| A.1688. | Cl | H | H | Me | cBu |
| A.1689. | Br | H | H | Me | cBu |
| A.1690. | CN | H | H | Me | cBu |
| A.1691. | NO₂ | H | H | Me | cBu |
| A.1692. | F | F | H | Me | cBu |
| A.1693. | F | Cl | H | Me | cBu |
| A.1694. | F | Br | H | Me | cBu |
| A.1695. | F | CN | H | Me | cBu |
| A.1696. | F | NO₂ | H | Me | cBu |
| A.1697. | Cl | F | H | Me | cBu |
| A.1698. | Cl | Cl | H | Me | cBu |
| A.1699. | Cl | Br | H | Me | cBu |
| A.1700. | Cl | CN | H | Me | cBu |
| A.1701. | Cl | NO₂ | H | Me | cBu |
| A.1702. | H | H | H | Et | cBu |
| A.1703. | H | F | H | Et | cBu |
| A. 1704. | H | Cl | H | Et | cBu |
| A.1705. | H | Br | H | Et | cBu |
| A. 1706. | H | CN | H | Et | cBu |
| A.1707. | H | NO₂ | H | Et | cBu |
| A.1708. | F | H | H | Et | cBu |
| A.1709. | Cl | H | H | Et | cBu |
| A.1710. | Br | H | H | Et | cBu |
| A.1711. | CN | H | H | Et | cBu |
| A.1712. | NO₂ | H | H | Et | cBu |
| A.1713. | F | F | H | Et | cBu |
| A.1714. | F | Cl | H | Et | cBu |
| A.1715. | F | Br | H | Et | cBu |
| A.1716. | F | CN | H | Et | cBu |
| A.1717. | F | NO₂ | H | Et | cBu |
| A.1718. | Cl | F | H | Et | cBu |
| A.1719. | Cl | Cl | H | Et | cBu |
| A.1720. | Cl | Br | H | Et | cBu |
| A.1721. | Cl | CN | H | Et | cBu |
| A.1722. | Cl | NO₂ | H | Et | cBu |
| A.1723. | H | H | H | nPr | cBu |
| A.1724. | H | F | H | nPr | cBu |
| A.1725. | H | Cl | H | nPr | cBu |
| A.1726. | H | Br | H | nPr | cBu |
| A.1727. | H | CN | H | nPr | cBu |
| A.1728. | H | NO₂ | H | nPr | cBu |
| A.1729. | F | H | H | nPr | cBu |
| A.1730. | Cl | H | H | nPr | cBu |
| A.1731. | Br | H | H | nPr | cBu |
| A.1732. | CN | H | H | nPr | cBu |
| A.1733. | NO₂ | H | H | nPr | cBu |
| A.1734. | F | F | H | nPr | cBu |
| A.1735. | F | Cl | H | nPr | cBu |
| A.1736. | F | Br | H | nPr | cBu |
| A.1737. | F | CN | H | nPr | cBu |
| A.1738. | F | NO₂ | H | nPr | cBu |
| A.1739. | Cl | F | H | nPr | cBu |
| A.1740. | Cl | Cl | H | nPr | cBu |
| A.1741. | Cl | Br | H | nPr | cBu |
| A.1742. | Cl | CN | H | nPr | cBu |
| A.1743. | Cl | NO₂ | H | nPr | cBu |
| A. 1744. | H | H | H | iPr | cBu |
| A.1745. | H | F | H | iPr | cBu |
| A.1746. | H | Cl | H | iPr | cBu |
| A.1747. | H | Br | H | iPr | cBu |
| A.1748. | H | CN | H | iPr | cBu |
| A.1749. | H | NO₂ | H | iPr | cBu |
| A.1750. | F | H | H | iPr | cBu |
| A.1751. | Cl | H | H | iPr | cBu |
| A.1752. | Br | H | H | iPr | cBu |
| A.1753. | CN | H | H | iPr | cBu |
| A.1754. | NO₂ | H | H | iPr | cBu |
| A.1755. | F | F | H | iPr | cBu |
| A.1756. | F | Cl | H | iPr | cBu |
| A.1757. | F | Br | H | iPr | cBu |
| A.1758. | F | CN | H | iPr | cBu |
| A.1759. | F | NO₂ | H | iPr | cBu |
| A.1760. | Cl | F | H | iPr | cBu |
| A.1761. | Cl | Cl | H | iPr | cBu |
| A.1762. | Cl | Br | H | iPr | cBu |
| A.1763. | Cl | CN | H | iPr | cBu |
| A.1764. | Cl | NO₂ | H | iPr | cBu |
| A. 1765. | H | H | H | vinyl | cBu |
| A. 1766. | H | F | H | vinyl | cBu |
| A. 1767. | H | Cl | H | vinyl | cBu |
| A. 1768. | H | Br | H | vinyl | cBu |
| A. 1769. | H | CN | H | vinyl | cBu |
| A.1770. | H | NO₂ | H | vinyl | cBu |
| A.1771. | F | H | H | vinyl | cBu |
| A.1772. | Cl | H | H | vinyl | cBu |
| A.1773. | Br | H | H | vinyl | cBu |
| A. 1774. | CN | H | H | vinyl | cBu |
| A.1775. | NO₂ | H | H | vinyl | cBu |
| A. 1776. | F | F | H | vinyl | cBu |
| A.1777. | F | Cl | H | vinyl | cBu |
| A.1778. | F | Br | H | vinyl | cBu |
| A.1779. | F | CN | H | vinyl | cBu |
| A.1780. | F | NO₂ | H | vinyl | cBu |
| A.1781. | Cl | F | H | vinyl | cBu |
| A.1782. | Cl | Cl | H | vinyl | cBu |
| A.1783. | Cl | Br | H | vinyl | cBu |
| A. 1784. | Cl | CN | H | vinyl | cBu |
| A.1785. | Cl | NO₂ | H | vinyl | cBu |
| A. 1786. | H | H | H | allyl | cBu |
| A.1787. | H | F | H | allyl | cBu |
| A.1788. | H | Cl | H | allyl | cBu |
| A.1789. | H | Br | H | allyl | cBu |
| A.1790. | H | CN | H | allyl | cBu |
| A.1791. | H | NO₂ | H | allyl | cBu |
| A.1792. | F | H | H | allyl | cBu |
| A.1793. | Cl | H | H | allyl | cBu |
| A.1794. | Br | H | H | allyl | cBu |
| A.1795. | CN | H | H | allyl | cBu |
| A. 1796. | NO₂ | H | H | allyl | cBu |
| A.1797. | F | F | H | allyl | cBu |
| A.1798. | F | Cl | H | allyl | cBu |
| A.1799. | F | Br | H | allyl | cBu |
| A.1800. | F | CN | H | allyl | cBu |
| A.1801. | F | NO₂ | H | allyl | cBu |
| A.1802. | Cl | F | H | allyl | cBu |
| A.1803. | Cl | Cl | H | allyl | cBu |
| A.1804. | Cl | Br | H | allyl | cBu |
| A.1805. | Cl | CN | H | allyl | cBu |
| A.1806. | Cl | NO₂ | H | allyl | cBu |
| A.1807. | H | H | H | OMe | cBu |
| A.1808. | H | F | H | OMe | cBu |
| A.1809. | H | Cl | H | OMe | cBu |
| A.1810. | H | Br | H | OMe | cBu |
| A.1811. | H | CN | H | OMe | cBu |
| A.1812. | H | NO₂ | H | OMe | cBu |
| A.1813. | F | H | H | OMe | cBu |
| A.1814. | Cl | H | H | OMe | cBu |
| A.1815. | Br | H | H | OMe | cBu |
| A.1816. | CN | H | H | OMe | cBu |
| A.1817. | NO₂ | H | H | OMe | cBu |
| A.1818. | F | F | H | OMe | cBu |
| A.1819. | F | Cl | H | OMe | cBu |
| A.1820. | F | Br | H | OMe | cBu |
| A.1821. | F | CN | H | OMe | cBu |
| A.1822. | F | NO₂ | H | OMe | cBu |
| A.1823. | Cl | F | H | OMe | cBu |
| A.1824. | Cl | Cl | H | OMe | cBu |
| A.1825. | Cl | Br | H | OMe | cBu |
| A.1826. | Cl | CN | H | OMe | cBu |
| A.1827. | Cl | NO₂ | H | OMe | cBu |
| A.1828. | H | H | H | OEt | cBu |
| A.1829. | H | F | H | OEt | cBu |
| A.1830. | H | Cl | H | OEt | cBu |
| A.1831. | H | Br | H | OEt | cBu |
| A.1832. | H | CN | H | OEt | cBu |
| A.1833. | H | NO₂ | H | OEt | cBu |
| A. 1834. | F | H | H | OEt | cBu |
| A.1835. | Cl | H | H | OEt | cBu |
| A. 1836. | Br | H | H | OEt | cBu |
| A.1837. | CN | H | H | OEt | cBu |
| A.1838. | NO₂ | H | H | OEt | cBu |
| A.1839. | F | F | H | OEt | cBu |
| A. 1840. | F | Cl | H | OEt | cBu |
| A.1841. | F | Br | H | OEt | cBu |
| A.1842. | F | CN | H | OEt | cBu |
| A. 1843. | F | NO₂ | H | OEt | cBu |
| A. 1844. | Cl | F | H | OEt | cBu |
| A. 1845. | Cl | Cl | H | OEt | cBu |
| A. 1846. | Cl | Br | H | OEt | cBu |
| A.1847. | Cl | CN | H | OEt | cBu |
| A. 1848. | Cl | NO₂ | H | OEt | cBu |
| A. 1849. | H | H | -(CH₂)₃- | | H |
| A.1850. | H | F | -(CH₂)₃- | | H |
| A.1851. | H | Cl | -(CH₂)₃- | | H |
| A.1852. | H | Br | -(CH₂)₃- | | H |
| A.1853. | H | CN | -(CH₂)₃- | | H |
| A. 1854. | H | NO₂ | -(CH₂)₃- | | H |
| A.1855. | F | H | -(CH₂)₃- | | H |
| A. 1856. | Cl | H | -(CH₂)₃- | | H |
| A.1857. | Br | H | -(CH₂)₃- | | H |
| A.1858. | CN | H | -(CH₂)₃- | | H |
| A.1859. | NO₂ | H | -(CH₂)₃- | | H |
| A. 1860. | F | F | -(CH₂)₃- | | H |
| A.1861. | F | Cl | -(CH₂)₃- | | H |
| A. 1862. | F | Br | -(CH₂)₃- | | H |
| A. 1863. | F | CN | -(CH₂)₃- | | H |
| A. 1864. | F | NO₂ | -(CH₂)₃- | | H |
| A. 1865. | Cl | F | -(CH₂)₃- | | H |
| A. 1866. | Cl | Cl | -(CH₂)₃- | | H |
| A. 1867. | Cl | Br | -(CH₂)₃- | | H |
| A. 1868. | Cl | CN | -(CH₂)₃- | | H |
| A. 1869. | Cl | NO₂ | -(CH₂)₃- | | H |
| A.1870. | H | H | -(CH₂)₃- | | Me |
| A.1871. | H | F | -(CH₂)₃- | | Me |
| A.1872. | H | Cl | -(CH₂)₃- | | Me |
| A.1873. | H | Br | -(CH₂)₃- | | Me |
| A. 1874. | H | CN | -(CH₂)₃- | | Me |
| A.1875. | H | NO₂ | -(CH₂)₃- | | Me |
| A. 1876. | F | H | -(CH₂)₃- | | Me |
| A.1877. | Cl | H | -(CH₂)₃- | | Me |
| A.1878. | Br | H | -(CH₂)₃- | | Me |
| A.1879. | CN | H | -(CH₂)₃- | | Me |
| A.1880. | NO₂ | H | -(CH₂)₃- | | Me |
| A.1881. | F | F | -(CH₂)₃- | | Me |
| A.1882. | F | Cl | -(CH₂)₃- | | Me |
| A.1883. | F | Br | -(CH₂)₃- | | Me |
| A. 1884. | F | CN | -(CH₂)₃- | | Me |
| A.1885. | F | NO₂ | -(CH₂)₃- | | Me |
| A. 1886. | Cl | F | -(CH₂)₃- | | Me |
| A.1887. | Cl | Cl | -(CH₂)₃- | | Me |
| A.1888. | Cl | Br | -(CH₂)₃- | | Me |
| A.1889. | Cl | CN | -(CH₂)₃- | | Me |
| A.1890. | Cl | NO₂ | -(CH₂)₃- | | Me |
| A.1891. | H | H | -(CH₂)₃- | | Et |
| A.1892. | H | F | -(CH₂)₃- | | Et |
| A.1893. | H | Cl | -(CH₂)₃- | | Et |
| A. 1894. | H | Br | -(CH₂)₃- | | Et |
| A.1895. | H | CN | -(CH₂)₃- | | Et |
| A. 1896. | H | NO₂ | -(CH₂)₃- | | Et |
| A.1897. | F | H | -(CH₂)₃- | | Et |
| A.1898. | Cl | H | -(CH₂)₃- | | Et |
| A.1899. | Br | H | -(CH₂)₃- | | Et |
| A.1900. | CN | H | -(CH₂)₃- | | Et |
| A.1901. | NO₂ | H | -(CH₂)₃- | | Et |
| A.1902. | F | F | -(CH₂)₃- | | Et |
| A.1903. | F | Cl | -(CH₂)₃- | | Et |
| A. 1904. | F | Br | -(CH₂)₃- | | Et |
| A.1905. | F | CN | -(CH₂)₃- | | Et |
| A. 1906. | F | NO₂ | -(CH₂)₃- | | Et |
| A.1907. | Cl | F | -(CH₂)₃- | | Et |
| A.1908. | Cl | Cl | -(CH₂)₃- | | Et |
| A.1909. | Cl | Br | -(CH₂)₃- | | Et |
| A.1910. | Cl | CN | -(CH₂)₃- | | Et |
| A.1911. | Cl | NO₂ | -(CH₂)₃- | | Et |
| A.1912. | H | H | -(CH₂)₃- | | nPr |
| A.1913. | H | F | -(CH₂)₃- | | nPr |
| A.1914. | H | Cl | -(CH₂)₃- | | nPr |
| A.1915. | H | Br | -(CH₂)₃- | | nPr |
| A.1916. | H | CN | -(CH₂)₃- | | nPr |
| A.1917. | H | NO₂ | -(CH₂)₃- | | nPr |
| A.1918. | F | H | -(CH₂)₃- | | nPr |
| A.1919. | Cl | H | -(CH₂)₃- | | nPr |
| A.1920. | Br | H | -(CH₂)₃- | | nPr |
| A.1921. | CN | H | -(CH₂)₃- | | nPr |
| A.1922. | NO₂ | H | -(CH₂)₃- | | nPr |
| A.1923. | F | F | -(CH₂)₃- | | nPr |
| A. 1924. | F | Cl | -(CH₂)₃- | | nPr |
| A.1925. | F | Br | -(CH₂)₃- | | nPr |
| A.1926. | F | CN | -(CH₂)₃- | | nPr |
| A.1927. | F | NO₂ | -(CH₂)₃- | | nPr |
| A.1928. | Cl | F | -(CH₂)₃- | | nPr |
| A.1929. | Cl | Cl | -(CH₂)₃- | | nPr |
| A.1930. | Cl | Br | -(CH₂)₃- | | nPr |
| A.1931. | Cl | CN | -(CH₂)₃- | | nPr |
| A.1932. | Cl | NO₂ | -(CH₂)₃- | | nPr |
| A.1933. | H | H | -(CH₂)₃- | | iPr |
| A. 1934. | H | F | -(CH₂)₃- | | iPr |
| A.1935. | H | Cl | -(CH₂)₃- | | iPr |
| A. 1936. | H | Br | -(CH₂)₃- | | iPr |
| A.1937. | H | CN | -(CH₂)₃- | | iPr |
| A.1938. | H | NO₂ | -(CH₂)₃- | | iPr |
| A.1939. | F | H | -(CH₂)₃- | | iPr |
| A. 1940. | Cl | H | -(CH₂)₃- | | iPr |
| A.1941. | Br | H | -(CH₂)₃- | | iPr |
| A.1942. | CN | H | -(CH₂)₃- | | iPr |
| A. 1943. | NO₂ | H | -(CH₂)₃- | | iPr |
| A. 1944. | F | F | -(CH₂)₃- | | iPr |
| A. 1945. | F | Cl | -(CH₂)₃- | | iPr |
| A. 1946. | F | Br | -(CH₂)₃- | | iPr |
| A.1947. | F | CN | -(CH₂)₃- | | iPr |
| A. 1948. | F | NO₂ | -(CH₂)₃- | | iPr |
| A. 1949. | Cl | F | -(CH₂)₃- | | iPr |
| A.1950. | Cl | Cl | -(CH₂)₃- | | iPr |
| A.1951. | Cl | Br | -(CH₂)₃- | | iPr |
| A.1952. | Cl | CN | -(CH₂)₃- | | iPr |
| A.1953. | Cl | NO₂ | -(CH₂)₃- | | iPr |
| A. 1954. | H | H | -(CH₂)₃- | | nBu |
| A.1955. | H | F | -(CH₂)₃- | | nBu |
| A. 1956. | H | Cl | -(CH₂)₃- | | nBu |
| A.1957. | H | Br | -(CH₂)₃- | | nBu |
| A.1958. | H | CN | -(CH₂)₃- | | nBu |
| A.1959. | H | NO₂ | -(CH₂)₃- | | nBu |
| A. 1960. | F | H | -(CH₂)₃- | | nBu |
| A.1961. | Cl | H | -(CH₂)₃- | | nBu |
| A. 1962. | Br | H | -(CH₂)₃- | | nBu |
| A.1963. | CN | H | -(CH₂)₃- | | nBu |
| A. 1964. | NO₂ | H | -(CH₂)₃- | | nBu |
| A. 1965. | F | F | -(CH₂)₃- | | nBu |
| A. 1966. | F | Cl | -(CH₂)₃- | | nBu |
| A. 1967. | F | Br | -(CH₂)₃- | | nBu |
| A.1968. | F | CN | -(CH₂)₃- | | nBu |
| A. 1969. | F | NO₂ | -(CH₂)₃- | | nBu |
| A.1970. | Cl | F | -(CH₂)₃- | | nBu |
| A.1971. | Cl | Cl | -(CH₂)₃- | | nBu |
| A.1972. | Cl | Br | -(CH₂)₃- | | nBu |
| A.1973. | Cl | CN | -(CH₂)₃- | | nBu |
| A. 1974. | Cl | NO₂ | -(CH₂)₃- | | nBu |
| A.1975. | H | H | -(CH₂)₃- | | 2Bu |
| A. 1976. | H | F | -(CH₂)₃- | | 2Bu |
| A.1977. | H | Cl | -(CH₂)₃- | | 2Bu |
| A.1978. | H | Br | -(CH₂)₃- | | 2Bu |
| A.1979. | H | CN | -(CH₂)₃- | | 2Bu |
| A.1980. | H | NO₂ | -(CH₂)₃- | | 2Bu |
| A.1981. | F | H | -(CH₂)₃- | | 2Bu |
| A.1982. | Cl | H | -(CH₂)₃- | | 2Bu |
| A.1983. | Br | H | -(CH₂)₃- | | 2Bu |
| A.1984. | CN | H | -(CH₂)₃- | | 2Bu |
| A.1985. | NO₂ | H | -(CH₂)₃- | | 2Bu |
| A. 1986. | F | F | -(CH₂)₃- | | 2Bu |
| A.1987. | F | Cl | -(CH₂)₃- | | 2Bu |
| A.1988. | F | Br | -(CH₂)₃- | | 2Bu |
| A.1989. | F | CN | -(CH₂)₃- | | 2Bu |
| A.1990. | F | NO₂ | -(CH₂)₃- | | 2Bu |
| A.1991. | Cl | F | -(CH₂)₃- | | 2Bu |
| A.1992. | Cl | Cl | -(CH₂)₃- | | 2Bu |
| A.1993. | Cl | Br | -(CH₂)₃- | | 2Bu |
| A.1994. | Cl | CN | -(CH₂)₃- | | 2Bu |
| A.1995. | Cl | NO₂ | -(CH₂)₃- | | 2Bu |
| A. 1996. | H | H | -(CH₂)₃- | | iBu |
| A.1997. | H | F | -(CH₂)₃- | | iBu |
| A.1998. | H | Cl | -(CH₂)₃- | | iBu |
| A.1999. | H | Br | -(CH₂)₃- | | iBu |
| A.2000. | H | CN | -(CH₂)₃- | | iBu |
| A.2001. | H | NO₂ | -(CH₂)₃- | | iBu |
| A.2002. | F | H | -(CH₂)₃- | | iBu |
| A.2003. | Cl | H | -(CH₂)₃- | | iBu |
| A.2004. | Br | H | -(CH₂)₃- | | iBu |
| A.2005. | CN | H | -(CH₂)₃- | | iBu |
| A.2006. | NO₂ | H | -(CH₂)₃- | | iBu |
| A.2007. | F | F | -(CH₂)₃- | | iBu |
| A.2008. | F | Cl | -(CH₂)₃- | | iBu |
| A.2009. | F | Br | -(CH₂)₃- | | iBu |
| A.2010. | F | CN | -(CH₂)₃- | | iBu |
| A.2011. | F | NO₂ | -(CH₂)₃- | | iBu |
| A.2012. | Cl | F | -(CH₂)₃- | | iBu |
| A.2013. | Cl | Cl | -(CH₂)₃- | | iBu |
| A.2014. | Cl | Br | -(CH₂)₃- | | iBu |
| A.2015. | Cl | CN | -(CH₂)₃- | | iBu |
| A.2016. | Cl | NO₂ | -(CH₂)₃- | | iBu |
| A.2017. | H | H | -(CH₂)₃- | | tBu |
| A.2018. | H | F | -(CH₂)₃- | | tBu |
| A.2019. | H | Cl | -(CH₂)₃- | | tBu |
| A.2020. | H | Br | -(CH₂)₃- | | tBu |
| A.2021. | H | CN | -(CH₂)₃- | | tBu |
| A.2022. | H | NO₂ | -(CH₂)₃- | | tBu |
| A.2023. | F | H | -(CH₂)₃- | | tBu |
| A.2024. | Cl | H | -(CH₂)₃- | | tBu |
| A.2025. | Br | H | -(CH₂)₃- | | tBu |
| A.2026. | CN | H | -(CH₂)₃- | | tBu |
| A.2027. | NO₂ | H | -(CH₂)₃- | | tBu |
| A.2028. | F | F | -(CH₂)₃- | | tBu |
| A.2029. | F | Cl | -(CH₂)₃- | | tBu |
| A.2030. | F | Br | -(CH₂)₃- | | tBu |
| A.2031. | F | CN | -(CH₂)₃- | | tBu |
| A.2032. | F | NO₂ | -(CH₂)₃- | | tBu |
| A.2033. | Cl | F | -(CH₂)₃- | | tBu |
| A.2034. | Cl | Cl | -(CH₂)₃- | | tBu |
| A.2035. | Cl | Br | -(CH₂)₃- | | tBu |
| A.2036. | Cl | CN | -(CH₂)₃- | | tBu |
| A.2037. | Cl | NO₂ | -(CH₂)₃- | | tBu |
| A.2038. | H | H | -(CH₂)₃- | | cPr |
| A.2039. | H | F | -(CH₂)₃- | | cPr |
| A.2040. | H | Cl | -(CH₂)₃- | | cPr |
| A.2041. | H | Br | -(CH₂)₃- | | cPr |
| A.2042. | H | CN | -(CH₂)₃- | | cPr |
| A.2043. | H | NO₂ | -(CH₂)₃- | | cPr |
| A.2044. | F | H | -(CH₂)₃- | | cPr |
| A.2045. | Cl | H | -(CH₂)₃- | | cPr |
| A.2046. | Br | H | -(CH₂)₃- | | cPr |
| A.2047. | CN | H | -(CH₂)₃- | | cPr |
| A.2048. | NO₂ | H | -(CH₂)₃- | | cPr |
| A.2049. | F | F | -(CH₂)₃- | | cPr |
| A.2050. | F | Cl | -(CH₂)₃- | | cPr |
| A.2051. | F | Br | -(CH₂)₃- | | cPr |
| A.2052. | F | CN | -(CH₂)₃- | | cPr |
| A.2053. | F | NO₂ | -(CH₂)₃- | | cPr |
| A.2054. | Cl | F | -(CH₂)₃- | | cPr |
| A.2055. | Cl | Cl | -(CH₂)₃- | | cPr |
| A.2056. | Cl | Br | -(CH₂)₃- | | cPr |
| A.2057. | Cl | CN | -(CH₂)₃- | | cPr |
| A.2058. | Cl | NO₂ | -(CH₂)₃- | | cPr |
| A.2059. | H | H | -(CH₂)₃- | | cBu |
| A.2060. | H | F | -(CH₂)₃- | | cBu |
| A.2061. | H | Cl | -(CH₂)₃- | | cBu |
| A.2062. | H | Br | -(CH₂)₃- | | cBu |
| A.2063. | H | CN | -(CH₂)₃- | | cBu |
| A.2064. | H | NO₂ | -(CH₂)₃- | | cBu |
| A.2065. | F | H | -(CH₂)₃- | | cBu |
| A.2066. | Cl | H | -(CH₂)₃- | | cBu |
| A.2067. | Br | H | -(CH₂)₃- | | cBu |
| A.2068. | CN | H | -(CH₂)₃- | | cBu |
| A.2069. | NO₂ | H | -(CH₂)₃- | | cBu |
| A.2070. | F | F | -(CH₂)₃- | | cBu |
| A.2071. | F | Cl | -(CH₂)₃- | | cBu |
| A.2072. | F | Br | -(CH₂)₃- | | cBu |
| A.2073. | F | CN | -(CH₂)₃- | | cBu |
| A.2074. | F | NO₂ | -(CH₂)₃- | | cBu |
| A.2075. | Cl | F | -(CH₂)₃- | | cBu |
| A.2076. | Cl | Cl | -(CH₂)₃- | | cBu |
| A.2077. | Cl | Br | -(CH₂)₃- | | cBu |
| A.2078. | Cl | CN | -(CH₂)₃- | | cBu |
| A.2079. | Cl | NO₂ | -(CH₂)₃- | | cBu |
| A.2080. | H | H | -(CH₂)₂- | | H |
| A.2081. | H | F | -(CH₂)₂- | | H |
| A.2082. | H | Cl | -(CH₂)₂- | | H |
| A.2083. | H | Br | -(CH₂)₂- | | H |
| A.2084. | H | CN | -(CH₂)₂- | | H |
| A.2085. | H | NO₂ | -(CH₂)₂- | | H |
| A.2086. | F | H | -(CH₂)₂- | | H |
| A.2087. | Cl | H | -(CH₂)₂- | | H |
| A.2088. | Br | H | -(CH₂)₂- | | H |
| A.2089. | CN | H | -(CH₂)₂- | | H |
| A.2090. | NO₂ | H | -(CH₂)₂- | | H |
| A.2091. | F | F | -(CH₂)₂- | | H |
| A.2092. | F | Cl | -(CH₂)₂- | | H |
| A.2093. | F | Br | -(CH₂)₂- | | H |
| A.2094. | F | CN | -(CH₂)₂- | | H |
| A.2095. | F | NO₂ | -(CH₂)₂- | | H |
| A.2096. | Cl | F | -(CH₂)₂- | | H |
| A.2097. | Cl | Cl | -(CH₂)₂- | | H |
| A.2098. | Cl | Br | -(CH₂)₂- | | H |
| A.2099. | Cl | CN | -(CH₂)₂- | | H |
| A.2100. | Cl | NO₂ | -(CH₂)₂- | | H |
| A.2101. | H | H | -(CH₂)₂- | | Me |
| A.2102. | H | F | -(CH₂)₂- | | Me |
| A.2103. | H | Cl | -(CH₂)₂- | | Me |
| A.2104. | H | Br | -(CH₂)₂- | | Me |
| A.2105. | H | CN | -(CH₂)₂- | | Me |
| A.2106. | H | NO₂ | -(CH₂)₂- | | Me |
| A.2107. | F | H | -(CH₂)₂- | | Me |
| A.2108. | Cl | H | -(CH₂)₂- | | Me |
| A.2109. | Br | H | -(CH₂)₂- | | Me |
| A.2110. | CN | H | -(CH₂)₂- | | Me |
| A.2111. | NO₂ | H | -(CH₂)₂- | | Me |
| A.2112. | F | F | -(CH₂)₂- | | Me |
| A.2113. | F | Cl | -(CH₂)₂- | | Me |
| A.2114. | F | Br | -(CH₂)₂- | | Me |
| A.2115. | F | CN | -(CH₂)₂- | | Me |
| A.2116. | F | NO₂ | -(CH₂)₂- | | Me |
| A.2117. | Cl | F | -(CH₂)₂- | | Me |
| A.2118. | Cl | Cl | -(CH₂)₂- | | Me |
| A.2119. | Cl | Br | -(CH₂)₂- | | Me |
| A.2120. | Cl | CN | -(CH₂)₂- | | Me |
| A.2121. | Cl | NO₂ | -(CH₂)₂- | | Me |
| A.2122. | H | H | -(CH₂)₂- | | Et |
| A.2123. | H | F | -(CH₂)₂- | | Et |
| A.2124. | H | Cl | -(CH₂)₂- | | Et |
| A.2125. | H | Br | -(CH₂)₂- | | Et |
| A.2126. | H | CN | -(CH₂)₂- | | Et |
| A.2127. | H | NO₂ | -(CH₂)₂- | | Et |
| A.2128. | F | H | -(CH₂)₂- | | Et |
| A.2129. | Cl | H | -(CH₂)₂- | | Et |
| A.2130. | Br | H | -(CH₂)₂- | | Et |
| A.2131. | CN | H | -(CH₂)₂- | | Et |
| A.2132. | NO₂ | H | -(CH₂)₂- | | Et |
| A.2133. | F | F | -(CH₂)₂- | | Et |
| A.2134. | F | Cl | -(CH₂)₂- | | Et |
| A.2135. | F | Br | -(CH₂)₂- | | Et |
| A.2136. | F | CN | -(CH₂)₂- | | Et |
| A.2137. | F | NO₂ | -(CH₂)₂- | | Et |
| A.2138. | Cl | F | -(CH₂)₂- | | Et |
| A.2139. | Cl | Cl | -(CH₂)₂- | | Et |
| A.2140. | Cl | Br | -(CH₂)₂- | | Et |
| A.2141. | Cl | CN | -(CH₂)₂- | | Et |
| A.2142. | Cl | NO₂ | -(CH₂)₂- | | Et |
| A.2143. | H | H | -(CH₂)₂- | | nPr |
| A.2144. | H | F | -(CH₂)₂- | | nPr |
| A.2145. | H | Cl | -(CH₂)₂- | | nPr |
| A.2146. | H | Br | -(CH₂)₂- | | nPr |
| A.2147. | H | CN | -(CH₂)₂- | | nPr |
| A.2148. | H | NO₂ | -(CH₂)₂- | | nPr |
| A.2149. | F | H | -(CH₂)₂- | | nPr |
| A.2150. | Cl | H | -(CH₂)₂- | | nPr |
| A.2151. | Br | H | -(CH₂)₂- | | nPr |
| A.2152. | CN | H | -(CH₂)₂- | | nPr |
| A.2153. | NO₂ | H | -(CH₂)₂- | | nPr |
| A.2154. | F | F | -(CH₂)₂- | | nPr |
| A.2155. | F | Cl | -(CH₂)₂- | | nPr |
| A.2156. | F | Br | -(CH₂)₂- | | nPr |
| A.2157. | F | CN | -(CH₂)₂- | | nPr |
| A.2158. | F | NO₂ | -(CH₂)₂- | | nPr |
| A.2159. | Cl | F | -(CH₂)₂- | | nPr |
| A.2160. | Cl | Cl | -(CH₂)₂- | | nPr |
| A.2161. | Cl | Br | -(CH₂)₂- | | nPr |
| A.2162. | Cl | CN | -(CH₂)₂- | | nPr |
| A.2163. | Cl | NO₂ | -(CH₂)₂- | | nPr |
| A.2164. | H | H | -(CH₂)₂- | | iPr |
| A.2165. | H | F | -(CH₂)₂- | | iPr |
| A.2166. | H | Cl | -(CH₂)₂- | | iPr |
| A.2167. | H | Br | -(CH₂)₂- | | iPr |
| A.2168. | H | CN | -(CH₂)₂- | | iPr |
| A.2169. | H | NO₂ | -(CH₂)₂- | | iPr |
| A.2170. | F | H | -(CH₂)₂- | | iPr |
| A.2171. | Cl | H | -(CH₂)₂- | | iPr |
| A.2172. | Br | H | -(CH₂)₂- | | iPr |
| A.2173. | CN | H | -(CH₂)₂- | | iPr |
| A.2174. | NO₂ | H | -(CH₂)₂- | | iPr |
| A.2175. | F | F | -(CH₂)₂- | | iPr |
| A.2176. | F | Cl | -(CH₂)₂- | | iPr |
| A.2177. | F | Br | -(CH₂)₂- | | iPr |
| A.2178. | F | CN | -(CH₂)₂- | | iPr |
| A.2179. | F | NO₂ | -(CH₂)₂- | | iPr |
| A.2180. | Cl | F | -(CH₂)₂- | | iPr |
| A.2181. | Cl | Cl | -(CH₂)₂- | | iPr |
| A.2182. | Cl | Br | -(CH₂)₂- | | iPr |
| A.2183. | Cl | CN | -(CH₂)₂- | | iPr |
| A.2184. | Cl | NO₂ | -(CH₂)₂- | | iPr |
| A.2185. | H | H | -(CH₂)₂- | | nBu |
| A.2186. | H | F | -(CH₂)₂- | | nBu |
| A.2187. | H | Cl | -(CH₂)₂- | | nBu |
| A.2188. | H | Br | -(CH₂)₂- | | nBu |
| A.2189. | H | CN | -(CH₂)₂- | | nBu |
| A.2190. | H | NO₂ | -(CH₂)₂- | | nBu |
| A.2191. | F | H | -(CH₂)₂- | | nBu |
| A.2192. | Cl | H | -(CH₂)₂- | | nBu |
| A.2193. | Br | H | -(CH₂)₂- | | nBu |
| A.2194. | CN | H | -(CH₂)₂- | | nBu |
| A.2195. | NO₂ | H | -(CH₂)₂- | | nBu |
| A.2196. | F | F | -(CH₂)₂- | | nBu |
| A.2197. | F | Cl | -(CH₂)₂- | | nBu |
| A.2198. | F | Br | -(CH₂)₂- | | nBu |
| A.2199. | F | CN | -(CH₂)₂- | | nBu |
| A.2200. | F | NO₂ | -(CH₂)₂- | | nBu |
| A.2201. | Cl | F | -(CH₂)₂- | | nBu |
| A.2202. | Cl | Cl | -(CH₂)₂- | | nBu |
| A.2203. | Cl | Br | -(CH₂)₂- | | nBu |
| A.2204. | Cl | CN | -(CH₂)₂- | | nBu |
| A.2205. | Cl | NO₂ | -(CH₂)₂- | | nBu |
| A.2206. | H | H | -(CH₂)₂- | | 2Bu |
| A.2207. | H | F | -(CH₂)₂- | | 2Bu |
| A.2208. | H | Cl | -(CH₂)₂- | | 2Bu |
| A.2209. | H | Br | -(CH₂)₂- | | 2Bu |
| A.2210. | H | CN | -(CH₂)₂- | | 2Bu |
| A.2211. | H | NO₂ | -(CH₂)₂- | | 2Bu |
| A.2212. | F | H | -(CH₂)₂- | | 2Bu |
| A.2213. | Cl | H | -(CH₂)₂- | | 2Bu |
| A.2214. | Br | H | -(CH₂)₂- | | 2Bu |
| A.2215. | CN | H | -(CH₂)₂- | | 2Bu |
| A.2216. | NO₂ | H | -(CH₂)₂- | | 2Bu |
| A.2217. | F | F | -(CH₂)₂- | | 2Bu |
| A.2218. | F | Cl | -(CH₂)₂- | | 2Bu |
| A.2219. | F | Br | -(CH₂)₂- | | 2Bu |
| A.2220. | F | CN | -(CH₂)₂- | | 2Bu |
| A.2221. | F | NO₂ | -(CH₂)₂- | | 2Bu |
| A.2222. | Cl | F | -(CH₂)₂- | | 2Bu |
| A.2223. | Cl | Cl | -(CH₂)₂- | | 2Bu |
| A.2224. | Cl | Br | -(CH₂)₂- | | 2Bu |
| A.2225. | Cl | CN | -(CH₂)₂- | | 2Bu |
| A.2226. | Cl | NO₂ | -(CH₂)₂- | | 2Bu |
| A.2227. | H | H | -(CH₂)₂- | | iBu |
| A.2228. | H | F | -(CH₂)₂- | | iBu |
| A.2229. | H | Cl | -(CH₂)₂- | | iBu |
| A.2230. | H | Br | -(CH₂)₂- | | iBu |
| A.2231. | H | CN | -(CH₂)₂- | | iBu |
| A.2232. | H | NO₂ | -(CH₂)₂- | | iBu |
| A.2233. | F | H | -(CH₂)₂- | | iBu |
| A.2234. | Cl | H | -(CH₂)₂- | | iBu |
| A.2235. | Br | H | -(CH₂)₂- | | iBu |
| A.2236. | CN | H | -(CH₂)₂- | | iBu |
| A.2237. | NO₂ | H | -(CH₂)₂- | | iBu |
| A.2238. | F | F | -(CH₂)₂- | | iBu |
| A.2239. | F | Cl | -(CH₂)₂- | | iBu |
| A.2240. | F | Br | -(CH₂)₂- | | iBu |
| A.2241. | F | CN | -(CH₂)₂- | | iBu |
| A.2242. | F | NO₂ | -(CH₂)₂- | | iBu |
| A.2243. | Cl | F | -(CH₂)₂- | | iBu |
| A.2244. | Cl | Cl | -(CH₂)₂- | | iBu |
| A.2245. | Cl | Br | -(CH₂)₂- | | iBu |
| A.2246. | Cl | CN | -(CH₂)₂- | | iBu |
| A.2247. | Cl | NO₂ | -(CH₂)₂- | | iBu |
| A.2248. | H | H | -(CH₂)₂- | | tBu |
| A.2249. | H | F | -(CH₂)₂- | | tBu |
| A.2250. | H | Cl | -(CH₂)₂- | | tBu |
| A.2251. | H | Br | -(CH₂)₂- | | tBu |
| A.2252. | H | CN | -(CH₂)₂- | | tBu |
| A.2253. | H | NO₂ | -(CH₂)₂- | | tBu |
| A.2254. | F | H | -(CH₂)₂- | | tBu |
| A.2255. | Cl | H | -(CH₂)₂- | | tBu |
| A.2256. | Br | H | -(CH₂)₂- | | tBu |
| A.2257. | CN | H | -(CH₂)₂- | | tBu |
| A.2258. | NO₂ | H | -(CH₂)₂- | | tBu |
| A.2259. | F | F | -(CH₂)₂- | | tBu |
| A.2260. | F | Cl | -(CH₂)₂- | | tBu |
| A.2261. | F | Br | -(CH₂)₂- | | tBu |
| A.2262. | F | CN | -(CH₂)₂- | | tBu |
| A.2263. | F | NO₂ | -(CH₂)₂- | | tBu |
| A.2264. | Cl | F | -(CH₂)₂- | | tBu |
| A.2265. | Cl | Cl | -(CH₂)₂- | | tBu |
| A.2266. | Cl | Br | -(CH₂)₂- | | tBu |
| A.2267. | Cl | CN | -(CH₂)₂- | | tBu |
| A.2268. | Cl | NO₂ | -(CH₂)₂- | | tBu |
| A.2269. | H | H | -(CH₂)₂- | | cPr |
| A.2270. | H | F | -(CH₂)₂- | | cPr |
| A.2271. | H | Cl | -(CH₂)₂- | | cPr |
| A.2272. | H | Br | -(CH₂)₂- | | cPr |
| A.2273. | H | CN | -(CH₂)₂- | | cPr |
| A.2274. | H | NO₂ | -(CH₂)₂- | | cPr |
| A.2275. | F | H | -(CH₂)₂- | | cPr |
| A.2276. | Cl | H | -(CH₂)₂- | | cPr |
| A.2277. | Br | H | -(CH₂)₂- | | cPr |
| A.2278. | CN | H | -(CH₂)₂- | | cPr |
| A.2279. | NO₂ | H | -(CH₂)₂- | | cPr |
| A.2280. | F | F | -(CH₂)₂- | | cPr |
| A.2281. | F | Cl | -(CH₂)₂- | | cPr |
| A.2282. | F | Br | -(CH₂)₂- | | cPr |
| A.2283. | F | CN | -(CH₂)₂- | | cPr |
| A.2284. | F | NO₂ | -(CH₂)₂- | | cPr |
| A.2285. | Cl | F | -(CH₂)₂- | | cPr |
| A.2286. | Cl | Cl | -(CH₂)₂- | | cPr |
| A.2287. | Cl | Br | -(CH₂)₂- | | cPr |
| A.2288. | Cl | CN | -(CH₂)₂- | | cPr |
| A.2289. | Cl | NO₂ | -(CH₂)₂- | | cPr |
| A.2290. | H | H | -(CH₂)₂- | | cBu |
| A.2291. | H | F | -(CH₂)₂- | | cBu |
| A.2292. | H | Cl | -(CH₂)₂- | | cBu |
| A.2293. | H | Br | -(CH₂)₂- | | cBu |
| A.2294. | H | CN | -(CH₂)₂- | | cBu |
| A.2295. | H | NO₂ | -(CH₂)₂- | | cBu |
| A.2296. | F | H | -(CH₂)₂- | | cBu |
| A.2297. | Cl | H | -(CH₂)₂- | | cBu |
| A.2298. | Br | H | -(CH₂)₂- | | cBu |
| A.2299. | CN | H | -(CH₂)₂- | | cBu |
| A.2300. | NO₂ | H | -(CH₂)₂- | | cBu |
| A.2301. | F | F | -(CH₂)₂- | | cBu |
| A.2302. | F | Cl | -(CH₂)₂- | | cBu |
| A.2303. | F | Br | -(CH₂)₂- | | cBu |
| A.2304. | F | CN | -(CH₂)₂- | | cBu |
| A.2305. | F | NO₂ | -(CH₂)₂- | | cBu |
| A.2306. | Cl | F | -(CH₂)₂- | | cBu |
| A.2307. | Cl | Cl | -(CH₂)₂- | | cBu |
| A.2308. | Cl | Br | -(CH₂)₂- | | cBu |
| A.2309. | Cl | CN | -(CH₂)₂- | | cBu |
| A.2310. | Cl | NO₂ | -(CH₂)₂- | | cBu |

- Me: methyl
- Et: ethyl
- nPr: n-propyl
- iPr: isopropyl
- nBu: n-butyl
- 2Bu: sec-butyl
- iBu: isobutyl
- tBu: tert-butyl
- OMe: methoxy
- OEt: ethoxy
- cPr: cyclopropyl
- cBu: cyclobutyl

The compounds of formula (I) according to the invention can be prepared by standard processes of organic chemistry, for example by the following processes:
Compounds (I) can be prepared by reacting the isoxazoline-substituted phenolic compound 1 first with thiophosgene and then with the amine NHR¹R², as shown in scheme 1. Due to the exothermy of the conversion, the reaction is generally carried out at rather low temperatures, e.g. at or below room temperature or around 0°C. The reaction conditions are generally anhydrous.

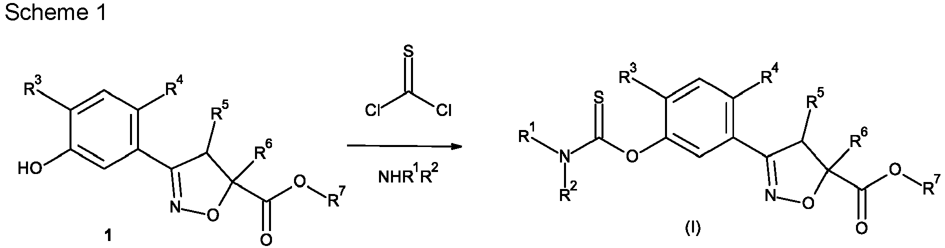

Compounds **1** can in turn be prepared by a [3+2] Huisgen cycloaddition of compounds **2** and **3,** as shown in scheme 2. R is H or a protective group, such as TIPS, and R' is H or a chlorine atom. If R' is H, the cycloaddition is generally carried out in the presence of an oxidizing agent, such as meta-chloroperbenzoi acid. If R⁶ in the final compound is to be an alkenyl or haloalkenyl group, compound **3** expediently carries a precursor group instead of the substituent R⁶.

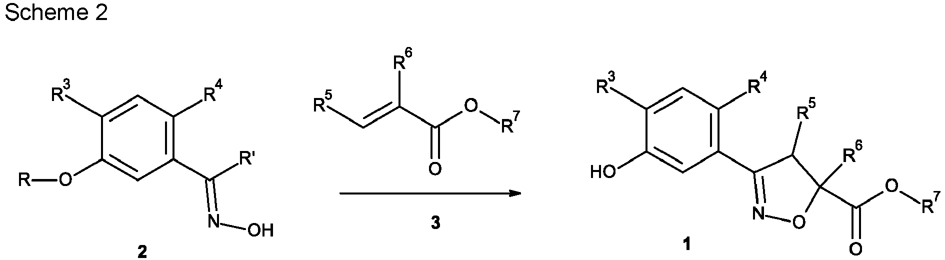

Compounds **2** can be prepared by the reaction sequences shown in schemes 3 and 4. A bromine-substituted phenolic compound **4** or **8,** in which the hydroxyl group is etherified or protected by a protective group PG, such as TIPS, is reacted in a Grignard (anaologous) reaction with Li or Mg organyl, such as n-BuLi or isopropylmagnesium chloride to the aldehyde **6** or **9,** respectively, which, after optional deprotection/ether hydrolysisis, is reacted with hydroxylamine to the hydroxylamine **2'** or **2".** To obtain compounds **2** wherein R' is Cl (see **2‴** in scheme 4; here R is a protective group PG), compounds **2** wherein R' is H are reacted with a suitable chlorimation agent, such as N-chlorosuccinimide (NCS). If R³ and/or R⁴ are Br or I, it might be expedient to change the reaction sequence to avoid the production of undesired side-products in competing reactions. For instance, the Br or I substituent could be introduced into a suitable benzaldehyde precursor. Several compounds 5 or 6, wherein R⁴ is Br, are moreover commercially available.

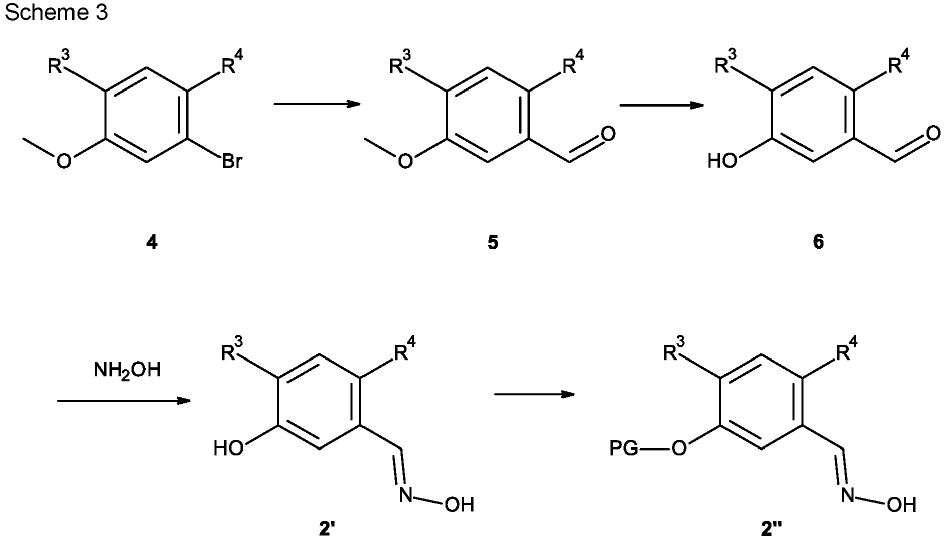

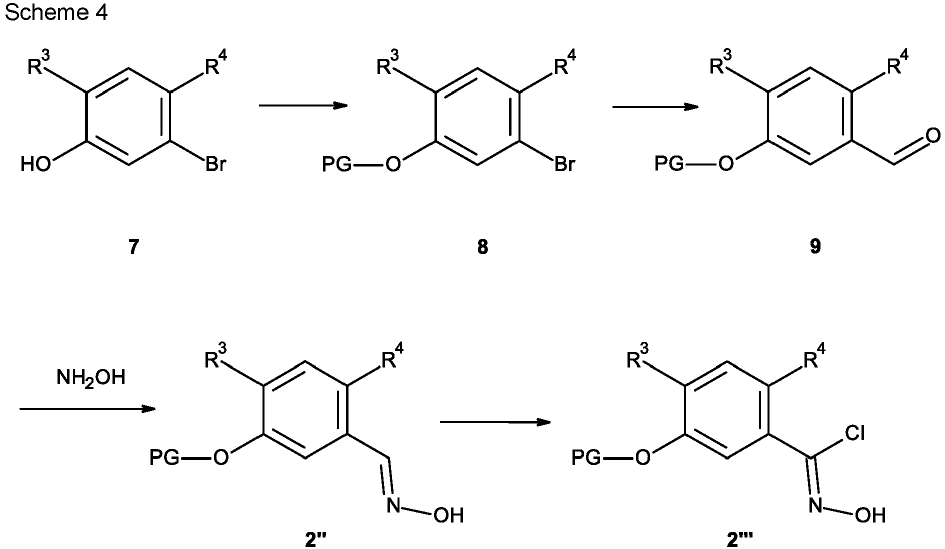

Compounds 3 are either commercially available or can be prepared by standards methods of organic chemistry. Just by way of example, the synthesis of a compound 3 wherein R⁵ is H, R⁶ is methoxy and R⁷ is methyl (termed **3'** below) is shown in scheme 5. Methyl 2-oxopropanoate **10** is converted with trimethyl orthoformate into the hemiacetal. Dehydration thereof, e.g. with P₂O₅, yields methyl 2-methoxyprop-2-enoate **3'.**

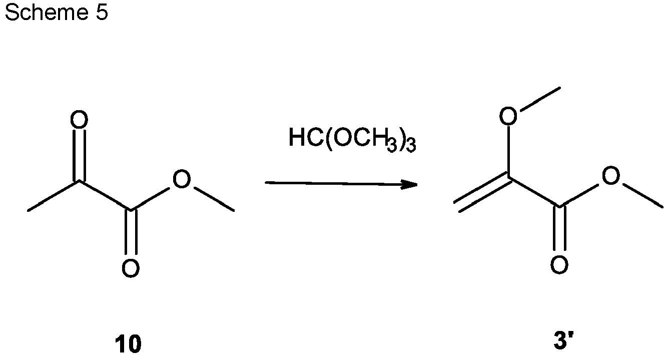

As mentioned above, to obtain compounds (I) wherein If R⁶ is an alkenyl or haloalkenyl group, compound **3** expediently carries a precursor group instead of the substituent R⁶. To obtain for example a compounds(I) wherein R⁶ is vinyl, a possible R⁶ precursor group in compound **3** is ethyl carrying a leaving group, e.g. a triflate group in 1-position. After the Huisgen cycloaddition, trifluorosulfonic acid is eliminated, e.g. using a suitable base, yielding a vinyl group.

To prepare salts of compounds (I), the respective carboxylic acids, i.e. compounds (I) wherein R⁷ is H, can be reacted with a suitablbe base, such as Li, Na or K hydroxide or carbonate.

To widen the spectrum of action, the compounds of formula (I) may be mixed with many representatives of other herbicidal or growth-regulating active ingredient groups and then applied concomitantly. Suitable components for combinations are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

It may furthermore be beneficial to apply the compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

In one embodiment of the present invention the combinations according to the present invention comprise at least one compound of formula (I) (compound A or component A) and at least one further active compound selected from herbicides B (compound B), preferably herbicides B of class b1) to b15), and safeners C (compound C).

In another embodiment of the present invention the combinations according to the present invention comprise at least one compound of formula (I) and at least one further active compound B (herbicide B).

Examples of herbicides B which can be used in combination with the *compounds* A of formula (I) according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron, imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam, pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8), sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone; among these, a preferred embodiment of the invention relates to those compositions comprising at least one imidazolinone herbicide;
b3) from the group of the photosynthesis inhibitors:
   amicarbazone, inhibitors of the photosystem II, e.g. 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8), 1-(5-tert-butylisoxazol-3-yl)-4-ethoxy-5-hydroxy-3-methyl-imidazolidin-2-one (CAS 1844836-64-1), triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, chlorphthalim, cinidon-ethyl, cyclopyranil, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoro-methylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), methyl (*E*)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4), 2-[2-chloro-5-[3-chloro-5-(trifluoromethyl)-2-pyridinyl]-4-fluorophenoxy]-2-methoxy-acetic acid methyl ester (CAS 1970221-16-9), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-acetic acid methyl ester (CAS 2158274-96-3), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy] acetic acid ethyl ester (CAS 158274-50-9), methyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2271389-22-9), ethyl 2-[[3-[2-chloro-5-[4-(difluoromethyl)-3-methyl-5-oxo-1,2,4-triazol-1-yl]-4-fluoro-phenoxy]-2-pyridyl]oxy]acetate (CAS 2230679-62-4), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-acetic acid methyl ester (CAS 2158275-73-9), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy] acetic acid ethyl ester (CAS 2158274-56-5), 2-[2-[[3-chloro-6-[3,6-dihydro-3-methyl-2,6-dioxo-4-(trifluoromethyl)-1(2H)-pyrimidinyl]-5-fluoro-2-pyridinyl]oxy]phenoxy]-N-(methylsulfonyl)-acetamide (CAS 2158274-53-2), 2-[[3-[[3-chloro-6-[3,6-dihydro-3-methyl-2,6-dioxo-4-(trifluoromethyl)-1(2H)-pyrimidinyl]-5-fluoro-2-pyridinyl]oxy]-2-pyridinyl]oxy]-N-(methylsulfonyl)-acetamide (CAS 2158276-22-1);
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)-pyrimidine (CAS 180608-33-7), HPPD inhibitors: benzobicyclon, benzofenap, bicyclopyrone, clomazone, fenquinotrione, isoxaflutole, mesotrione, oxotrione (CAS 1486617-21-3), pyrasulfotole, pyrazolynate, pyrazoxyfen, sulcotrione, tefuryltrione, tembotrione, tolpyralate, topramezone , bleacher, unknown target: aclonifen, amitrole flumeturon 2-chloro-3-methylsulfanyl-N-(1-methyltetrazol-5-yl)-4-(trifluoromethyl)benzamide (CAS 1361139-71-0), bixlozone and 2-(2,5-dichlorophenyl)methyl-4,4-dimethyl-3-isoxazolidinone (CAS 81778-66-7);
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, amidochlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufe-nacet and mefenacet, acetanilides such as diphenamid, naproanilide, napropamide and napropamide-M, tetrazolinones such fentrazamide, and other herbicides such as anilofos, cafenstrole, fenoxasulfone, ipfencarbazone, piperophos, pyroxasulfone and isoxazoline compounds of the formulae II.1, II.2, II.3, II.4, II.5, II.6, II.7, II.8 and II.9 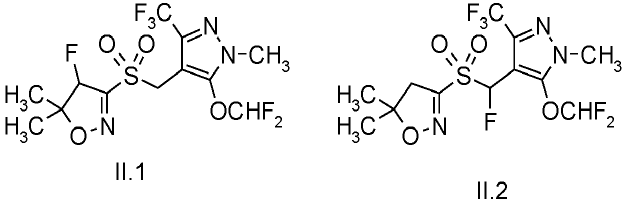 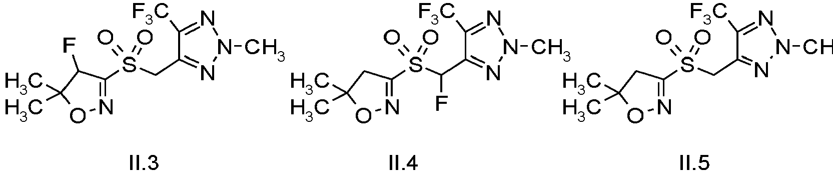 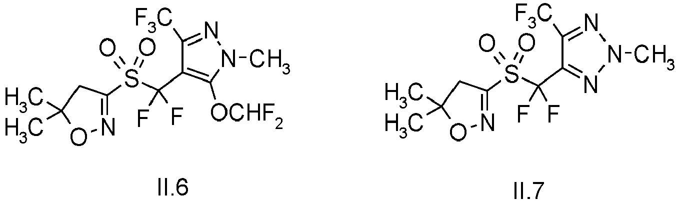 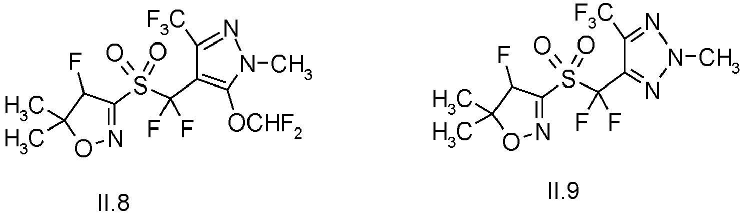 the isoxazoline compounds of the formula (II) are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, flopyrauxifen, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin, methyl azide, methyl bromide, methyl-dymron, methyl iodide, MSMA, oleic acid, oxaziclomefone, pelargonic acid, pyributicarb, quinoclamine tetflupyrolimet, and tridiphane.

Moreover, it may be useful to apply the compounds of formula (I) in combination with safeners. Safeners are chemical compounds which prevent or reduce damage on useful plants without having a major impact on the herbicidal action of the compounds of the formula (I) towards undesired vegetation. They can be applied either before sowings (e.g. on seed treatments, shoots or seedlings) or in the pre-emergence application or post-emergence application of the useful plant. The safeners and the compounds of formula (I) and optionally the herbicides B can be applied simultaneously or in succession.

In another embodiment of the present invention the combinations according to the present invention comprise at least one compound of formula (I) and at least one safener C (component C).

Examples of safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of safener compounds C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4), metcamifen and BPCMS (CAS 54091-06-4).

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

The invention also relates to formulations comprising at least an auxiliary and at least one compound of formula (I) according to the invention.

A formulation comprises a pesticidally effective amount of a compound of formula (I). The term "effective amount" denotes an amount of the combination or of the compound of formula (I), which is sufficient for controlling undesired vegetation, especially for controlling undesired vegetation in crops (i.e. cultivated plants) and which does not result in a substantial damage to the treated crop plants. Such an amount can vary in a broad range and is dependent on various factors, such as the undesired vegetation to be controlled, the treated crop plants or material, the climatic conditions and the specific compound of formula (I) used.

The compounds of formula (I), their salts, amides, esters or thioesters can be converted into customary types of formulations, e. g. solutions, emulsions, suspensions, dusts, powders, pastes, granules, pressings, capsules, and mixtures thereof. Examples for formulation types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further formulation types are defined in the "Catalogue of pesticide formulation types and international coding system", Technical Monograph No. 2, 6th Ed. May 2008, CropLife International.

The formulations are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetting agents, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, aryl phenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothia-zolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for formulation types and their preparation are:
i) Water-soluble concentrates (SL, LS)
   10-60 wt% of a compound of formula (I) or a combination comprising at least one compound of formula (I) (component A) and at least one further compound selected from the herbicidal compounds B (component B) and safeners C (component C) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound of formula (I) or a combination comprising at least one compound of formula (I) (component A) and at least one further compound selected from the herbicidal compounds B (component B) and safeners C (component C) according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of compound of formula (I) or a combination comprising at least one compound of formula (I) (component A) and at least one further compound selected from the herbicidal compounds B (component B) and safeners C (component C) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of compound of formula (I) or a combination comprising at least one compound of formula (I) (component A) and at least one further compound selected from the herbicidal compounds B (component B) and safeners C (component C) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound of formula (I) or a combination comprising at least one compound of formula (I) (component A) and at least one further compound selected from the herbicidal compounds B (component B) and safeners C (component C)according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type formulation up to 40 wt% binder (e.g. polyvinylalcohol) is added.
vi) Water-dispersible granules and water-soluble granules (WG, SG)
   50-80 wt% of a compound of formula (I) or a combination comprising at least one compound of formula (I) (component A) and at least one further compound selected from the herbicidal compounds B (component B) and safeners C (component C)according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) or a combination comprising at least one compound of formula (I) (component A) and at least one further compound selected from the herbicidal compounds B (component B) and safeners C (component C) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound of formula (I) or a combination comprising at least one compound of formula (I) (component A) and at least one further compound selected from the herbicidal compounds B (component B) and safeners C (component C) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound of formula (I) or a combination comprising at least one compound of formula (I) (component A) and at least one further compound selected from the herbicidal compounds B (component B) and safeners C (component C) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound of formula (I) or a combination comprising at least one compound of formula (I) (component A) and at least one further compound selected from the herbicidal compounds B (component B) and safeners C (component C) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS formulation.
ix) Dustable powders (DP, DS)
   1-10 wt% of a compound of formula (I) or a combination comprising at least one compound of formula (I) (component A) and at least one further compound selected from the herbicidal compounds B (component B) and safeners C (component C) according to the invention are ground finely and mixed intimately with solid carrier (e.g. finely divided kaolin) ad 100 wt%.
x) Granules (GR, FG)
   0.5-30 wt% of a compound of formula (I) or a combination comprising at least one compound of formula (I) (component A) and at least one further compound selected from the herbicidal compounds B (component B) and safeners C (component C) according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a compound of formula (I) or a combination comprising at least one compound of formula (I) (component A) and at least one further compound selected from the herbicidal compounds B (component B) and safeners C (component C) according to the invention are dissolved in organic solvent (e.g. aromatic hydrocarbon) ad 100 wt%.

The formulation types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The formulations and/or combinations generally comprise between 0.01 and 95%, preferably between 0.1 and 90%, and in particular between 0.5 and 75%, by weight of the compounds of formula (I).

The compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The formulations in question give, after two-to-tenfold dilution, active substance concentrations of from 0.01 to 60% by weight, preferably from 0.1 to 40% by weight, in the ready-to-use preparations. (nach unten verschoben)

Methods for applying compounds of formula (I), formulations and /or combinations thereof, on to plant propagation material, especially seeds, include dressing, coating, pelleting, dusting, soaking and in-furrow application methods of the propagation material. Preferably, compounds of formula (I), formulations and /or combinations thereof, respectively, are applied on to the plant propagation material by a method such that germination is not induced, e. g. by seed dressing, pelleting, coating and dusting.

Various types of oils, wetting agents, adjuvants, fertilizer, or micronutrients, and further pesticides (e.g. herbicides, insecticides, fungicides, growth regulators, safeners) may be added to the compounds of formula (I), the formulations and/or the combinations comprising them as premix or, if appropriate not until immediately prior to use (tank mix). These agents can be admixed with the formulations according to the invention in a weight ratio of 1:100 to 100:1, preferably 1:10 to 10:1.

The user applies the compounds of formula (I) according to the invention, the formulations and/or the combinations comprising them usually from a pre-dosage device, a knapsack sprayer, a spray tank, a spray plane, or an irrigation system. Usually, the formulation is made up with water, buffer, and/or further auxiliaries to the desired application concentration and the ready-to-use spray liquor or the formulation according to the invention is thus obtained. Usually, 20 to 2000 liters, preferably 50 to 400 liters, of the ready-to-use spray liquor are applied per hectare of agricultural useful area.

According to one embodiment, either individual components of the formulation according to the invention or partially premixed components, e. g. components comprising compounds of formula (I) and optionally active substances from the groups B and/or C), may be mixed by the user in a spray tank and further auxiliaries and additives may be added, if appropriate.

In a further embodiment, individual components of the formulation according to the invention such as parts of a kit or parts of a binary or ternary mixture may be mixed by the user himself in a spray tank and further auxiliaries may be added, if appropriate.

In a further embodiment, either individual components of the formulation according to the invention or partially premixed components, e. g components comprising compounds of formula (I) and optionally active substances from the groups B and/or C), can be applied jointly (e.g. after tank mix) or consecutively.

The compounds of formula (I), are suitable as herbicides. They are suitable as such, as an appropriate formulation or in combination with at least one further compound selected from the herbicidal active compounds B (component B) and safeners C (component C).

The compounds of formula (I), or the formulations and /or combinations comprising the compounds of formula (I), control undesired vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

The compounds of the invention are useful for controlling for example following weeds: *Abutilon theophrasti* (ABUTH), *Alopercurus myosuroides* (ALOMY), *Amaranthus retroflexus* (AMARE), *Apera spica-venti* (APESV), *Avena fatua* (AVEFA), *Chenopodium album* (CHEAL), *Digitaria sanguinalis* (DIGSA), *Echinocloa crus-galli* (ECHCG), *Polygonum convolvulus* (POLCO), *Setaria faberi* (SETFA), *Setaria viridis* (SETVI), to name just a few representative examples.

The compounds of formula (I), or the formulations and/or the combinations comprising them, are applied to the plants mainly by spraying the leaves. Here, the application can be carried out using, for example, water as carrier by customary spraying techniques using spray liquor amounts of from about 100 to 1000 l/ha (for example from 300 to 400 l/ha). The compounds of formula (I), or the formulations and/or the combinations comprising them, may also be applied by the low-volume or the ultra-low-volume method, or in the form of microgranules.

Application of the compounds of formula (I), or the formulations and/or the combinations comprising them, can be done before, during and/or after, preferably during and/or after, the emergence of the undesired vegetation.

Application of the compounds of formula (I), or the formulations and/or the combinations can be carried out before or during sowing.

The compounds of formula (I), or the formulations and/or the combinations comprising them, can be applied pre-, post-emergence or pre-plant, or together with the seed of a crop plant. It is also possible to apply the compounds of formula (I), or the formulations and/or the combinations comprising them, by applying seed, pretreated with the compounds of formula (I), or the formulations and/or the combinations comprising them, of a crop plant. If the active ingredients are less well tolerated by certain crop plants, application techniques may be used in which the combinations are sprayed, with the aid of the spraying equipment, in such a way that as far as possible they do not come into contact with the leaves of the sensitive crop plants, while the active ingredients reach the leaves of undesired vegetation growing underneath, or the bare soil surface (post-directed, lay-by).

In a further embodiment, the compounds of formula (I), or the formulations and/or the combinations comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula (I), or the formulations and/or the combinations prepared therefrom. Here, the combinations can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the crop plants mentioned above, but also the seed of transgenic plants or plants obtained by customary breeding methods.

When employed in plant protection, the amounts of active substances applied, i.e. the compounds of formula (I), component B and, if appropriate, component C without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the compounds of formula (I), component B and, if appropriate, component C, is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of formula (I) is 0.1 to 1000 g/ha, preferably1 to 750 g/ha, more preferably 5 to 500 g/ha.

The required application rates of herbicidal compounds B are generally in the range of from 0.0005 kg/ha to 2.5 kg/ha and preferably in the range of from 0.005 kg/ha to 2 kg/ha or 0.01 kg/ha to 1.5 kg/h of a.s.

The required application rates of safeners C are generally in the range of from 0.0005 kg/ha to 2.5 kg/ha and preferably in the range of from 0.005 kg/ha to 2 kg/ha or 0.01 kg/ha to 1.5 kg/h of a.s.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of formula (I), component B and, if appropriate, component C are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

In case of combinations according to the present invention it is immaterial whether the compounds of formula (I), and the further component B and/or the component C are formulated and applied jointly or separately.

In the case of separate application, it is of minor importance, in which order the application takes place. It is only necessary, that the compounds of formula (I), and the further component B and/or the component C are applied in a time frame that allows simultaneous action of the active ingredients on the plants, preferably within a time-frame of at most 14 days, in particular at most 7 days.

Depending on the application method in question, the compounds of formula (I), or the formulations and /or combinations comprising them, can additionally be employed in a further number of crop plants for eliminating undesired vegetation. Examples of suitable crops are the following:
*Allium cepa, Ananas comosus, Arachis hypogaea, Asparagus officinalis, Avena sativa, Beta vulgaris spec. altissima*, *Beta vulgaris spec. rapa*, *Brassica napus var. napus*, *Brassica napus var. napobrassica*, *Brassica rapa var. silvestris*, *Brassica oleracea, Brassica nigra, Camellia sinensis, Carthamus tinctorius, Carya illinoinensis*, *Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca*, *Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera* and *Zea mays.*

Preferred crops are *Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa, Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera* and *Zea mays.*

Especially preferred crops are crops of cereals, corn, soybeans, rice, oilseed rape, cotton, potatoes, peanuts or permanent crops.

The compounds of formula (I) according to the invention, or the formulations and /or combinations comprising them, can also be used in crops which have been modified by mutagenesis or genetic engineering in order to provide a new trait to a plant or to modify an already present trait.

The term "crops" as used herein includes also (crop) plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or mega-nucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clearfield^{®}. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hydroxyphenylpyruvate dioxygenase (HPPD) inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are for example, but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are for example, but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTOH2, W62, W98, FG72 and CV127.

Transgenic cotton events comprising herbicide tolerance genes are for example, but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are for example, but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are for example, but not excluding others, MON87701, MON87751 and DAS-81419.

Transgenic cotton events comprising genes for insecticidal proteins are for example, but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321. Increased yield has been created by increasing ear biomass using the transgene athb17, being present in corn event MON87403, or by enhancing photosynthesis using the transgene bbx32, being present in the soybean event MON87712.

Crops comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase), as well as in patent applications, like EP3028573 and WO2017/011288.

The use of the compounds of formula (I) or formulations or combinations comprising them according to the invention on crops may result in effects which are specific to a crop comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

Furthermore, plants are also covered that contain by the use of recombinant DNA techniques a modified amount of ingredients or new ingredients, specifically to improve raw material production, e.g., potatoes that produce increased amounts of amylopectin (e.g. Amflora^{®} potato, BASF SE, Germany).

Furthermore, it has been found that the compounds of formula (I) according to the invention, or the formulations and /or combinations comprising them, are also suitable for the defoliation and/or desiccation of plant parts of crops such as cotton, potato, oilseed rape, sunflower, soybean or field beans, in particular cotton. In this regard, formulations and /or combinations for the desiccation and/or defoliation of crops, processes for preparing these formulations and /or combinations and methods for desiccating and/or defoliating plants using the compounds of formula (I) have been found.

As desiccants, the compounds of formula (I) are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

Compounds (I) show very good herbicidal activity and also a high selectivity for undesired vegetation, i.e. a low phytotoxicity for crop plants, especially for cereals, corn, soybeans and rice.

### EXAMPLES

### A Synthesis Examples

Chemical bonds, drawn as bars in chemical formulae, indicate the relative stereochemistry on the ring system.

### Abbreviations:

- Me: methyl
- DCM: dichloromethane
- DMF: N,N-dimethylformamide
- EtOAc: ethyl acetate
- PE: petrol ether
- THF: tetrahydrofuran
- BuLi: n-butyl lithium
- TLC: thin-layer chromatography
- TFE: trifluoroethanol
- m-CPBA: meta-chloroperbenzoic acid
- Phl: phenyl iodide
- DMSO: dimethylsulfoxide
- TEA: trimethylamine
- TFA: trifluoroacetic acid
- MeCN: acetonitrile
- AcOH: acetic acid
- NCS: N-chlorosuccinimide
- TIPS: triisopropylsilyl
- TIPSCI: triisopropylsilyl chloride
- iPrMgCl: isopropylmagnesium chloride
- MTBE: methyl-tert-butyl ether
- TBAF: tetrabutylammonium fluoride
- DIEA: diisopropylethylamine
- EtSH: thioethanol
- DABCO: 1,4-diazabicyclo[2.2.2]octane
- Tf₂O: trifluoromethanesulfonic anhydride
- DBU: 1,8-diazabicyclo[5.4.0]undec-7-ene

### Example 1: Synthesis of methyl 3-[2-chloro-5-(pyrrolidine-1-carbothioyloxy)phenyl]-5-methoxy-4H-isoxazole-5-carboxylate (termed example 1 in Table 1 below)

### 1.1 Preparation of methyl 2-methoxyprop-2-enoate 1.2

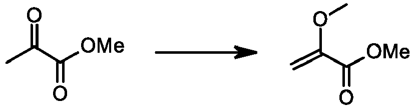

To a solution of methyl 2-oxopropanoate (30 g, 294.12 mmol) and HC(OCH₃)₃ (trimethyl orthoformate; 74.82 g, 705.88 mmol) was added concentrated H₂SO₄ (0.6 mL) dropwise at 0°C and the mixture was stirred at 15°C for 16h. ¹H NMR showed the reaction was completed. The reaction mixture was poured into ice water (200 mL) and extracted with DCM (200 mL x 3). The organic phase was washed with brine, dried over Na₂SO₄ and concentrated to give 44 g of crude product for the next step. To a solution of the crude product (44 g, 294.12 mmol) in DMF (30 mL) was added P₂O₅ (20.88 g, 147.06 mmol) at 15°C and stirred at 100°C for 16h. ¹H NMR showed the reaction was completed. The reaction mixture was poured into ice water (500 mL) and extracted with EtOAc (500 mL x 3). The organic phase was washed with brine, dried over Na₂SO₄, concentrated and purified by column chromatography (PE : EtOAc = 100% - 0%) to give the title compound (14.5 g, yield: 42%) as yellow oil.
¹H NMR (400 MHz, chloroform-d): δ = 5.35 (d, J = 2.6 Hz, 1H), 4.62 (d, J = 2.8 Hz, 1H), 3.81 (s, 3H), 3.65 (s, 3H)

### 1.2 Preparation of 2-chloro-5-methoxy-benzaldehyde

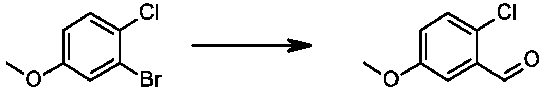

To a solution of 2-bromo-1-chloro-4-methoxy-benzene (10 g, 45.45 mmol) in THF (50 mL) was added n-BuLi (27.2 mL, 68.18 mmol) dropwise at-78°C and the mixture was stirred at -78 °C under N₂ for 30 min. TLC (PE: EtOAc = 5:1) showed the starting maerial was consumed. Then DMF (33.18 g, 454.55 mmol) was added dropwise at -78°C and stirred at -78 °C under N₂ for 1h. LCMS showed the reaction was completed. The reaction mixture was poured into ice 10% HCl solution (200 mL) and extracted with EtOAc (100 mL x 3). The organic phase was washed with brine, dried over Na₂SO₄, concentrated and purified by column chromatography (PE : EtOAc = 100% - 0%) to give the title compound (5.9 g, yield: 76%) as yellow oil.
¹H NMR (400 MHz, chloroform-d): Δ = 10.45 (s, 1H), 7.41 (d, J = 3.1 Hz, 1H), 7.36 (d, J = 8.8 Hz, 1H), 7.10 (dd, J = 3.2, 8.8 Hz, 1H), 3.85 (s, 3H)

### 1.3 Preparation of 2-chloro-5-hydroxy-benzaldehyde

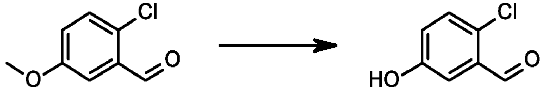

To a solution of 2-chloro-5-methoxy-benzaldehyde (1.7 g, 10 mmol) in DCM (17 mL) was added BBr₃ (4.96 g, 20 mmol) dropwise at 0°C and the mixture was stirred at 0°C for 3h. LCMS showed the reaction was completed. The reaction mixture was poured into water (50 mL) and extracted with DCM (50 mL x 3). The organic phase was washed with brine, dried over Na₂SO₄ and concentrated to give the title compound (1.4 g, yield: crude) as a yellow solid.
¹H NMR (400 MHz, methanol-d₄): δ = 10.42 (s, 1H), 7.42 (d, J = 3.1 Hz, 1H), 7.35 (d, J = 8.8 Hz, 1H), 7.08 (dd, J = 3.1, 8.7 Hz, 1H), 5.54 (br s, 1H)

### 1.4 Preparation of methyl 3-(2-chloro-5-hydroxy-phenyl)-5-methoxy-4H-isoxazole-5-carboxylate

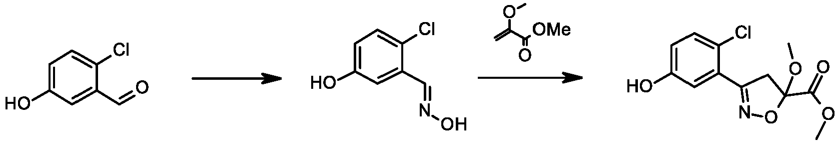

To a solution of 2-chloro-5-hydroxy-benzaldehyde (1.3 g, 8.33 mmol) in TFE (12 mL) was added NH₂OH•HCl (1.93 g, 16.67 mmol) at 15°C and the mixture was stirred at 15°C for 2h. TLC (PE : EtOAc = 3:1) showed that the starting material was consumed. Then m-CPBA (2.36 g, 11.67 mmol) and Phl (340 mg, 1.67 mmol) was added at 15°C and stirred at 15°C for 1h. Then methyl 2-methoxyprop-2-enoate (1.93 g, 16.67 mmol) was added at 15°C and stirred at 15°C for 16h. LCMS showed the reaction was completed. The reaction mixture was poured into NaHCOs solution (50 mL) and extracted with DCM (50 mL × 3). The organic phase was washed with brine, dried over Na₂SO₄, concentrated and purified by column chromatography (PE : EtOAc = 100% - 0%) to give the title compound (1.7 g, yield: 72%) as a yellow oil.
¹H NMR (400 MHz, DMSO-d₆): δ 10.03 (s, 1H), 7.36 (d, J = 8.8 Hz, 1H), 7.01 (d, J = 2.9 Hz, 1H), 6.90 (dd, J = 3.0, 8.8 Hz, 1H), 4.00 (d, J = 18.6 Hz, 1H), 3.80 (s, 3H), 3.61 (d, J = 18.6 Hz, 1H), 3.32 (s, 3H)
LCMS: Calculated MS: 285.0; Observed MS: 286.1

### 1.5 Preparation of methyl 3-[2-chloro-5-(pyrrolidine-1-carbothioyloxy)phenyl]-5-methoxy-4H-isoxazole-5-carboxylate

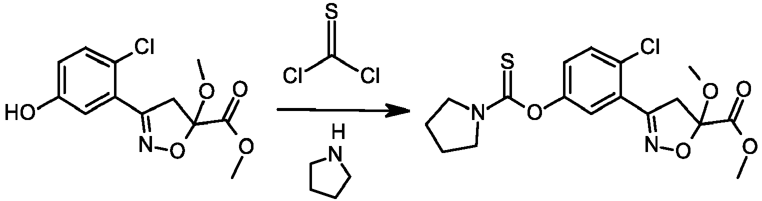

To a solution of thiophosgene (384 mg, 3.37 mmol) in DCM (4 mL) was added methyl 3-(2-chloro-5-hydroxy-phenyl)-5-methoxy-4H-isoxazole-5-carboxylate (800 mg, 2.81 mmol) in DCM (3 mL) dropwise at 0°C and stirred at 0°C for 5 min. Then TEA (1.13 g, 11.23 mmol) in DCM (3 mL) was added dropwise at 0°C and stirred at 0°C for 2 h. Then pyrrolidine (797 mg, 11.23 mmol) in DCM (3 mL) was added at 0°C and stirred at 0°C for 16 h. LCMS showed the reaction was completed. The reaction mixture was poured into ice water (50 mL) and extracted with DCM (50 mL x 3). The organic phase was washed with brine, dried over Na₂SO₄, concentrated and purified by preparative HPLC (TFA, MeCN-H₂O) to give the title compound (170 mg, yield: 15%) as a yellow oil.
¹H NMR (400 MHz, chloroform-d): δ = 7.48 (d, J = 2.8 Hz, 1H), 7.45 (d, J = 8.8 Hz, 1H), 7.17 (dd, J = 2.8, 8.7 Hz, 1H), 4.01 (d, J = 18.3 Hz, 1H), 3.92 - 3.88 (m, 3H), 3.84 - 3.73 (m, 4H), 3.66 (d, J = 18.3 Hz, 1H), 3.48 (s, 3H), 2.15 - 1.97 (m, 5H)
LCMS: Calculated MS: 398.0; Observed MS: 399.1

### Example 2: Synthesis of 3-[2-chloro-4-fluoro-5-(pyrrolidine-1-carbothioyloxy)phenyl]-5-methyl-4H-isoxazole-5-carboxylate (termed example 6 in Table 1 below)

### 2.1 Preparation of 5-bromo-4-chloro-2-fluoro-phenol

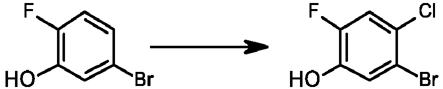

To solution of 5-bromo-2-fluoro-phenol (25 g, 131.6 mmol) in AcOH (150 mL) was added NCS (19.2 g, 144.7 mmol) at 20°C and the mixture was stirred at 60°Cfor 16 h. The reaction was quenched with H₂O (500 mL) and extracted with EtOAc (150 mL x 2). The combined organic phases were washed with brine, dried and concentrated. The crude product was purified by preparative HPLC (TFA, CH₃CN-H₂O) to give the title compound (21 g, 71.2%) as yellow oil.
¹H NMR (400 MHz, CDCl₃): δ = 7.28 (d, J = 8.6 Hz, 1H), 7.21 (d, J = 10.1 Hz, 1H), 6.04 (br d, J = 2.0 Hz, 1H)

### 2.2 Preparation of (5-bromo-4-chloro-2-fluoro-phenoxy)-triisopropyl-silane (TIPS (triisopropylsilyl)-protected 5-bromo-4-chloro-2-fluoro-phenol)

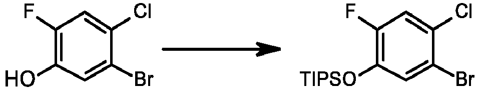

To a solution of 5-bromo-4-chloro-2-fluoro-phenol (13.5 g, 60.3 mol) in DCM (150 mL) was added imidazole (8.2 g, 120.6 mol) and TIPSCI (12.8 g, 66.33 mmol) at 20°C and the mixture was stirred at 20°Cfor 3 h. The reaction was quenched with H₂O (300 mL) and extracted with DCM (200 mL × 2). The combined organic phases were washed with brine, dried and concentrated. The crude product was purified by MPLC (EtOAc/PE = 0% to 10%) to give the title compound (22 g, 96%) as yellow oil.
¹H NMR (400 MHz, CDCl₃): δ = 7.20 (d, J = 0.9 Hz, 1H), 7.18 (s, 1H), 1.30 - 1.23 (m, 3H), 1.12 - 1.08 (m, 18H)

### 2.3 Preparation of 2-chloro-4-fluoro-5-triisopropylsilyloxy-benzaldehyde

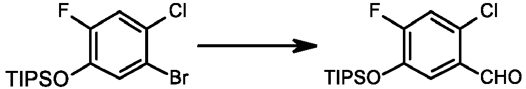

To a solution of (5-bromo-4-chloro-2-fluoro-phenoxy)-triisopropyl-silane (11.2 g, 29.5 mmol) (no solvent) was added i-PrMgCl•LiCl (45 mL, 59 mmol, 1.3M) dropwise at 0°C and stirred at 0°C for 4 h. The mixture was then cooled to -78 °C, DMF (21.5 g, 295 mmol) was added quickly and the mixture was stirred at -78~0°C for 4 h. The reaction was quenched with H₂O (100 mL) and extracted with MTBE (100 mL x 2). The combined organic phases were washed with brine, dried and concentrated to give the title compound (9.7 g, crude) as yellow oil.
¹H NMR (400 MHz, CDCl₃): δ = 10.34 (s, 1H), 7.50 (d, J = 9.0 Hz, 1H), 7.18 (d, J = 9.8 Hz, 1H), 1.33 - 1.25 (m, 3H), 1.11 (d, J = 7.3 Hz, 18H)

### 2.4 Preparation of 2-chloro-4-fluoro-5-triisopropylsilyloxy-benzaldehyde oxime

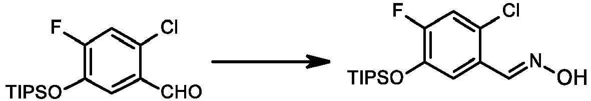

To a solution of 2-chloro-4-fluoro-5-triisopropylsilyloxy-benzaldehyde (12.3 g, 37.3 mmol) in THF (150 mL) was added a solution of NH₂OH•HCl (3.9 g, 55.9 mmol) and NaOH (1.94 g, 48.49 mmol) in water (30 mL) dropwise at 20°C and the mixture was stirred at 20°C for 16 h. The reaction was quenched with H₂O (200 mL) and extracted with EtOAc (200 mL x 2). The combined organic phases were washed with brine, dried and concentrated to give the title compound (13 g, crude) as yellow oil.

### 2.5 Preparation of methyl 3-(2-chloro-4-fluoro-5-triisopropylsilyloxy-phenyl)-5-methyl-4H-isoxazole-5-carboxylate

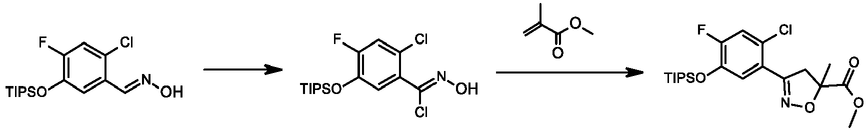

To a solution of 2-chloro-4-fluoro-5-triisopropylsilyloxy-benzaldehyde oxime (13 g, 37.7 mmol) in DMF (130 mL) was added NCS (10 g, 75.4 mmol) and the mixture was stirred at 20°C for 1 h. TLC showed 2-chloro-4-fluoro-5-triisopropylsilyloxy-benzaldehyde oxime was consumed. Then methyl 2-methylprop-2-enoate (113 g, 1131 mmol) was added at 20°C. Subsequently TEA (19 g, 188.5 mmol) in DMF (20 mL) was added dropwise at 0°C and the mixture was stirred at 0-20°C for 2 h. The reaction was poured into ice water (300 mL) and extracted with MTBE (300 mL x 2). The combined organic phases were washed with brine, dried and concentrated. The crude product was purified by column chromatography (EtOAc/PE = 0 % to 10%) to give the title compound (10.5 g, 62.9 %) as yellow oil.
¹H NMR (400 MHz, CDCl₃): δ = 7.33 (d, J = 9.0 Hz, 1H), 7.13 (d, J = 10.1 Hz, 1H), 3.98 (d, J = 17.5 Hz, 1H), 3.83 (s, 3H), 3.41 (d, J = 17.5 Hz, 1H), 1.73 (s, 3H), 1.32 - 1.24 (m, 3H), 1.10 (d, J = 7.2 Hz, 18H)

### 2.6 Preparation of methyl 3-(2-chloro-4-fluoro-5-hydroxy-phenyl)-5-methyl-4H-isoxazole-5-carboxylate

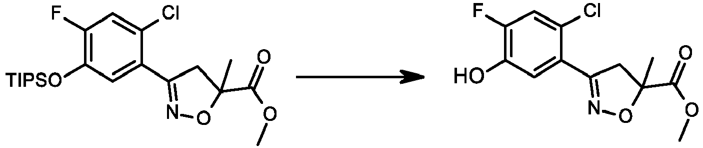

To a solution of methyl 3-(2-chloro-4-fluoro-5-triisopropylsilyloxy-phenyl)-5-methyl-4H-isoxazole-5-carboxylate (5 g, 11.3 mmol) in THF (50 mL) was added TBAF (22.6 mL, 1 M in THF, 22.6 mmol) dropwise at 0°C and the mixture was stirred at 0-20°C for 4 h. The reaction was poured into ice water (100 mL) and extracted with DCM (50 mL x 2). The combined organic phases were washed with brine, dried and concentrated. The crude product was purified by MPLC (EtOAc/PE= 0% to 30%) to give the title compound (3 g, 93.8 %) as yellow solid.
¹H NMR (400 MHz, CDCl₃): δ = 7.39 (d, J = 9.1 Hz, 1H), 7.16 (d, J = 10.0 Hz, 1H), 6.35 - 5.66 (m, 1H), 3.96 (d, J = 17.5 Hz, 1H), 3.83 (s, 3H), 3.43 (d, J = 17.4 Hz, 1H), 1.74 (s, 3H)
LCMS: Calculated MS: 287.1; Observed MS: 288.1

### 2.7 Preparation of methyl 3-[2-chloro-4-fluoro-5-(pyrrolidine-1-carbothioyloxy)phenyl]-5-methyl-4H-isoxazole-5-carboxylate

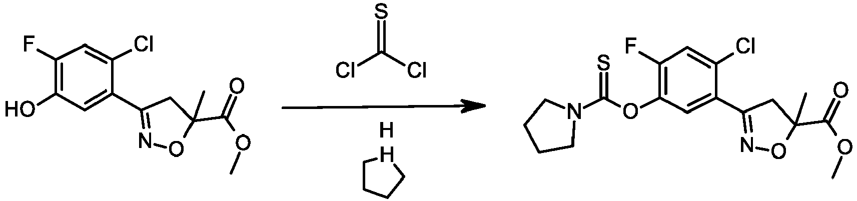

To a solution of thiophosgene (192 mg, 1.68 mmol) in DCM (1 mL) was added methyl 3-(2-chloro-4-fluoro-5-hydroxy-phenyl)-5-methyl-4H-isoxazole-5-carboxylate (0.4 g, 1.4 mmol) in DCM (1 mL) dropwise at 0°C and the mixture was stirred at 0°Cfor 5 min. Then DIEA (722 mg, 5.6 mmol) in DCM (1 mL) was added dropwise at 0°C and the mixture was stirred at 0°C for 2 h. Subsequently pyrrolidine (398 mg, 5.6 mmol) in DCM (1 mL) was added dropwise at 0°C and the mixture was stirred at 0-20°C for 16 h. The reaction was poured into ice water (10 mL) and extracted with DCM (10 mL × 2). The combined organic phases were washed with brine, dried and concentrated. The crude product was purified by preparative HPLC (TFA, CH₃CN-H₂O) to give the title compound (550 mg, 97.7 %) as yellow oil.
¹H NMR (400 MHz, CDCl₃): δ = 7.54 (d, J = 8.0 Hz, 1H), 7.28 - 7.25 (m, 1H), 4.01 (d, J = 17.4 Hz, 1H), 3.83 (s, 3H), 3.82 - 3.75 (m, 4H), 3.42 (d, J = 17.4 Hz, 1H), 2.13 - 1.99 (m, 4H), 1.72 (s, 3H)
LCMS: Calculated: 400; Observed MS: 401.2

### Example 3: Synthesis of methyl 3-[2-chloro-5-(3,3-difluoropyrrolidine-1-carbothioyl)oxy-4-fluoro-phenyl]-5-methyl-4H-isoxazole-5-carboxylate (termed example 7 in Table 1 below)

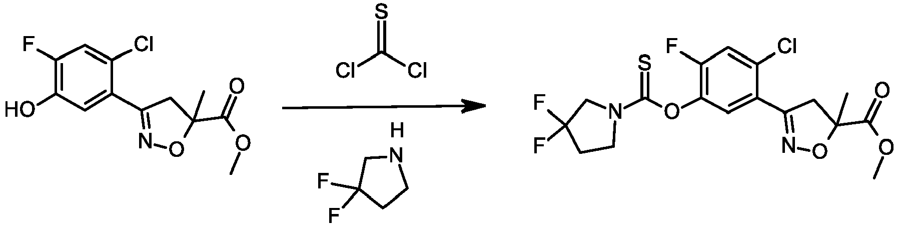

To a solution of thiophosgene (575 mg, 5.0 mmol) in THF (5 mL) was added methyl 3-(2-chloro-4-fluoro-5-hydroxy-phenyl)-5-methyl-4H-isoxazole-5-carboxylate (prepared as in example 2, steps 2.1 to 2.6; 1.196 g, 4.17 mmol) in THF (10 mL) dropwise at 0°C under N₂. The mixture was stirred at 0°C for 20 min. Then to the mixture was added 3,3-difluoropyrrolidine (2.384 mg, 16.67 mmol) and DIEA (4.3 g, 33.34 mmol) in THF (4 mL) dropwise at 0°C. The mixture was stirred at 20°C for 3 h. TLC (PE: EtOAc =3:1) showed the reaction was complete. The mixture was poured into H₂O (20 mL) and extracted with EtOAc (20 mL x 3). The organic phase was washed with brine, dried over Na₂SO₄, filtered and concentrated. The residue was purified by preparative HPLC (TFA-MeCN-H₂O) to give the title compound (914 mg, 50.27%) as yellow solid.
¹H NMR (400 MHz, CDCl₃): δ = 7.55 (dd, J = 2.0, 8.0 Hz, 1H), 7.30 - 7.26 (m, 1H), 4.19 - 4.06 (m, 2H), 4.05 - 3.97 (m, 3H), 3.83 (s, 3H), 3.42 (d, J = 17.4 Hz, 1H), 2.66 - 2.42 (m, 2H), 1.73 (s, 3H)
LCMS: Calculated MS: 436.0; Observed MS: 437.1

### Example 4: Synthesis of methyl 3-[2-chloro-4-fluoro-5-(pyrrolidine-1-carbothioyloxy)phenyl]-3a,4,5,6-tetrahydrocyclopenta[d]isoxazole-6a-carboxylate (termed example 15 in Table 1 below)

### 4.1 Preparation of 1-bromo-2-chloro-4-fluoro-5-methoxy-benzene

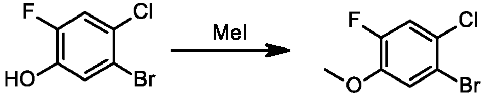

To a solution of 1-bromo-2-chloro-4-fluoro-5-hydroxy-benzene (20 g, 89.2 mmol) in DMF (200 mL) was added K₂CO₃ (25.6 g, 178.4 mmol) and Mel (25.4 g, 178.4 mmol. The reaction was stirred at 20°C for 16 h. The reaction solution was poured into ice water (300 mL), and extracted with MTBE (100 mL x 2). The organic phases were washed with brine (100 mL), dried over Na₂SO₄, filtered and concentrated. The crude product was purified by column chromatography (PE:EtOAc= 100%-20%) to give title compound (16.2 g, 75.4 %) as white solid.
¹H NMR (400 MHz, CDCl₃): δ = 7.24 - 7.15 (m, 2H), 3.88 (s, 3H)

### 4.2 Preparation of 2-chloro-4-fluoro-5-methoxy-benzaldehyde

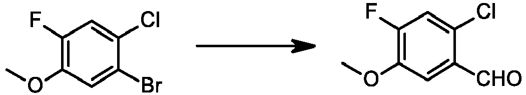

To a solution of 1-bromo-2-chloro-4-fluoro-5-methoxy-benzene (15 g, 63.0 mmol) in THF (220 mL) was added iPrMgCl•LiCl (97 mL, 1.3 M, 126.1 mmol) dropwise at -78°C. The reaction was stirred at -78°C for 2 h. DMF (46.0 g, 630.3 mmol) was added to the mixture dropwise at -78°C. The reaction was stirred at -78-20°C for 2 h. The reaction solution was poured into ice water (300 mL), and extracted with MTBE (100 mL × 2). The organic phases were washed with brine (100 mL), dried over Na₂SO₄, filtered and concentrated. The crude product was purified by column chromatography (PE:EtOAc= 100%-20%) to give the title compound (10 g, 84.4%) as yellow oil.
¹H NMR (400 MHz, CDCl₃): δ = 10.37 (s, 1H), 7.52 (d, J = 9.1 Hz, 1H), 7.20 (d, J = 10.3 Hz, 1H), 3.94 (s, 3H)

### 4.3 Preparation of 2-chloro-4-fluoro-5-methoxy-benzaldehyde oxime

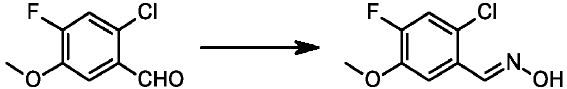

To a solution of 2-chloro-4-fluoro-5-methoxy-benzaldehyde (10 g, 53.2 mmol) in THF (150 mL) and H₂O (90 mL) was added NH₂OH•HCl (11.0 g, 159.6 mmol) and NaOH (5.5 g, 138.3 mmol) in H₂O (90 mL) at 20°C. The reaction was stirred at 20°C for 2 h. The reaction solution was poured into ice water (200 mL), and extracted with EtOAc (150 mL x 2). The organic phases were washed with brine (150 mL), dried over Na₂SO₄, filtered and concentrated to give the title compound (10.3 g, 95.4%) as yellow oil.
¹H NMR (400 MHz, CDCl₃): δ = 8.49 (s, 1H), 7.45 (d, J = 8.9 Hz, 1H), 7.14 (d, J = 10.5 Hz, 1H), 3.91 (s, 3H)

### 4.4 Preparation of 2-chloro-4-fluoro-N-hydroxy-5-methoxy-benzimidoyl chloride

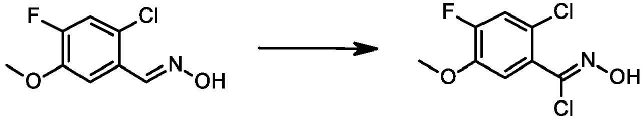

To a solution of 2-chloro-4-fluoro-5-methoxy-benzaldehyde oxime (10.3 g, 50.7 mmol) in DMF (100 mL) was added NCS (13.5 g, 101.5 mmol) at 0°C. The reaction was stirred at 20°C for 30 min. The reaction solution was poured into ice water (300 mL), and extracted with MTBE (100 mL x 2). The organic phases were washed with brine (150 mL), dried over Na₂SO₄, filtered and concentrated. The crude product was purifited by column chromatography (PE:EtOAc= 100%-20%) to give the title compound (11.9 g, crude) as yellow solid.
¹H NMR (400 MHz, CDCl₃): δ = 8.22 (s, 1H), 7.22 - 7.17 (m, 1H), 7.06 (d, J = 8.6 Hz, 1H), 3.94 - 3.85 (m, 3H)

### 4.5 Preparation of methyl 3-(2-chloro-4-fluoro-5-methoxy-phenyl)-3a,4,5,6-tetrahydrocyclopenta[d]isoxazole-6a-carboxylate

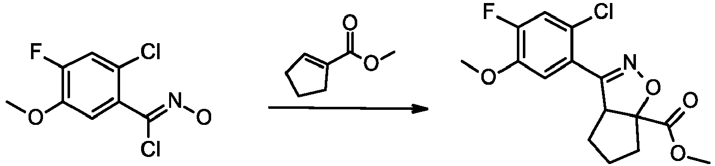

To a solution of 2-chloro-4-fluoro-N-hydroxy-5-methoxy-benzimidoyl chloride (11.9 g, 50.2 mmol) and methyl cyclopentene-1-carboxylate (19.0 g, 150.6 mmol) in toluene (120 mL) was added and TEA (5.1 g, 50.2 mmol) at 20°C. The reaction was stirred at 20°C for 2 h. The reaction solution was poured into ice water (300 mL), and extracted with EtOAc (150 mL × 2). The organic phases were washed with brine (150 mL), dried over Na₂SO₄, filtered and concentrated. The crude product was purified by column chromatography (PE:EtOAc= 100%-20%) to give the title compound (10.9 g, 65.6%) as yellow solid.
¹H NMR (400 MHz, CDCl₃):
δ = 7.27 (s, 1H), 7.16 (d, J = 10.5 Hz, 1H), 4.67 (dd, J = 1.3, 9.0 Hz, 1H), 3.90 (s, 3H), 3.85 (s, 3H), 2.35 (dd, J = 6.3, 13.8 Hz, 1H), 2.19 (ddd, J = 6.4, 12.5, 13.7 Hz, 1H), 2.01 - 1.78 (m, 3H), 1.74 - 1.60 (m, 1H)

### 4.6 Preparation of methyl 3-(2-chloro-4-fluoro-5-hydroxy-phenyl)-3a,4,5,6-tetrahydrocyclopenta[d]isoxazole-6a-carboxylate

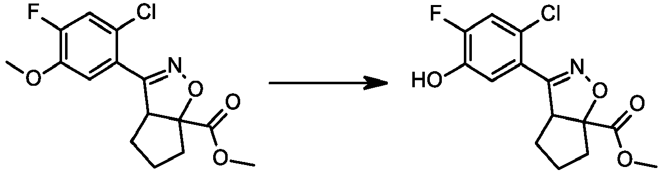

To a solution of methyl 3-(2-chloro-4-fluoro-5-methoxy-phenyl)-3a,4,5,6-tetrahydrocyclopenta[d]isoxazole-6a-carboxylate (1 g, 3.1 mmol) in DCM (50 mL) was added AlCl₃ (2.0 g, 15.3 mmol) at 0°C. The reaction was stirred at 0°C for 2 min. EtSH (951 mg, 15.3 mmol) in DCM (2 mL) was added to the mixture dropwise at 0°C, and the mixture was stirred at 0°C for 5 min. The reaction solution was poured into ice water (100 mL), and extracted with DCM (50 mL x 2). The organic phases were washed with brine (50 mL), dried over Na₂SO₄, filtered and concentrated. The crude product was purified by column chromatography (PE:EtOAc= 100%-20%) to give the title compound (305 mg, 31.4%) as white solid.
¹H NMR (400 MHz, CDCl₃): δ = 7.25 (s, 1H), 7.20 (d, J = 9.9 Hz, 1H), 4.58 (d, J = 9.0 Hz, 1H), 3.85 (s, 3H), 2.36 (dd, J = 6.1, 13.6 Hz, 1H), 2.18 (dt, J = 6.3, 13.1 Hz, 1H), 2.00 - 1.76 (m, 4H), 1.67 (br dd, J = 6.3, 12.4 Hz, 1H)

### 4.7 Preparation of methyl 3-[2-chloro-4-fluoro-5-(pyrrolidine-1-carbothioyloxy)phenyl]-3a,4,5,6-tetrahydrocyclopenta[d]isoxazole-6a-carboxylate

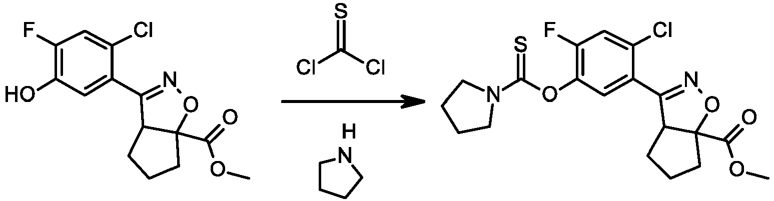

To a solution of CSCl₂ (thiophosgene; 192 mg, 1.67 mmol) in DCM (3.5 mL) was added methyl 3-(2-chloro-4-fluoro-5-hydroxy-phenyl)-3a,4,5,6-tetrahydrocyclopenta[d]isoxazole-6a-carboxylate (435 mg, 1.39 mmol) in DCM (3.5 mL) dropwise at 0°C. The mixture was stirred at 0°C for 5 min. DIEA (717 mg, 5.56 mmol) in DCM (3.5 mL) was added to the mixture dropwise at 0°C, and the mixture stirred at 0°C for 2 h. Pyrrolidine (395 mg, 5.56 mmol) in DCM (3.5 mL) was added to the mixture dropwise at 0°C. The mixture was stirred at 0°C for 1 h. The reaction solution was poured into H₂O (20 mL), and extracted with DCM (20 mL × 2). The organic phases were washed with brine (50 mL), dried over Na₂SO₄, filtered and concentrated. The crude product was purified by preparative HPLC (TFA; MeCN-H₂O) to give the title compound (290 mg, 49.0%) as yellow oil.
¹H NMR (400 MHz, CDCl₃): δ = 7.41 (d, J = 8.0 Hz, 1H), 7.29 (s, 1H), 4.57 (br d, J = 8.8 Hz, 1H), 3.85 (s, 3H), 3.82 - 3.76 (m, 4H), 2.39 - 2.33 (m, 1H), 2.18 (td, J = 6.7, 13.1 Hz, 1H), 2.11 - 2.01 (m, 4H), 2.00 - 1.93 (m, 1H), 1.88 (td, J = 6.5, 12.7 Hz, 2H), 1.66 (dt, J = 6.1, 12.5 Hz, 1H)
LCMS: Calculated MS: 426.1; Observed MS: 427.1

### Example 5: Synthesis of methyl 3-[2-chloro-5-(3,3-difluoropyrrolidine-1-carbothioyl)oxy-4-fluoro-phenyl]-5-vinyl-4H-isoxazole-5-carboxylate (termed example 23 in Table 1 below)

### 5.1 Preparation of methyl 3-hydroxy-2-methylene-butanoate

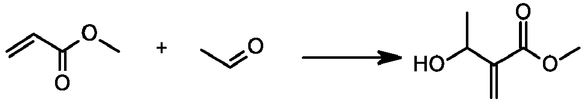

To a mixture of methyl prop-2-enoate (150 g, 1.36 mol) and DABCO (152.55 g, 1.36 mol) in dioxane/H₂O (300/300 mL) was added acetaldehyde (351.74 g, 4.09 mmol) dropwise and stirred at 20°C for 16 h. TLC (PE: EtOAc=5:1) showed the reaction was completed. The reaction mixture was poured into water (1000 mL) and DCM with EtOAc (500 mL x 2). The organic phase was washed with brine, dried over Na₂SO₄, concentrated and purified by column chromatography (PE: EtOAc=100%-0%) to give the title compound (70 g, 39.5%) as a yellow oil.

### 5.2 Preparation of methyl 3-(2-chloro-4-fluoro-5-triisopropylsilyloxy-phenyl)-5-(1-hydroxyethyl)-4H-isoxazole-5-carboxylate

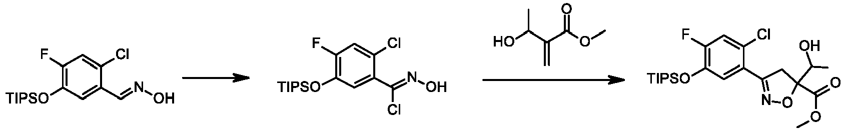

To a solution of 2-chloro-4-fluoro-5-triisopropylsilyloxy-benzaldehyde oxime (10 g, 28.98 mmol) in DMF (130 mL) was added NCS (8.52 g, 57.97 mmol) and the mixture was stirred for 30 min. Then methyl 3-hydroxy-2-methylene-butanoate (4.5 g, 34.76 mmol) and TEA (20 mL, 144.9 mmol) in DMF (20 mL) were added dropwise to the reaction mixture and the mixture was stirred at 20°C for 1 h. LCMS showed the reaction was completed. The reaction mixture was poured into water (350 mL) and extracted with EtOAc (100 mL x 3). The organic phase was washed with brine, dried over Na₂SO₄, concentrated and purified by column chromatography (PE: EtOAc=100%-0%) to give the title compound (8 g, 58.36%) as a yellow oil.
¹H NMR (400 MHz, CDCl₃): δ = 7.35 - 7.29 (m, 1H), 7.18 - 7.12 (m, 1H), 4.32 (br d, J = 6.4 Hz, 1H), 3.92 - 3.84 (m, 4H), 3.79 - 3.71 (m, 1H), 1.35 - 1.18 (m, 6H), 1.10 (d, J = 7.3 Hz, 18H)

### 5.3 Preparation of methyl 3-(2-chloro-4-fluoro-5-triisopropylsilyloxy-phenyl)-5-[1-(trifluoromethylsulfonyloxy)ethyl]-4H-isoxazole-5-carboxylate

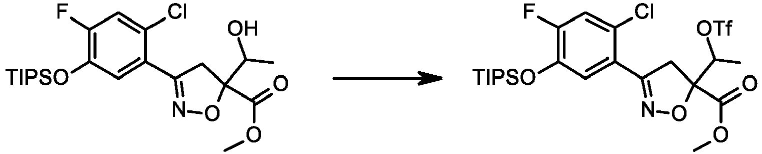

To a solution of methyl 3-(2-chloro-4-fluoro-5-triisopropylsilyloxy-phenyl)-5-(1-hydroxyethyl)-4H-isoxazole-5-carboxylate (4 g, 8.45 mmol) and pyridine (2.67 g, 33.82 mmol) in DCM (40 mL) was added TfzO (4.765 g, 16.9 mmol) dropwise at -78°C and the mixture was stirred at 20°C for 1 h. LCMS showed the reaction was completed. The reaction mixture was poured into 2N HCl (100 mL) and extracted with EtOAc (100 mL x 3). The organic phase was washed with brine, dried over Na₂SO₄, concentrated and purified by column chromatography (PE: EtOAc=100%-0%) to give the title compound (4 g, 78.2%) as a yellow oil.
¹H NMR (400 MHz, CDCl₃): δ = 7.32 - 7.27 (m, 1H), 7.19 - 7.13 (m, 1H), 5.57 - 5.43 (m, 1H), 4.18 (d, J = 17.8 Hz, 1H), 3.95 - 3.86 (m, 3H), 3.59 (d, J = 17.9 Hz, 1H), 1.54 (d, J = 6.6 Hz, 3H), 1.35 - 1.21 (m, 3H), 1.10 (d, J = 7.3 Hz, 17H)

### 5.4 Preparation of methyl 3-(2-chloro-4-fluoro-5-hydroxy-phenyl)-5-vinyl-4H-isoxazole-5-carboxylate

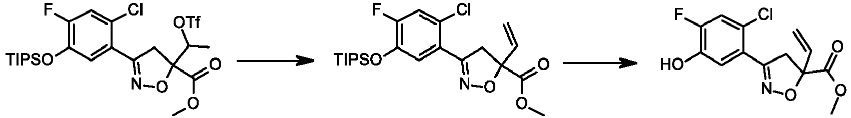

To a solution of methyl 3-(2-chloro-4-fluoro-5-triisopropylsilyloxy-phenyl)-5-[1-(trifluoromethylsulfonyloxy)ethyl]-4H-isoxazole-5-carboxylate (4 g, 6.61 mmol) in MeCN (40 mL) was added DBU (2 g, 13.22 mmol) and stirred at 20°C for 16 h. Then to the reaction was added TBAF (7.9 mL, 7.932 mmol) and the mixture was stirred at 20°C for 4 h. LCMS showed the reaction was completed. The reaction mixture was poured into 2N HCl (100 mL) and extracted with EtOAc (100 mL x 3). The organic phase was washed with brine, dried over Na₂SO₄, concentrated and purified by column chromatography (PE: EtOAc=100%-0%) to give the title compound (2.5 g, crude) as a yellow oil.
¹H NMR (400 MHz, CDCl₃): δ = 7.38 (d, J = 9.1 Hz, 1H), 7.16 (d, J = 10.0 Hz, 1H), 6.14 (dd, J = 10.7, 17.2 Hz, 1H), 5.93 (br s, 1H), 5.56 (d, J = 17.3 Hz, 1H), 5.39 (d, J = 10.8 Hz, 1H), 4.06 (d, J = 17.4 Hz, 1H), 3.89 - 3.82 (m, 3H), 3.58 (d, J = 17.5 Hz, 1H)

### 5.5 Preparation of (3,3-difluoropyrrolidine-1-carbothioyl)sulfanyl 3,3-difluoropyrrolidine-1-carbodithioate

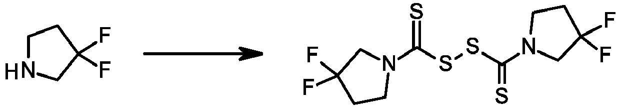

To a solution of NaOH (2.8 g, 69.93 mmol) in H₂O (100 mL) was added 3,3-difluoropyrrolidine (10 g, 69.93 mmol) at 0°C and the mixture was stirred at 20°C for 5 min. Then CS₂ (7.97 g, 104.90 mmol) was added dropwise at 35°C and the mixture was stirred at 35°C for 3 h. Subsequently H₂O₂ (30%, 4.76 g, 41.96 mmol) was added dropwise at 55°C and the mixture was stirred at 55°C for 1 h. TLC (EtOAc) showed that the starting material was consumed, LCMS showed major desired MS. The reaction mixture was filtered and the cake was collected to give the title compound (7 g, yield: 55%) as white solid.
¹H NMR (400 MHz, CDCl₃): δ = 4.40 - 4.15 (m, 4H), 2.72 - 2.46 (m, 2H)

### 5.6 Preparation of 3,3-difluoropyrrolidine-1-carbothioyl chloride

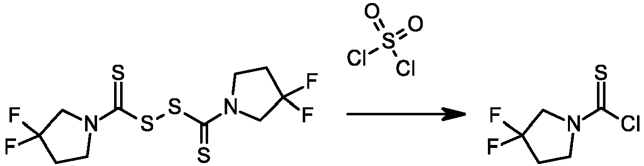

To a solution of (3,3-difluoropyrrolidine-1-carbothioyl)sulfanyl 3,3-difluoropyrrolidine-1-carbodithioate (3 g, 8.2 mmol) in CCl₄ (10 mL) was added sulfuryl chloride (1.1 g, 8.2 mmol) in CCl₄ (5 mL) dropwise at 20°C and the mixture was stirred at 20°C for 10 min, followed by heating to 80°C under stirring for 1 h. LCMS showed the reaction was completed. The reaction mixture was filtered and the filtrate was concentrated to give the title compound (3.5 g, yield: crude) as yellow solid.
¹H NMR (400 MHz, CDCl₃): δ = 4.23 - 4.02 (m, 4H), 2.65 - 2.48 (m, 2H)

### 5.7 Preparation of methyl 3-[2-chloro-5-(3,3-difluoropyrrolidine-1-carbothioyl)oxy-4-fluoro-phenyl]-5-vinyl-4H-isoxazole-5-carboxylate

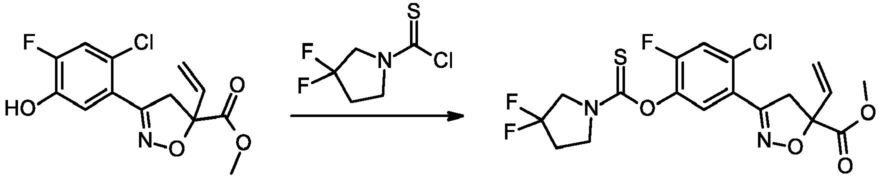

To a solution of methyl 3-(2-chloro-4-fluoro-5-hydroxy-phenyl)-5-vinyl-4H-isoxazole-5-carboxylate (500 mg, 1.67 mmol) and DABCO (374 mg, 3.34 mmol) in THF (2 mL) was added 3,3-difluoropyrrolidine-1-carbothioyl chloride (495 mg, 2.67 mmol) dropwise at 0°C and the mixture was stirred at 20°C for 16 h. LCMS showed the reaction was completed. The reaction mixture was poured into H₂O (50 mL) and extracted with EtOAc (50 mL x 3). The organic phase was washed with brine, dried over Na₂SO₄, concentrated and purified by preparative HPLC (TFA-MeCN-H₂O) to give the title compound (517 mg, 69.1%) as a yellow oil.
¹H NMR (400 MHz, CDCl₃): δ = 7.55 (dd, J = 2.0, 8.0 Hz, 1H), 7.29 (s, 1H), 6.13 (dd, J = 10.7, 17.2 Hz, 1H), 5.56 (d, J = 17.3 Hz, 1H), 5.39 (d, J = 10.6 Hz, 1H), 4.18 - 3.99 (m, 6H), 3.85 (s, 3H), 3.58 (d, J = 17.5 Hz, 1H), 2.63 - 2.47 (m, 2H)
LCMS: Calculated MS: 448.0; Observed MS: 449.1

In analogy to the examples described above, the following compounds of formula (I) listed in Table 1 were prepared.

The compound of example 27 is the sodium salt of the compound of example 13, and was prepared by reacting the latter with an equimolar amount of NaOH in water/THF and evaporating the solvents.

The chiral compounds were obtained by resolving the corresponding racemate via supercritical fluid chromatography (SFC).

**Table 1**

| 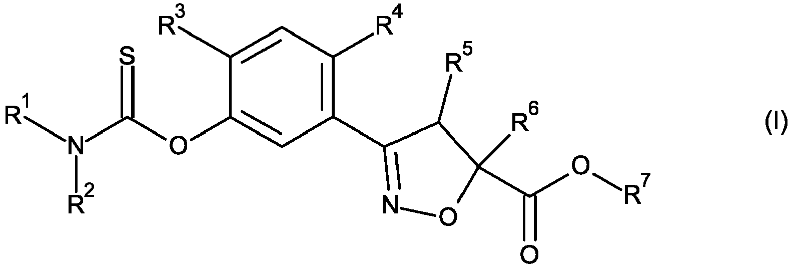 | | | | | | | | | |
|---|---|---|---|---|---|---|---|---|---|
| **Ex. No** | **Stereochemistry** | **R³** | **R⁴** | **R¹** | **R²** | **R⁵** | **R⁶** | **R⁷** | **LCMS MZ** |
| 1 | Racemate | H | Cl | -CH₂CH₂CH₂CH₂- | | H | OMe | Me | 399.1 |
| 2 | Racemate | H | Cl | -CH₂CH₂CH₂CH₂- | | H | Me | H | 369.0 |
| 3 | Racemate | H | Cl | -CH₂CH₂CH₂CH₂- | | H | Me | Me | 383.0 |
| 4 | Racemate | H | Cl | Et | Et | H | Me | Me | 385.2 |
| 5 | Racemate | H | Cl | -CH₂CF₂CH₂CH₂- | | H | OMe | Me | 435.0 |
| 6 | Racemate | F | Cl | -CH₂CH₂CH₂CH₂- | | H | Me | Me | 401.2 |
| 7 | Racemate | F | Cl | -CH₂CF₂CH₂CH₂- | | H | Me | Me | 437.1 |
| 8 | Racemate | F | Cl | -CH₂CH(CH₃)CH₂CH₂- | | H | Me | Me | 415.2 |
| 9 | Racemate | H | Cl | -CH₂CH(CH₃)CH₂CH₂- | | H | OMe | Me | 413.1 |
| 10 | Racemate | F | Cl | -CH₂CHFCH₂CH₂- | | H | Me | Me | 418.7 |
| 11 | Chiral - (*R*) | F | Cl | -CH₂CH₂CH₂CH₂- | | H | Me | Me | 401.0 |
| 12 | Chiral - (*S*) | F | Cl | -CH₂CH₂CH₂CH₂- | | H | Me | Me | 401.0 |
| 13 | Racemate | F | Cl | -CH₂CH₂CH₂CH₂- | | H | Me | H | 387.0 |
| 14 | Racemate | F | Cl | -CH₂CF₂CH₂CH₂- | | H | Me | H | 422.9 |
| 15 | Racemate | F | Cl | -CH₂CH₂CH₂CH₂- | | -CH₂CH₂CH₂- | | Me | 427.1 |
| 16 | Racemate | F | Cl | -CH₂CF₂CH₂CH₂- | | -CH₂CH₂CH₂- | | Me | 463.0 |
| 17 | Racemate | F | Cl | -CH₂CH₂CH₂CH₂- | | H | Me | Et | 415.0 |
| 18 | Chiral - (*S*) | F | Cl | -CH₂CF₂CH₂CH₂- | | H | Me | Et | 450.9 |
| 19 | Chiral - (*R*) | F | Cl | -CH₂CF₂CH₂CH₂- | | H | Me | Et | 450.9 |
| 20 | Racemate | F | Cl | -CH₂CF₂CH₂CH₂- | | H | Me | Et | 450.9 |
| 21 | Racemate | F | Cl | -CH₂CH₂CH₂CH₂- | | H | Et | Et | 429.0 |
| 22 | Racemate | F | Cl | -CH₂CH₂CH₂CH₂- | | H | Vinyl | Me | 413.1 |
| 23 | Racemate | F | Cl | -CH₂CF₂CH₂CH₂- | | H | Vinyl | Me | 449.1 |
| 24 | Racemate | F | Cl | -CH₂CF₂CH₂CH₂- | | H | Vinyl | Et | 463.1 |
| 25 | Racemate | F | Cl | -CH₂CF₂CH₂CH₂- | | H | Vinyl | H | 435.1 |
| 26 | Racemate | H | Cl | -CH₂CF₂CH₂CH₂- | | H | Me | Et | 433.1 |
| 27 | Racemate | F | Cl | -CH₂CH₂CH₂CH₂- | | H | Me | Na | 431.9 (M+23) |

| | | | | | | | | | |
|---|---|---|---|---|---|---|---|---|---|
| Me: methyl Et: ethyl OMe: methoxy | | | | | | | | | |

### B Biological examples

### B.1 Herbicidal activity

The herbicidal activity of the compounds of formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the test plants had rooted. This cover caused uniform germination of the test plants, unless this had been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the test plants were kept at 10 -25°C or 20 - 35°C, respectively.

The test period extended over 2 to 4 weeks. During this time, the test plants were tended, and their response to the individual treatments was evaluated.

Evaluation was carried out using a scale from 0 to 100. 100 means no emergence of the test plants, or complete destruction of at least the aerial moieties, and 0 means no damage, or normal course of growth. A good herbicidal activity is given at values of 65 to < 90 and a very good herbicidal activity is given at values of 90 to 100.

The test plants used in the greenhouse experiments were of the following species:

| Bayer code | Scientific name |
|---|---|
| ABUTH | *Abutilon theophrasti* |
| ALOMY | *Alopercurus myosuroides* |
| AMARE | *Amaranthus retroflexus* |
| AVEFA | *Avena fatua* |
| CHEAL | *Chenopodium album* |
| DIGSA | *Digitaria sanguinalis* |
| ECHCG | *Echinocloa crus-galli* |
| POLCO | *Polygonum convolvulus* |
| SETVI | *Setaria viridis* |

At an application rate of 0.031 kg/ha, applied by the pre-emergence method:
- compound 7 showed good herbicidal activity against AMARE

At an application rate of 0.031 kg/ha, applied by the post-emergence method:
- compounds 6, 7, 10 showed very good herbicidal activity against AMARE
- compound 10 showed very good herbicidal activity against CHEAL
- compound 10 showed very good herbicidal activity against POLCO

At an application rate of 0.125 kg/ha, applied by the pre-emergence method:
- compounds 6, 7 showed very good herbicidal activity against AMARE

At an application rate of 0.125 kg/ha, applied by the post-emergence method:
- compounds 2, 3, 5, 6 showed very good herbicidal activity against AMARE
- compound 5 showed very good herbicidal activity against CHEAL
- compound 5 showed very good herbicidal activity against POLCO

At an application rate of 0.25 kg/ha, applied by the pre-emergence method:
- compounds 14, 26 showed very good herbicidal activity against AMARE
- compound 14 showed very good herbicidal activity against DIGSA
- compound 14 showed very good herbicidal activity against SETVI

At an application rate of 0.25 kg/ha, applied by the post-emergence method:
- compounds 14, 26 showed very good herbicidal activity against AMARE
- compound 26 showed good herbicidal activity against AVEFA
- compound 14 showed very good herbicidal activity against AVEFA
- compounds 14, 26 showed very good herbicidal activity against ECHCG

At an application rate of 0.5 kg/ha, applied by the pre-emergence method:
- compound 8 showed very good herbicidal activity against AMARE

At an application rate of 0.5 kg/ha, applied by the post-emergence method:
- compound 8 showed good herbicidal activity against ALOMY
- compounds 4, 8 showed very good herbicidal activity against AMARE
- compound 4 showed good herbicidal activity against ECHCG
- compound 8 showed very good herbicidal activity against ECHCG

At an application rate of 1 kg/ha, applied by the pre-emergence method:
- compounds 1, 9 showed good herbicidal activity against AMARE
- compounds 2, 3, 6, 7, 11, 12, 13, 15, 16, 17, 18, 19, 20, 21, 23, 24, 25 showed very good herbicidal activity against AMARE
- compounds 11, 13, 19, 21 showed good herbicidal activity against DIGSA
- compounds 7, 11, 12, 15, 16, 17, 18, 20, 23, 24, 25 showed very good herbicidal activity against DIGSA
- compounds 13, 15, 19 showed good herbicidal activity against SETVI
- compounds 12, 16, 17, 18, 20, 21, 23, 24, 25 showed very good herbicidal activity against SETVI

At an application rate of 1 kg/ha, applied by the post-emergence method:
- compounds 2, 3 showed very good herbicidal activity against ABUTH
- compounds 12, 24 showed good herbicidal activity against ALOMY
- compound 23 showed very good herbicidal activity against ALOMY
- compounds 1, 2, 3, 6, 7, 9, 11, 12, 13, 15, 16, 17, 18, 19, 20, 21, 22, 23, 24, 25 showed very good herbicidal activity against AMARE
- compounds 7, 15, 16, 17, 18 showed good herbicidal activity against AVEFA
- compounds 19, 20, 25 showed very good herbicidal activity against AVEFA
- compounds 2, 3, 6, 7, 11, 12, 13, 15, 16, 17, 18, 19, 20, 21, 22, 23, 24, 25 showed very good herbicidal activity against ECHCG

### B.2 Selectivity

To test the selectivity of compounds (I) for undesired vegetation, crop plants were submitted to a herbicidal treatment with a selection of representative compounds (I) in greenhouse experiments carried out in analogy to example B.1 to test whether compounds (I) show phytotoxicity vis-à-vis these plants.

The crop plants used in the greenhouse experiments were of the following species:

| Bayer code | Scientific name |
|---|---|
| GLXMA | *Glycine max* |
| ORYSA | *Oryza sativa* |
| TRZAW | *Triticum aestivum* |
| ZEAMX | *Zea mays* |

A low phytotoxicity is given at values of >10 to 20 and a very low phytotoxicity is given at values of 0 to 10.

At an application rate of 0.031 kg/ha, applied by the post-emergence method:
- compound 7 showed low phytotoxicity vis-à-vis GLXMA

At an application rate of 0.125 kg/ha, applied by the pre-emergence method:
- compounds 6, 7 showed very low phytotoxicity vis-à-vis TRZAW
- compound, 6, 7 showed very low phytotoxicity vis-à-vis ZEAMX

At an application rate of 0.125 kg/ha, applied by the post-emergence method:
- compounds 2, 3 showed very low phytotoxicity vis-à-vis GLXMA
- compounds 2, 3, 5, 6 showed very low phytotoxicity vis-à-vis TRZAW

At an application rate of 0.25 kg/ha, applied by the pre-emergence method:
- compound 14 showed low phytotoxicity vis-à-vis GLXMA

At an application rate of 0.5 kg/ha, applied by the pre-emergence method:
- compound 8 showed very low phytotoxicity vis-à-vis GLXMA

At an application rate of 0.5 kg/ha, applied by the post-emergence method:
- compound 4 showed very low phytotoxicity vis-à-vis GLXMA

At an application rate of 1 kg/ha, applied by the pre-emergence method:
- compounds 6, 15, 19 showed low phytotoxicity vis-à-vis GLXMA
- compounds 1, 9, 11, 12, 13, 17, 18, 21, 23, 25 showed very low phytotoxicity vis-à-vis GLXMA

At an application rate of 1 kg/ha, applied by the post-emergence method:
- compound 11 showed very low phytotoxicity vis-à-vis GLXMA

## Claims

1. Compounds of the formula (I) 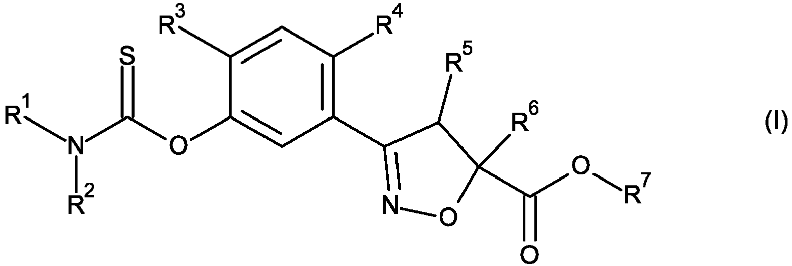 wherein:
R¹ and R², independently of each other, are C₁-C₆-alkyl, C₁-C₆-haloalkyl, C₃-C₆-cycloalkyl or C₃-C₆-halocycloalkyl; or
R¹ and R², together with the nitrogen atom they are bound to, form a 3- to 7-membered saturated heterocyclic ring which may contain a further heteroatom selected from N, O and S as ring member, and where the heterocyclic ring may carry one or more substituents selected from the group consisting of halogen, cyano, C₁-C₄-alkyl and C₁-C₄-haloalkyl;
R³ and R⁴, independently of each other, are hydrogen, halogen, cyano or nitro;
R⁵ is hydrogen, C₁-C₆-alkyl, C₁-C₆-haloalkyl, C₂-C₆-alkenyl, C₂-C₆-haloalkenyl, C₁-C₆-alkoxy or C₁-C₆-haloalkoxy;
R⁶ is C₁-C₆-alkyl, C₁-C₆-haloalkyl, C₂-C₆-alkenyl, C₂-C₆-haloalkenyl, C₁-C₆-alkoxy or C₁-C₆-haloalkoxy; or
R⁵ and R⁶ form together a C₃-C₅-alkylene group which may carry one or more substituents selected from the group consisting of halogen, cyano, C₁-C₄-alkyl and C₁-C₄-haloalkyl; and
R⁷ is hydrogen, C₁-C₆-alkyl, C₁-C₆-haloalkyl, C₃-C₆-cycloalkyl or C₃-C₆-halocycloalkyl;
or an agriculturally acceptable salt thereof, or a stereoisomer thereof.

2. The compounds as claimed in claim 1, wherein R¹ and R², together with the nitrogen atom they are bound to, form a 5-membered saturated heterocyclic ring which may carry one or more substituents selected from the group consisting of halogen, cyano, C₁-C₄-alkyl and C₁-C₄-haloalkyl.

3. The compounds as claimed in claim 2, wherein R¹ and R², together with the nitrogen atom they are bound to, form a 5-membered saturated heterocyclic ring which may carry one or two substituents selected from the group consisting of halogen and C₁-C₄-alkyl, preferably from fluorine and methyl.

4. The compounds as claimed in any of the preceding claims, wherein R³ is hydrogen or halogen, and R⁴ is halogen.

5. The compounds as claimed in claim 4, wherein R³ is hydrogen or fluorine, and R⁴ is chlorine.

6. The compounds as claimed in any of the preceding claims, wherein R⁵ is hydrogen and R⁶ is C₁-C₄-alkyl, C₂-C₄-alkenyl or C₁-C₄-alkoxy.

7. The compounds as claimed in any of claims 1 to 5, wherein R⁵ and R⁶ form together a linear C₃-alkylene group.

8. The compounds as claimed in any of the preceding claims, wherein R⁷ is hydrogen or C₁-C₄-alkyl.

9. The compounds as claimed in any of the preceding claims, wherein:
R¹ and R², independently of each other, are C₁-C₄-alkyl; or
R¹ and R², together with the nitrogen atom they are bound to, form a 5-membered saturated heterocyclic ring which may carry one or more substituents selected from the group consisting of halogen, cyano, C₁-C₄-alkyl and C₁-C₄-haloalkyl;
R³ is hydrogen or halogen;
R⁴ is halogen;
R⁵ is hydrogen;
R⁶ is C₁-C₄-alkyl, C₂-C₄-alkenyl or C₁-C₄-alkoxy; or
R⁵ and R⁶ form together a linear C₃-alkylene group; and
R⁷ is hydrogen or C₁-C₄-alkyl.

10. The compounds as claimed in claim 9, wherein:
R¹ and R², independently of each other, are C₁-C₄-alkyl; or
R¹ and R², together with the nitrogen atom they are bound to, form a 5-membered saturated heterocyclic ring which may carry one or more substituents selected from the group consisting of halogen and C₁-C₄-alkyl;
R³ is hydrogen or halogen;
R⁴ is halogen;
R⁵ is hydrogen;
R⁶ is C₁-C₄-alkyl, C₂-C₄-alkenyl or C₁-C₄-alkoxy; or
R⁵ and R⁶ form together a linear C₃-alkylene group; and
R⁷ is hydrogen or C₁-C₄-alkyl.

11. The compounds as claimed in claim 10, wherein:
R¹ and R², independently of each other, are C₁-C₃-alkyl; or
R¹ and R², together with the nitrogen atom they are bound to, form a 5-membered saturated heterocyclic ring which may carry one or two substituents selected from the group consisting of halogen and C₁-C₄-alkyl, preferably from fluorine and methyl; where in particular R¹ and R² form together a group -(CH₂)₄-, -(CH₂-CHF-CH₂-CH₂)-, -(CH₂-CF₂-CH₂-CH₂)- or -(CH₂-CH(CH₃)-CH₂-CH₂)-;
R³ is hydrogen or fluorine;
R⁴ is chlorine;
R⁵ is hydrogen;
R⁶ is C₁-C₃-alkyl, C₂-C₃-alkenyl or C₁-C₂-alkoxy; or
R⁵ and R⁶ form together a linear C₃-alkylene group; and
R⁷ is hydrogen or C₁-C₃-alkyl.

12. A composition comprising at least one compound as claimed in any one of claims 1 to 11, and at least one auxiliary which is customary for formulating crop protection compounds.

13. The composition as claimed in claim 12, comprising a further herbicide.

14. The use of a compound as claimed in any of claims 1 to 11, or of a composition as claimed in claims 12 or 13 for controlling unwanted vegetation.

15. A method for controlling unwanted vegetation which comprises allowing a herbicidally effective amount of at least one compound as claimed in any of claims 1 to 11, or a composition as claimed in claim 12 or 13 to act on plants, their seed and/or their habitat.
